# EUROPÄISCHE PATENTSCHRIFT

(11) **EP 3 535 240 B1**
(45) Veröffentlichungstag und Bekanntmachung des Hinweises auf die Patenterteilung: **16.11.2022**
(21) Anmeldenummer: 17788266.9
(22) Anmeldetag: 30.10.2017
(51) Int. Cl.: C07C 211/61, C07C 255/50, C07C 255/51, C07D 221/18, C07D 405/12, C07D 333/76, C07D 239/70, C07D 239/74, C07D 241/38, C07D 471/04, C07D 491/048, C07D 495/04, C07D 213/38, C07D 307/91, C09K 11/06, H01L 51/00

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 02.11.2016 EP 16196934
(43) Veröffentlichungstag der Anmeldung: 11.09.2019
(73) Patentinhaber: Merck Patent GmbH, 64293 Darmstadt (DE)
(72) Erfinder: PARHAM, Amir, 60486 Frankfurt am Main (DE); JOOSTEN, Dominik, 60487 Frankfurt am Main (DE); LUDEMANN, Aurélie, 60322 Frankfurt am Main (DE); GROSSMANN, Tobias, 64297 Darmstadt (DE); KROEBER, Jonas, 60311 Frankfurt am Main (DE)
(86) Internationale Anmeldenummer: PCT/EP2017/077728
(87) Internationale Veröffentlichungsnummer: WO 2018/083053

(56) Entgegenhaltungen:
- EP-A1- 2 175 005
- WO-A1-2010/050779
- WO-A1-2013/081410
- CN-A- 103 524 399
- CN-A- 105 669 467
- CN-A- 107 056 626
- JP-A- H0 378 757
- JP-A- 2004 339 064
- KR-A- 20110 076 271

## Beschreibung

Die vorliegende Anmeldung betrifft Fluoranthenyl-Amin-Verbindungen gemäß einer weiter unten definierten Formel (I). Diese Verbindungen eignen sich zur Verwendung in elektronischen Vorrichtungen. Weiterhin betrifft die vorliegende Anmeldung Verfahren zur Herstellung der genannten Verbindungen, sowie elektronische Vorrichtungen enthaltend die genannten Verbindungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs (organische Elektrolumineszenzvorrichtungen) verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Emissionsschichten und Schichten mit lochtransportierender Funktion. Zur Verwendung in diesen Schichten werden weiterhin neue Materialien, insbesondere Materialien mit lochtransportierenden und elektronentransportierenden Eigenschaften, gesucht. Von besonderem Interesse sind Materialien, die loch- und elektronentransportierende Eigenschaften in einer Verbindung vereinigen. Derartige Materialien werden als bipolare Materialien bezeichnet. Bevorzugt sind dabei die lochtransportierenden Eigenschaften in einem Teil der Verbindung, und die elektronentransportierenden Eigenschaften sind in einem anderen Teil der Verbindung lokalisiert.

Im Stand der Technik sind insbesondere Triarylamin-Verbindungen als Lochtransportmaterialien für elektronische Vorrichtungen bekannt.

Weiterhin werden in CN 105669467 A, CN 103524399 A, KR 2011-0076271 A, WO 2010/050779 A1, EP 2175005 A1, und JP 2004-339064 A Fluoranthenyl-Amine zur Verwendung in elektronischen Vorrichtungen offenbart.

Es besteht jedoch weiter Bedarf an alternativen Verbindungen, die zur Verwendung in elektronischen Vorrichtungen geeignet sind.

Es besteht auch Verbesserungsbedarf bezüglich der Leistungsdaten bei Verwendung in elektronischen Vorrichtungen, insbesondere bezüglich Lebensdauer und Effizienz.

Nun wurde gefunden, dass sich bestimmte Fluoranthen-Amin-Verbindungen hervorragend zur Verwendung in elektronischen Vorrichtungen eignen, insbesondere zur Verwendung in OLEDs, nochmals insbesondere darin zur Verwendung als Matrixmaterialien für phosphoreszierende Emitter.

Gegenstand der vorliegenden Anmeldung sind damit Verbindungen wie definiert in Anspruch 1.

Dabei bedeutet die Bindung, die den Sechsring der Einheit 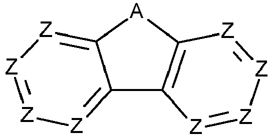 in Formel (I) durchzieht, dass die betreffende Einheit an jeder beliebigen Position am Sechsring an den Rest der Struktur der Formel (I) gebunden sein kann.

Für den Fall i=0 ist die Fluorenyl- bzw. Spirobifluorenylgruppe direkt mit dem Amin-Stickstoffatom verbunden.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugt ist in Formel (I) die Fluorenylgruppe bzw. Spirobifluorenylgruppe in einer der Positionen 1, 3, und 4 gebunden, besonders bevorzugt in einer der beiden Positionen 1 und 4, ganz besonders bevorzugt in der Position 4. Bei der Bindung in den betreffenden bevorzugten Positionen werden verbesserte Leistungsdaten der Verbindungen, insbesondere bei Verwendung als Matrixmaterial für phosphoreszierende Emitter, erreicht. Die Positionen an der Fluorenylgruppe bzw. der Spirobifluorenylgruppe sind wie folgt nummeriert:

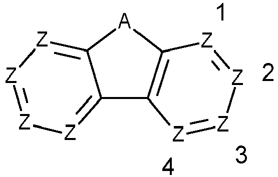

Es ist bevorzugt, dass A gleich C(R¹)₂ ist.

Es ist bevorzugt, dass maximal zwei Gruppen Z pro aromatischem Sechsring gleich N sind, besonders bevorzugt nur eine Gruppe Z pro aromatischem Sechsring gleich N ist.

Bevorzugt ist Z gleich CR² oder C, wobei eine Gruppe Z genau dann gleich C ist, wenn an sie die Gruppe [L¹]ᵢ gebunden ist.

Es ist bevorzugt, dass maximal zwei Gruppen X in der Fluoranthengruppe gleich N sind, besonders bevorzugt nur eine Gruppe X in der Fluoranthengruppe gleich N ist. Ganz besonders bevorzugt ist X gleich CR³ oder C, wobei eine Gruppe X genau dann gleich C ist, wenn sie an die Gruppe [L²]ₖ gebunden ist.

L¹, L² sind bevorzugt bei jedem Auftreten gleich oder verschieden gewählt aus wahlweise mit Resten R⁴ substituiertem para-, meta-, oder ortho-Phenylen, Naphthylen, Biphenylen, Dibenzofuranylen und Dibenzothiophenylen. Bevorzugt sind die genannten Gruppen nicht mit Resten R⁴ substituiert.

L¹, L² sind bevorzugt gewählt aus Gruppen der folgenden Formeln:

| | | |
|---|---|---|
| 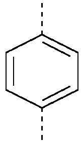 | 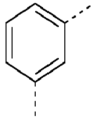 | 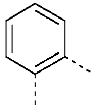 |
| L-1 | L-2 | L-3 |
| 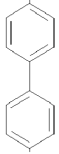 | 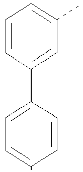 | 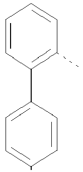 |
| L-4 | L-5 | L-6 |
| 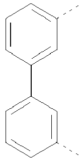 | 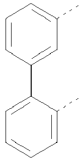 | 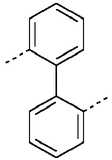 |
| L-7 | L-8 | L-9 |
| 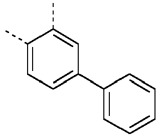 | 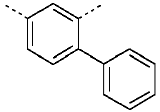 | 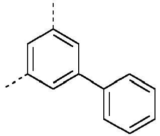 |
| L-10 | L-11 | L-12 |
| 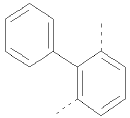 | 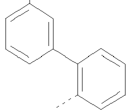 | |
| L-13 | L-14 | |
| | | 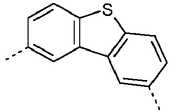 |
| | | L-18 |
| 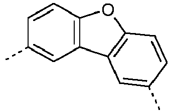 | 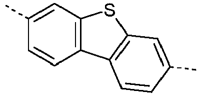 | 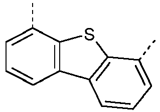 |
| L-19 | L-20 | L-21 |
| 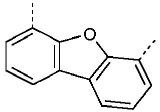 | 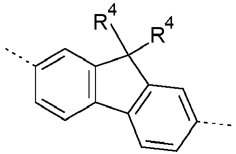 | 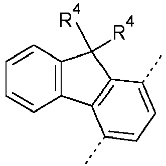 |
| L-22 | L-23 | L-24 |
| 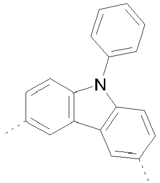 | 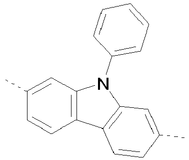 | 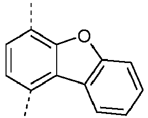 |
| L-25 | L-26 | L-27 |
| 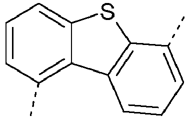 | 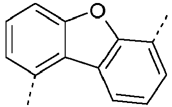 | 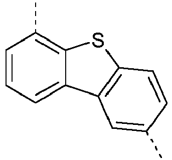 |
| L-28 | L-29 | L-30 |
| 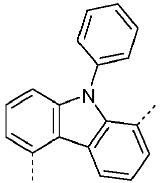 | 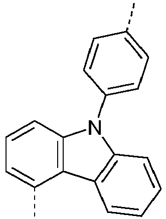 | |
| L-31 | L-32 | |

wobei die gestrichelten Bindungen die Bindungen an den Rest der Formel darstellen und die Gruppen an den freien Positionen durch einen oder mehrere Reste R⁴ substituiert sein können, bevorzugt an den freien Positionen aber unsubstituiert sind.

Bevorzugt ist Ar¹ ein aromatisches Ringsystem mit 6 bis 24 aromatischen Ringatomen, das mit einem oder mehreren Resten R⁵ substituiert sein kann, oder ein heteroaromatisches Ringsystem mit 5 bis 24 aromatischen Ringatomen, das mit einem oder mehreren Resten R⁵ substituiert sein kann, wobei Ar¹ keine stickstoffhaltige Heteroarylgruppe umfasst. Besonders bevorzugt ist Ar¹ gewählt aus wahlweise mit Resten R⁵ substituiertem Phenyl, Biphenyl, verzweigtem Terphenyl, unverzweigtem Terphenyl, verzweigtem Quaterphenyl, unverzweigtem Quaterphenyl, Fluorenyl, Dibenzofuranyl, Dibenzothiophenyl, Fluorenyl-Phenylen, Dibenzofuranyl-Phenylen, Dibenzothiophen-Phenylen, Phenanthrenyl und Triphenylyl.

Ganz besonders bevorzugt ist Ar¹ gewählt aus den folgenden Gruppen

| | | |
|---|---|---|
| 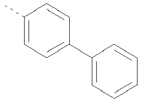 | 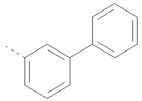 | 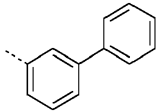 |
| Ar-1 | Ar-3 | Ar-3 |
| 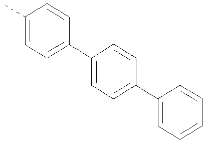 | 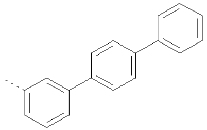 | 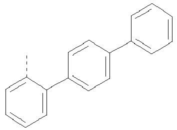 |
| Ar-4 | Ar-5 | Ar-6 |
| 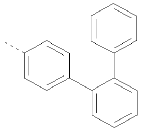 | 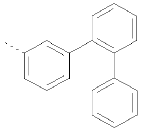 | 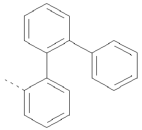 |
| Ar-7 | Ar-8 | Ar-9 |
| 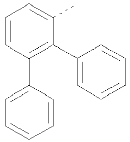 | 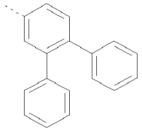 | 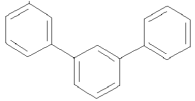 |
| Ar-10 | Ar-11 | Ar-12 |
| 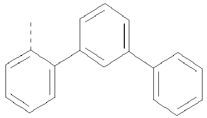 | 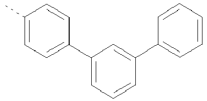 | 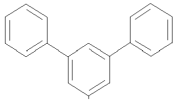 |
| Ar-13 | Ar-14 | Ar-15 |
| 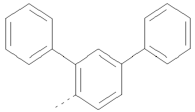 | 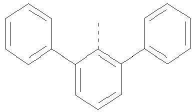 | 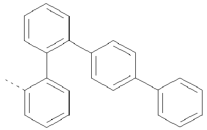 |
| Ar-16 | Ar-17 | Ar-18 |
| 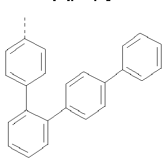 | 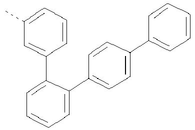 | 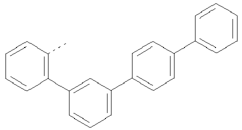 |
| Ar-19 | Ar-20 | Ar-21 |
| 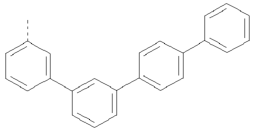 | 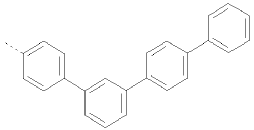 | 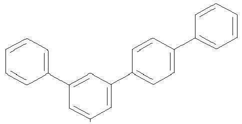 |
| Ar-22 | Ar-23 | Ar-24 |
| 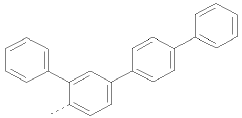 | 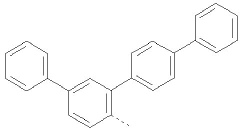 | 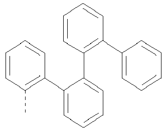 |
| Ar-25 | Ar-26 | Ar-27 |
| 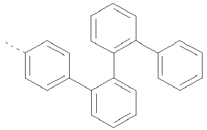 | 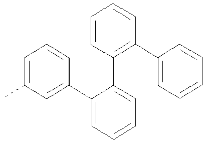 | 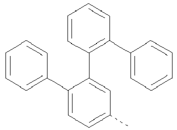 |
| Ar-28 | Ar-29 | Ar-30 |
| 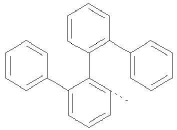 | 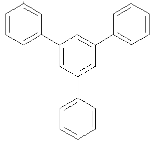 | 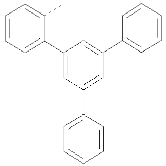 |
| Ar-31 | Ar-32 | Ar-33 |
| 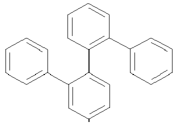 | 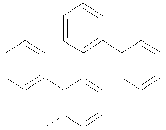 | 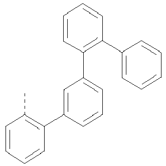 |
| Ar-34 | Ar-35 | Ar-36 |
| 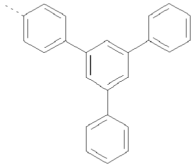 | 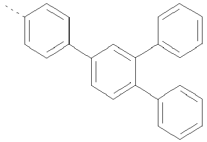 | 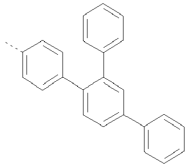 |
| Ar-37 | Ar-38 | Ar-39 |
| 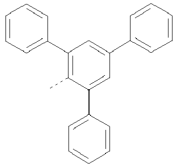 | 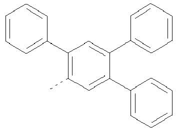 | 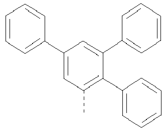 |
| Ar-40 | Ar-41 | Ar-42 |
| 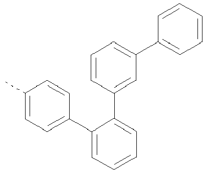 | 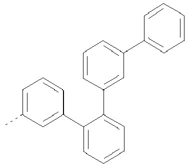 | 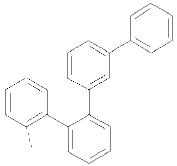 |
| Ar-43 | Ar-44 | Ar-45 |
| 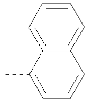 | 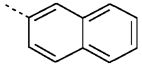 | 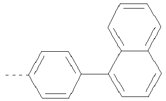 |
| Ar-46 | Ar-47 | Ar-48 |
| 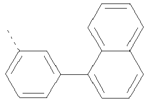 | 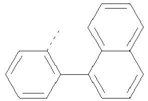 | 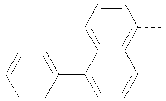 |
| Ar-49 | Ar-50 | Ar-51 |
| 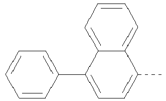 | 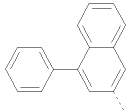 | 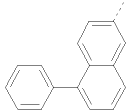 |
| Ar-52 | Ar-53 | Ar-54 |
| 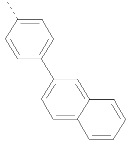 | 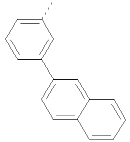 | 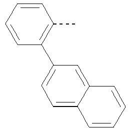 |
| Ar-55 | Ar-56 | Ar-57 |
| 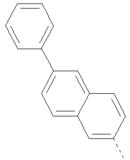 | 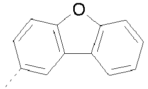 | 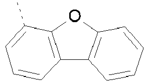 |
| Ar-58 | Ar-59 | Ar-60 |
| 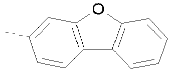 | 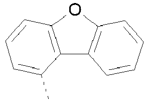 | 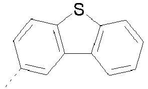 |
| Ar-61 | Ar-62 | Ar-63 |
| 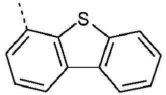 | 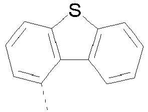 | 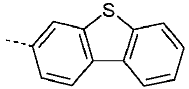 |
| Ar-64 | Ar-65 | Ar-66 |
| 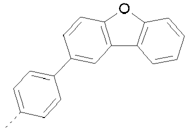 | 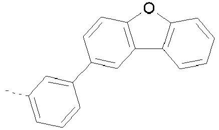 | 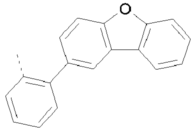 |
| Ar-67 | Ar-68 | Ar-69 |
| 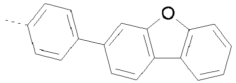 | 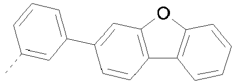 | 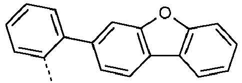 |
| Ar-70 | Ar-71 | Ar-72 |
| 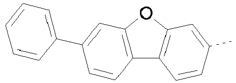 | 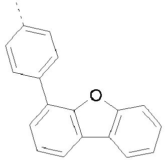 | 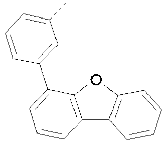 |
| Ar-73 | Ar-74 | Ar-75 |
| 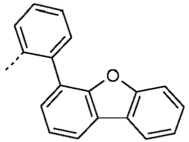 | 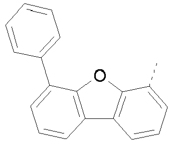 | 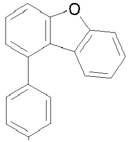 |
| Ar-76 | Ar-77 | Ar-78 |
| 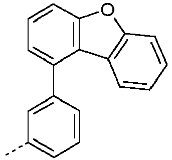 | 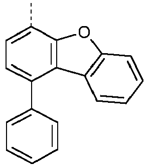 | 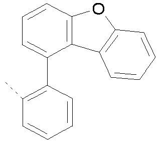 |
| Ar-79 | Ar-80 | Ar-81 |
| 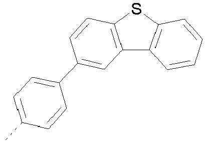 | 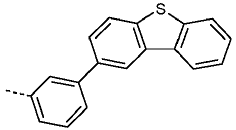 | 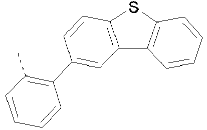 |
| Ar-82 | Ar-83 | Ar-84 |
| 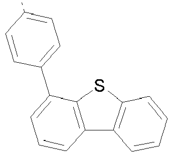 | 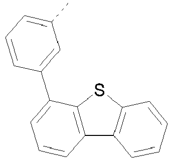 | 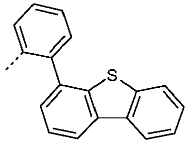 |
| Ar-85 | Ar-86 | Ar-87 |
| 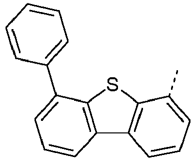 | 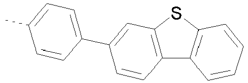 | 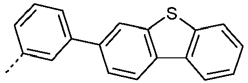 |
| Ar-88 | Ar-89 | Ar-90 |
| 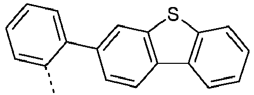 | 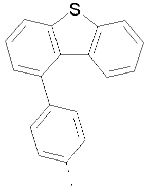 | 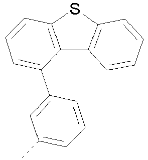 |
| Ar-91 | Ar-92 | Ar-93 |
| 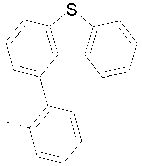 | 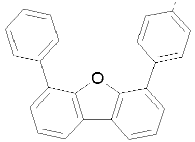 | 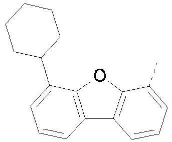 |
| Ar-94 | Ar-95 | Ar-96 |
| 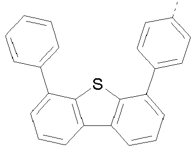 | 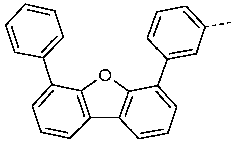 | 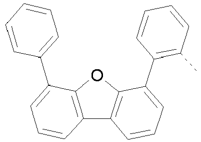 |
| Ar-97 | Ar-98 | Ar-99 |
| 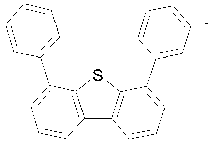 | 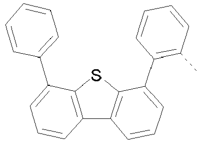 | 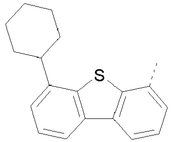 |
| Ar-100 | Ar-101 | Ar-102 |
| 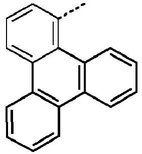 | 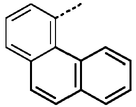 | 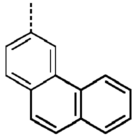 |
| Ar-103 | Ar-104 | Ar-105 |
| 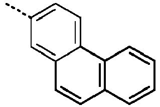 | 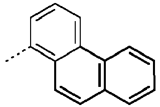 | 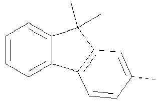 |
| Ar-106 | Ar-107 | Ar-108 |
| 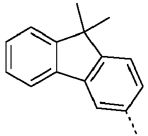 | 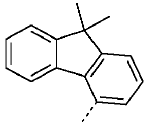 | 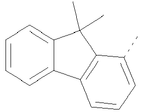 |
| Ar-109 | Ar-110 | Ar-111 |
| 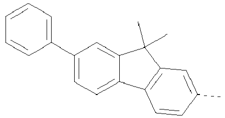 | 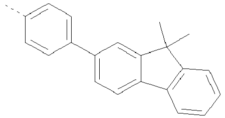 | 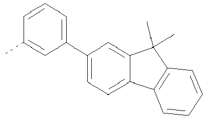 |
| Ar-112 | Ar-113 | Ar-114 |
| 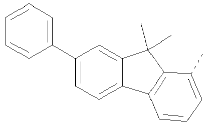 | 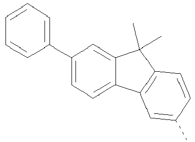 | 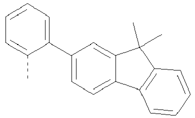 |
| Ar-115 | Ar-116 | Ar-117 |
| 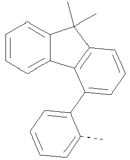 | 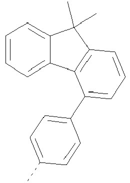 | 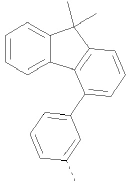 |
| Ar-118 | Ar-119 | Ar-120 |
| 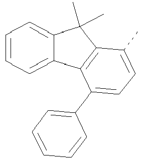 | 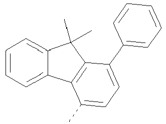 | 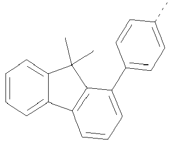 |
| Ar-121 | Ar-122 | Ar-123 |
| 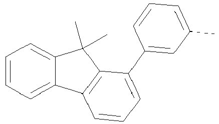 | 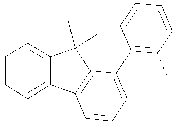 | 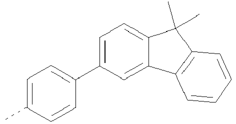 |
| Ar-124 | Ar-125 | Ar-126 |
| 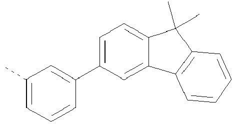 | 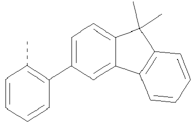 | 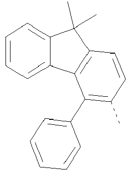 |
| Ar-127 | Ar-128 | Ar-129 |
| 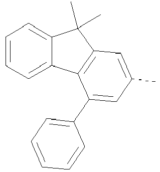 | 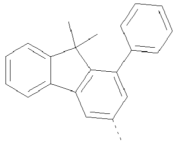 | 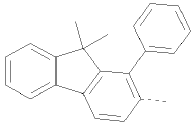 |
| Ar-130 | Ar-131 | Ar-132 |
| 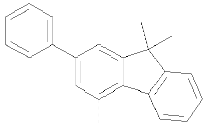 | 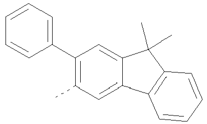 | 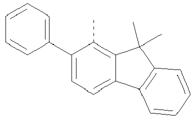 |
| Ar-133 | Ar-134 | Ar-135 |
| 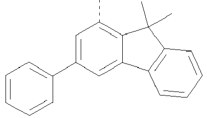 | 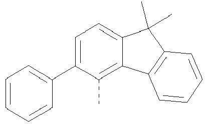 | 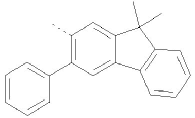 |
| Ar-136 | Ar-137 | Ar-138 |
| 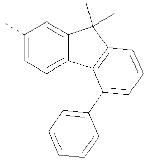 | 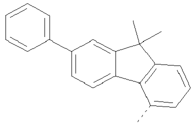 | 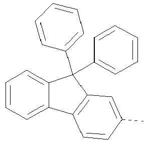 |
| Ar-139 | Ar-140 | Ar-141 |
| 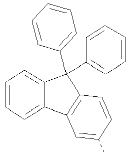 | 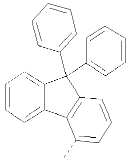 | 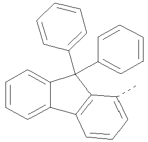 |
| Ar-142 | Ar-143 | Ar-144 |
| 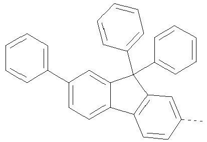 | 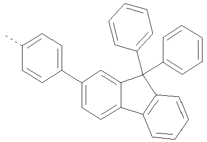 | 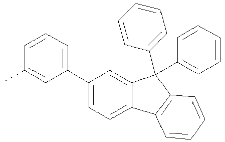 |
| Ar-145 | Ar-146 | Ar-147 |
| 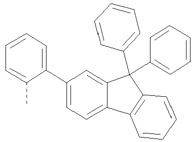 | 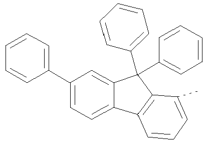 | 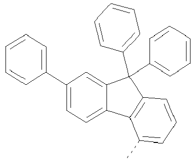 |
| Ar-148 | Ar-149 | Ar-150 |
| 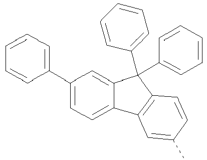 | 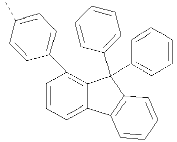 | 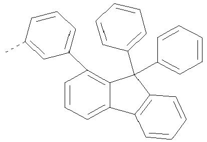 |
| Ar-151 | Ar-152 | Ar-153 |
| 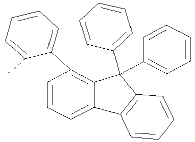 | 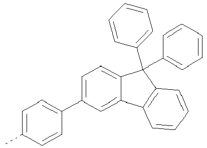 | 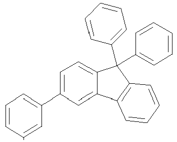 |
| Ar-154 | Ar-155 | Ar-156 |
| 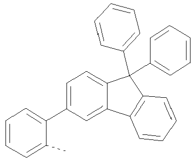 | 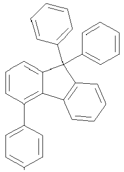 | 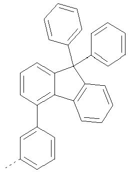 |
| Ar-157 | Ar-158 | Ar-159 |
| 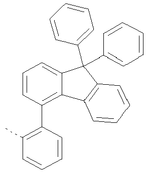 | 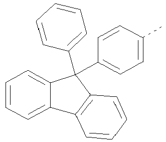 | 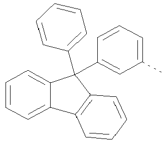 |
| Ar-160 | Ar-161 | Ar-162 |
| 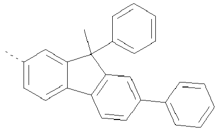 | 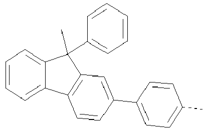 | 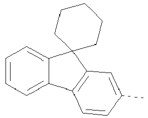 |
| Ar-163 | Ar-164 | Ar-165 |
| 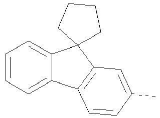 | 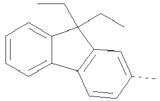 | 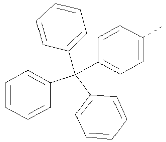 |
| Ar-166 | Ar-167 | Ar-168 |
| 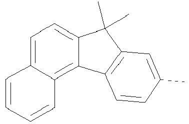 | 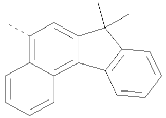 | 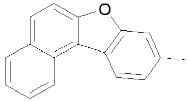 |
| Ar-169 | Ar-170 | Ar-171 |
| 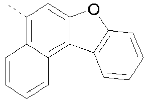 | 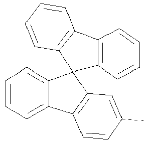 | 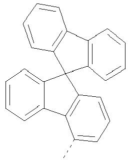 |
| Ar-172 | Ar-173 | Ar-174 |
| 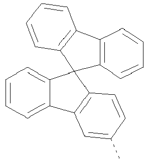 | 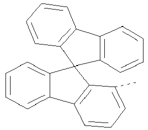 | 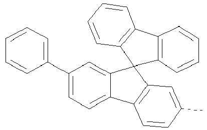 |
| Ar-175 | Ar-176 | Ar-177 |
| 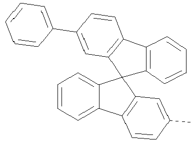 | 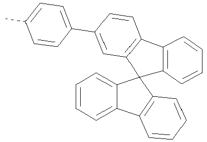 | 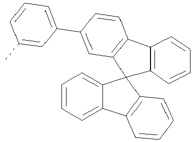 |
| Ar-178 | Ar-179 | Ar-180 |
| 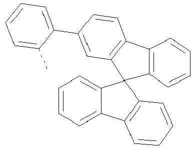 | 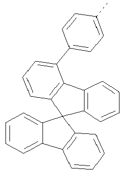 | 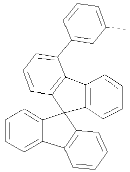 |
| Ar-181 | Ar-182 | Ar-183 |
| 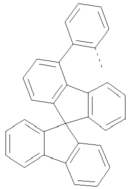 | 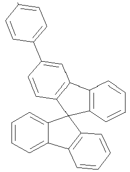 | 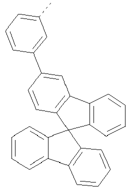 |
| Ar-184 | Ar-185 | Ar-186 |
| 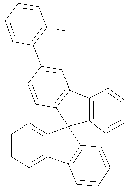 | 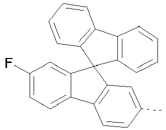 | 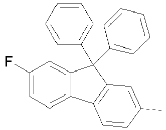 |
| Ar-187 | Ar-188 | Ar-189 |
| 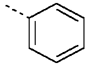 | 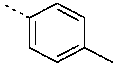 | 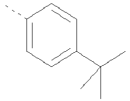 |
| Ar-190 | Ar-191 | Ar-192 |
| 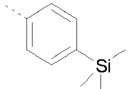 | 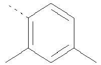 | 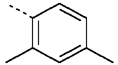 |
| Ar-193 | Ar-194 | Ar-195 |
| 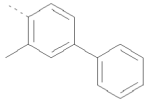 | 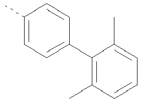 | 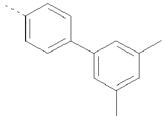 |
| Ar-196 | Ar-197 | Ar-198 |
| 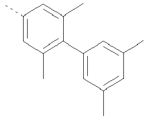 | 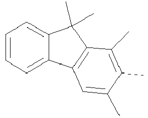 | 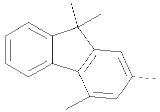 |
| Ar-199 | Ar-200 | Ar-201 |
| 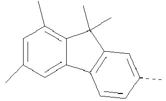 | 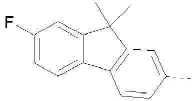 | 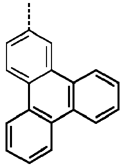 |
| Ar-202 | Ar-203 | Ar-204 |

welche an den unsubstituiert gezeichneten Positionen mit Resten R⁵ substituiert sein können, bevorzugt an diesen Positionen aber unsubstituiert sind, und welche über die gestrichelte Bindung an den Rest der Formel (I) gebunden sind.

R¹ ist bevorzugt bei jedem Auftreten gleich oder verschieden gewählt aus F, Si(R⁶)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R⁶ substituiert sein können; und wobei zwei Gruppen R¹, die an dasselbe Kohlenstoffatom binden, miteinander zu einem Ring verbunden sein können, so dass ein Spiro-Kohlenstoffatom entsteht. Bevorzugt entsteht in diesem Fall ein Cyclopropyl-, Cyclobutyl-, Cyclopentyl-, Cyclohexyl- oder Cycloheptylring, der ein Spiro-Kohlenstoffatom umfasst.

R³ und R⁴ sind bevorzugt bei jedem Auftreten gleich oder verschieden gewählt aus H, D, F, CN, Si(R⁶)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R⁶ substituiert sein können; und wobei in den genannten Alkylgruppen eine oder mehrere CH₂-Gruppen durch -C=C-, -R⁶C=CR⁶-, Si(R⁶)₂, C=O, C=NR⁶, -NR⁶-, -O-, -S-, -C(=O)O- oder -C(=O)NR⁶- ersetzt sein können.

R² ist bevorzugt bei jedem Auftreten gleich oder verschieden gewählt aus H, D, F, CN, Si(R⁶)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R⁶ substituiert sein können; und wobei in den genannten Alkylgruppen eine oder mehrere CH₂-Gruppen durch -C=C-, Si(R⁶)₂, C=O, C=NR⁶, -NR⁶-, -O-, -S-, -C(=O)O- oder -C(=O)NR⁶- ersetzt sein können. Besonders bevorzugt ist R² gleich H oder ein aromatisches Ringsystem mit 6 bis 24 aromatischen Ringatomen, das mit einem oder mehreren Resten R⁶ substituiert sein kann, oder ein heteroaromatisches Ringsystem mit 5 bis 24 aromatischen Ringatomen, das mit einem oder mehreren Resten R⁶ substituiert sein kann, ganz besonders bevorzugt gleich H. Weiterhin besonders bevorzugt ist R³ gleich H oder ein aromatisches Ringsystem mit 6 bis 24 aromatischen Ringatomen, das mit einem oder mehreren Resten R⁶ substituiert sein kann, oder ein heteroaromatisches Ringsystem mit 5 bis 24 aromatischen Ringatomen, das mit einem oder mehreren Resten R⁶ substituiert sein kann, ganz besonders bevorzugt gleich H.

R⁵ ist bevorzugt bei jedem Auftreten gleich oder verschieden gewählt aus H, D, Si(R⁶)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R⁶ substituiert sein können, wobei R⁵ und Substituenten, die an R⁵ binden, keine Carbazolgruppe enthalten. Besonders bevorzugt ist R⁵ gleich H oder ein aromatisches Ringsystem mit 6 bis 24 aromatischen Ringatomen, das mit einem oder mehreren Resten R⁶ substituiert sein kann, wobei Substituenten, die an R⁵ binden, keine Carbazolgruppe enthalten; ganz besonders bevorzugt ist R⁵ gleich H.

R⁶ ist bevorzugt bei jedem Auftreten gleich oder verschieden gewählt aus H, D, F, CN, Si(R⁷)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R⁷ substituiert sein können; und wobei in den genannten Alkylgruppen eine oder mehrere CH₂-Gruppen durch -C=C-, -R⁷C=CR⁷-, Si(R⁷)2, C=O, C=NR⁷, -NR⁷-, -O-, -S-, -C(=O)O- oder -C(=O)NR⁷- ersetzt sein können.

Index i ist bevorzugt gleich 0 oder 1, besonders bevorzugt gleich 0.

Index k ist bevorzugt gleich 1. Für derartige Verbindungen werden verbesserte Leistungsdaten bei der Verwendung in elektronischen Vorrichtungen, insbesondere in OLEDs, nochmals insbesondere bei der Verwendung als Matrixmaterialien für phosphoreszierende Emitter in OLEDs, erhalten. Diese Verbesserungen betreffen insbesondere die Effizienz, die Lebensdauer und die Betriebsspannung der Vorrichtungen.

Bevorzugte Verbindungen entsprechen einer der folgenden Formeln (I-1) bis (I-12) 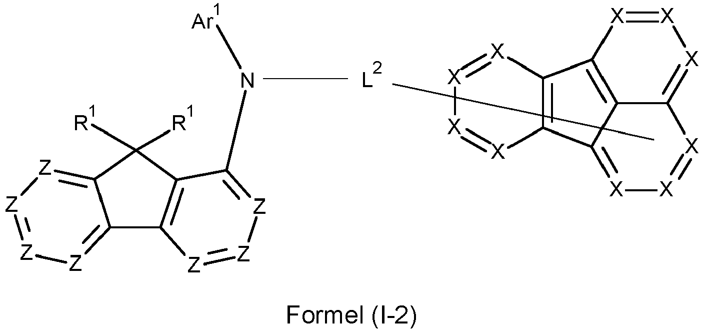 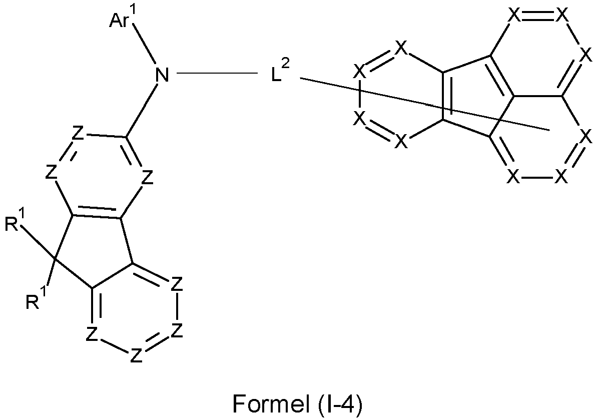 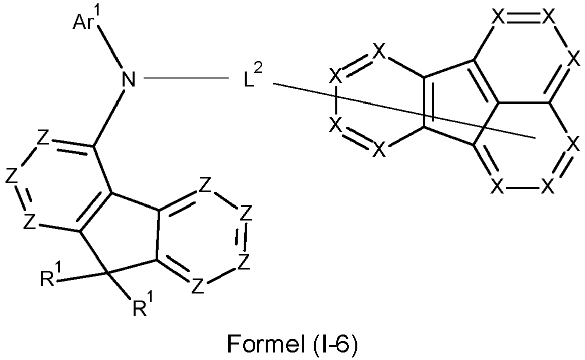 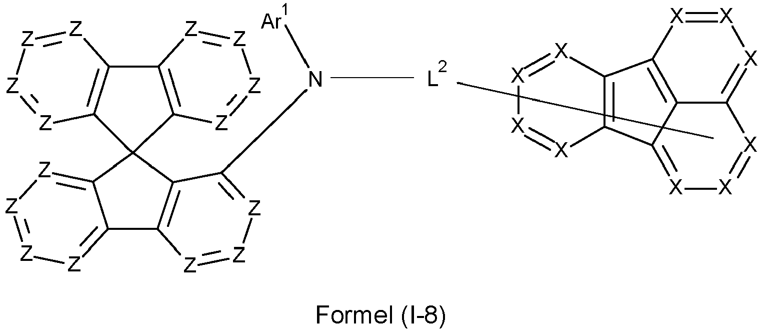 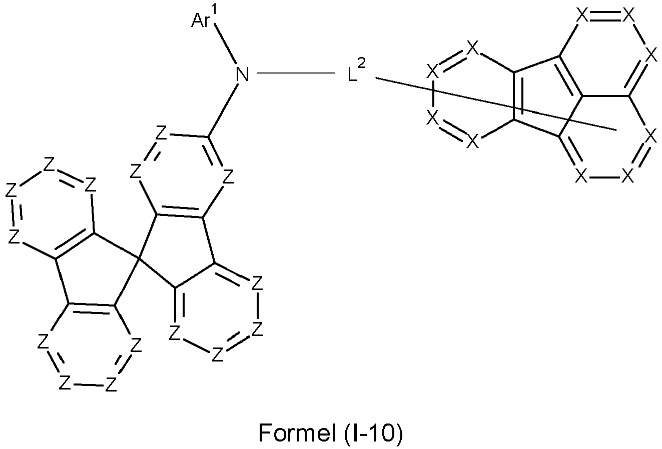 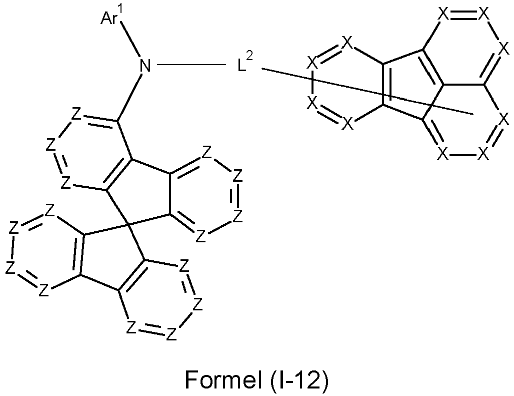 wobei die auftretenden Symbole definiert sind wie oben. Bevorzugt gelten für die Formeln die oben angegebenen bevorzugten Ausführungsformen der Variablen. Insbesondere ist es bevorzugt, dass die Fluoranthengruppe in der Position 3 gebunden ist, wie oben definiert. Weiterhin insbesondere ist es bevorzugt, dass Z gleich CR² ist, und/oder X gleich CR³ oder C ist, wobei X genau dann gleich C ist, wenn an das betreffende X das Stickstoffatom beziehungsweise die Gruppe L² gebunden ist; und/oder dass L² gemäß den oben genannten bevorzugten Ausführungsformen definiert ist, und/oder dass Ar¹ gemäß den oben genannten bevorzugten Ausführungsformen definiert ist.

Besonders bevorzugt unter den oben angegebenen Formeln sind die Formeln (I-2), (I-6), (I-8) und (I-12), ganz besonders bevorzugt die Formel (I-6).

Bevorzugte Verbindungen der Formel (I) sind im Folgenden abgebildet. Verbindungen, die nicht unter die geänderten Ansprüche fallen, sind mit # gekennzeichnet:

| | | |
|---|---|---|
| 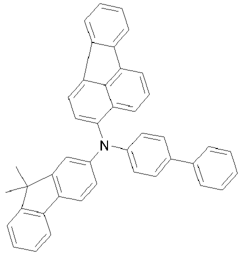 | 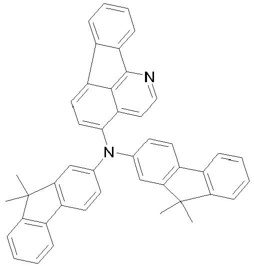 | 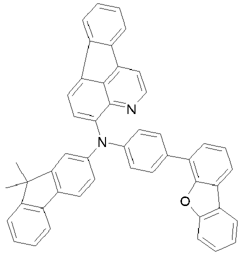 |
| 1# | 2# | 3# |
| 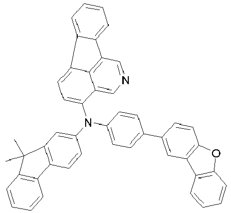 | 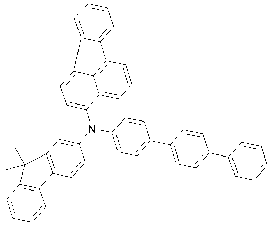 | 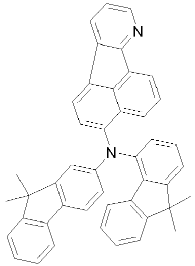 |
| 4# | 5# | 6# |
| 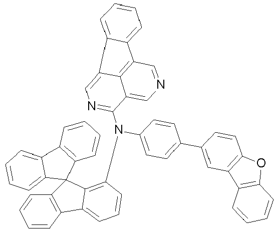 | 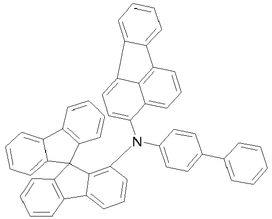 | 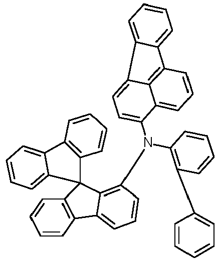 |
| 7# | 8# | 9# |
| 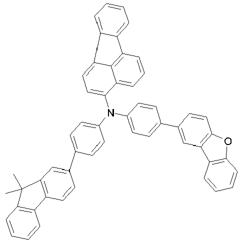 | 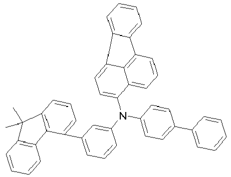 | 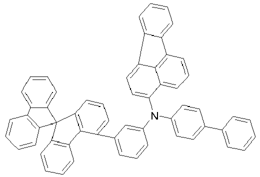 |
| 10# | 11# | 12# |
| 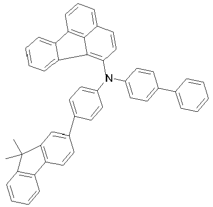 | 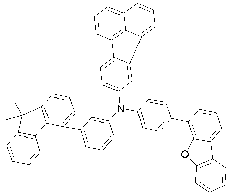 | 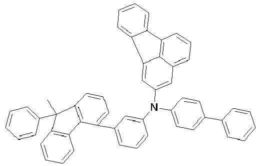 |
| 13# | 14# | 15# |
| 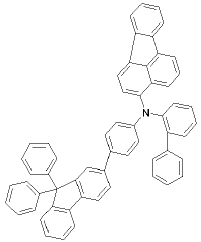 | 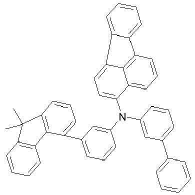 | 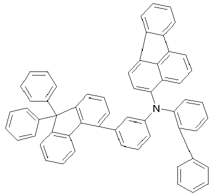 |
| 16# | 17# | 18# |
| 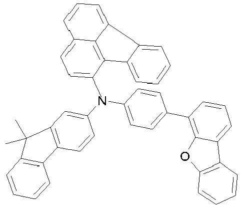 | 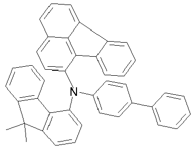 | 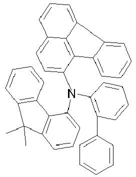 |
| 19# | 20# | 21# |
| 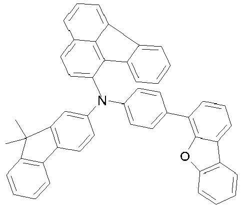 | 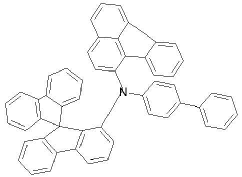 | 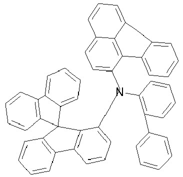 |
| 22# | 23# | 24# |
| 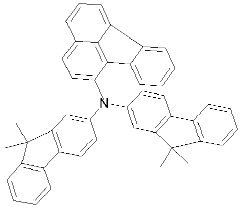 | 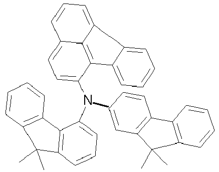 | 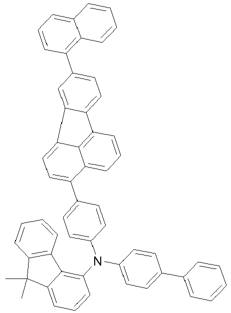 |
| 25# | 26# | 27 |
| 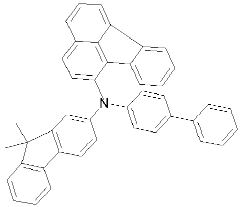 | 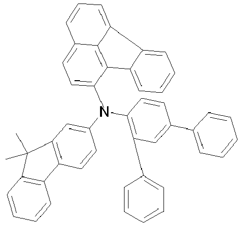 | 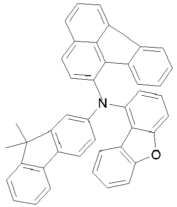 |
| 28# | 29# | 30# |
| 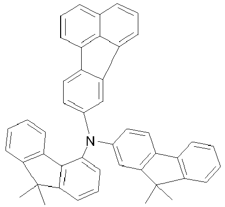 | 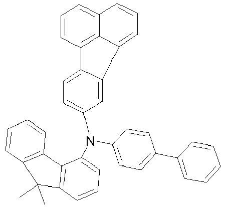 | 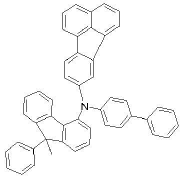 |
| 31# | 32# | 33# |
| 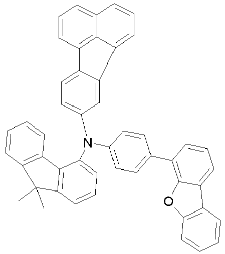 | 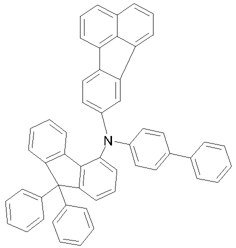 | 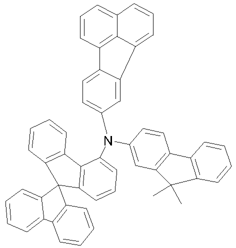 |
| 34# | 35# | 36# |
| 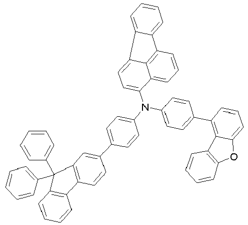 | 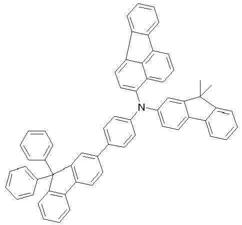 | 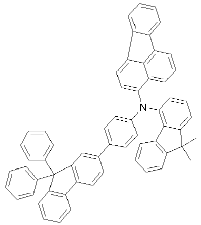 |
| 37# | 38# | 39# |
| 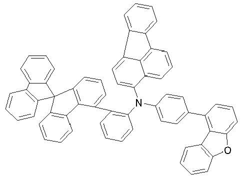 | 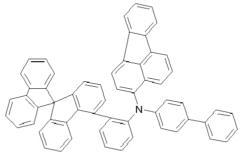 | 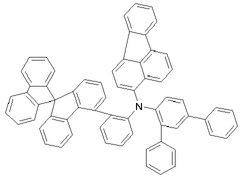 |
| 40# | 41# | 42# |
| 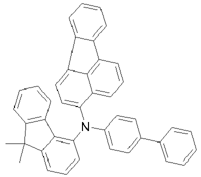 | 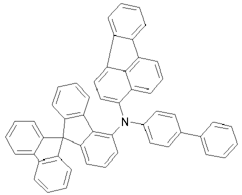 | 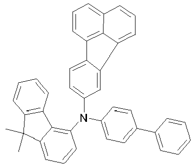 |
| 43# | 44# | 45# |
| 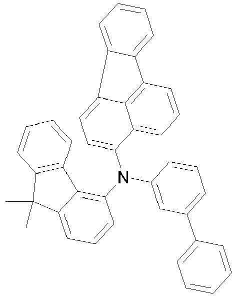 | 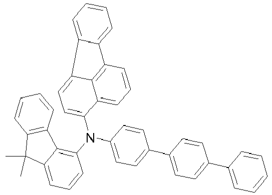 | 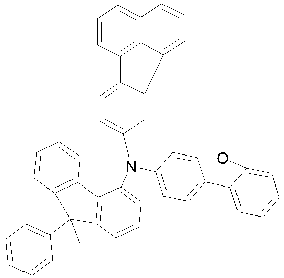 |
| 46# | 47# | 48# |
| 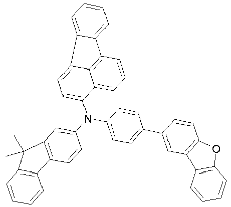 | 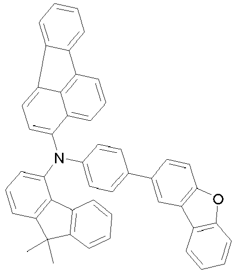 | 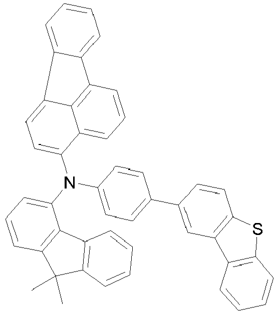 |
| 49# | 50# | 51# |
| 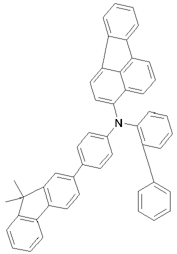 | 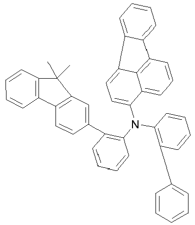 | 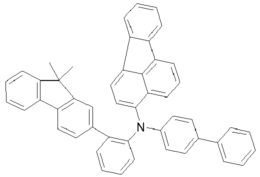 |
| 52# | 53# | 54# |
| 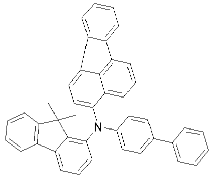 | 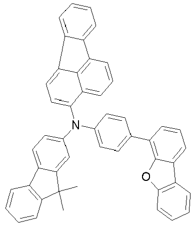 | 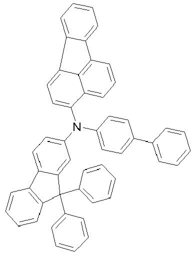 |
| 55# | 56# | 57# |
| 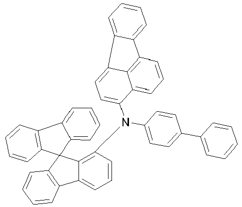 | 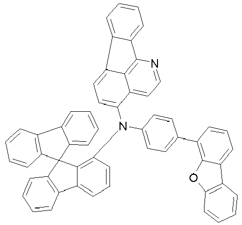 | 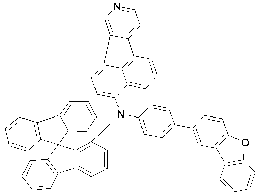 |
| 58# | 59# | 60# |
| 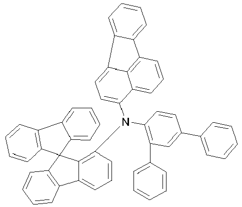 | 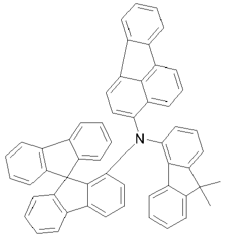 | 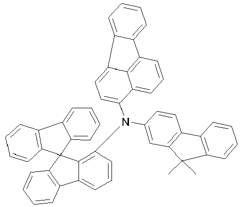 |
| 61# | 62# | 63# |
| 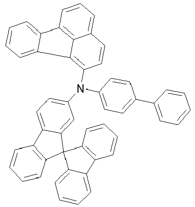 | 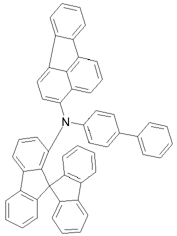 | 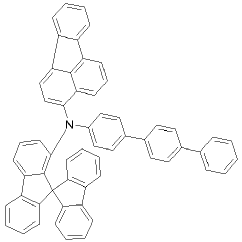 |
| 64# | 65# | 66# |
| 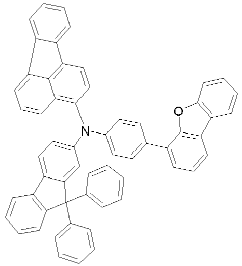 | 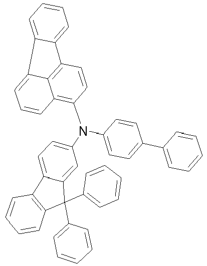 | 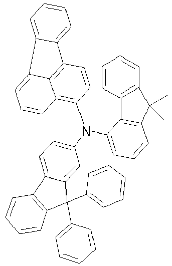 |
| 67# | 68# | 69# |
| 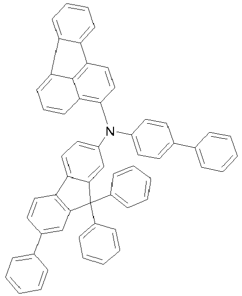 | 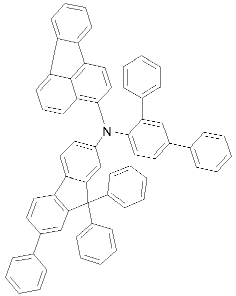 | 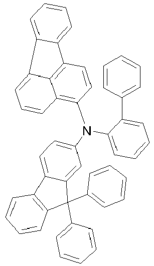 |
| 70# | 71# | 72# |
| 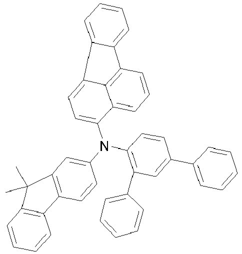 | 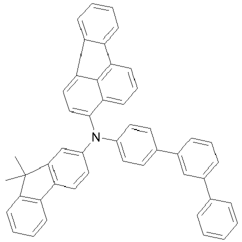 | 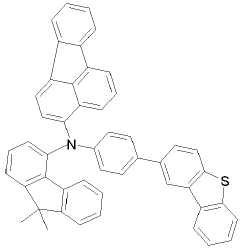 |
| 73# | 74# | 75# |
| 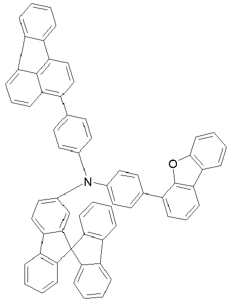 | 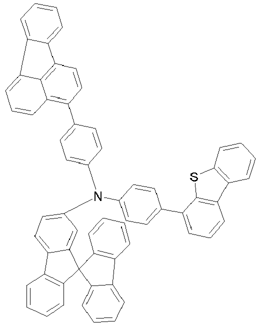 | 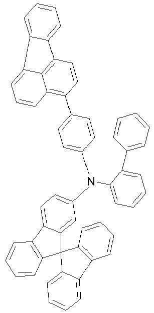 |
| 76 | 77 | 78 |
| 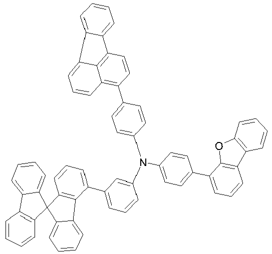 | 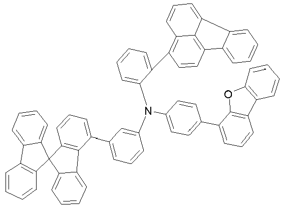 | 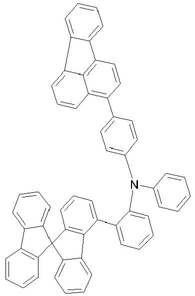 |
| 79 | 80 | 81 |
| 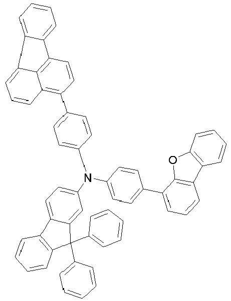 | 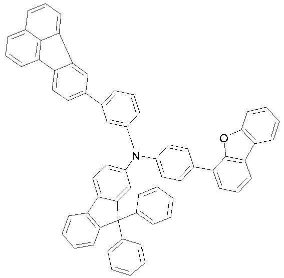 | 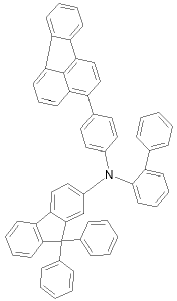 |
| 82 | 83# | 84 |
| 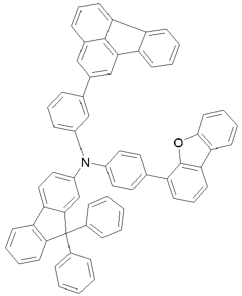 | 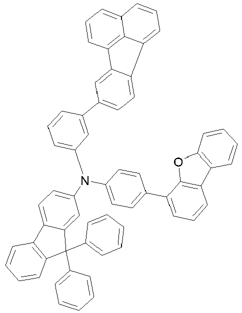 | 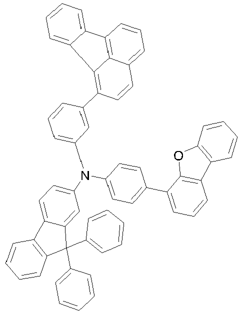 |
| 85# | 86# | 87# |
| 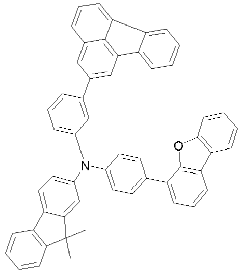 | 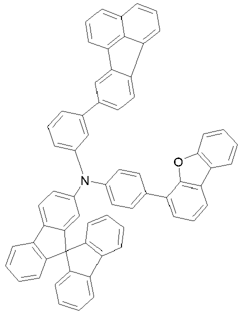 | 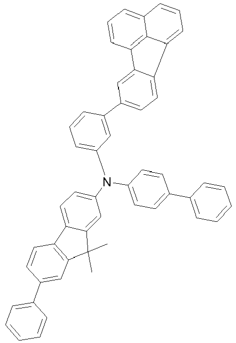 |
| 88# | 89# | 90# |
| 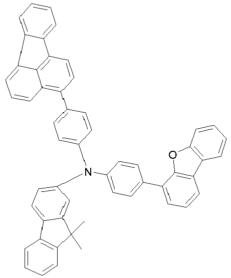 | 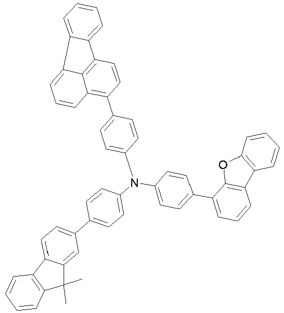 | 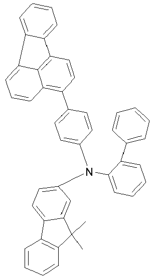 |
| 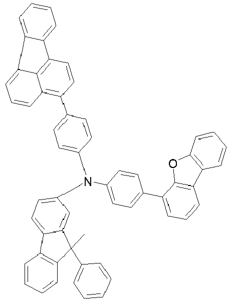 | 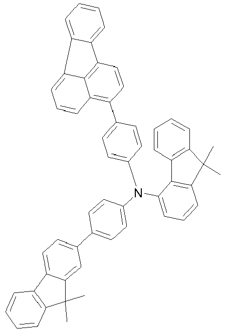 | 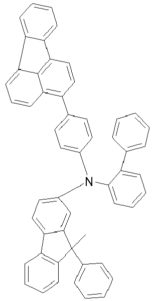 |
| 91 | 92 | 93 |
| 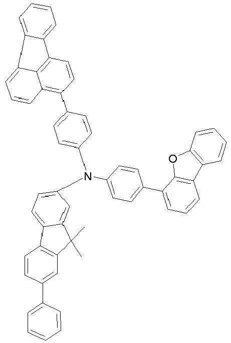 | 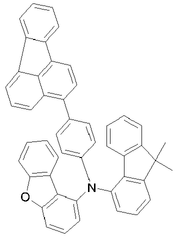 | 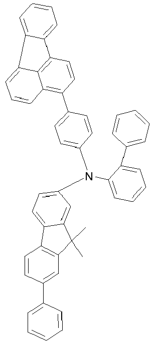 |
| 94 | 95 | 96 |
| 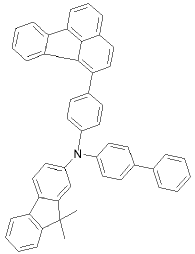 | 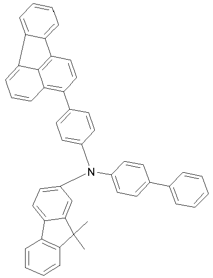 | 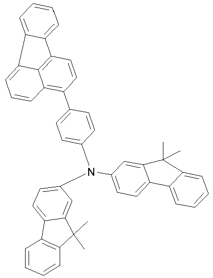 |
| 97# | 98 | 99 |
| 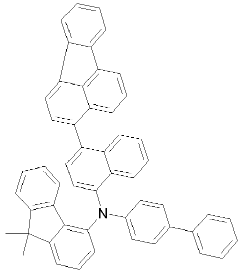 | 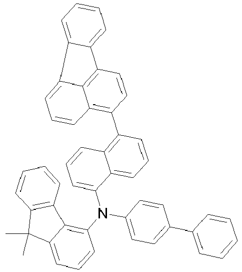 | 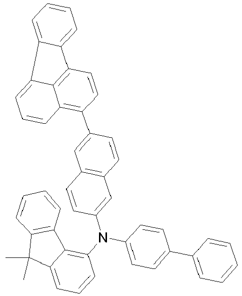 |
| 100# | 101# | 102# |
| 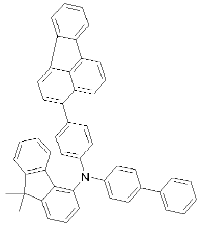 | 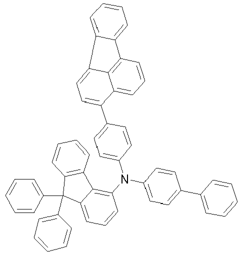 | 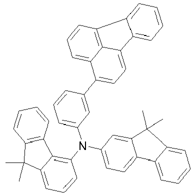 |
| 103 | 104 | 105 |
| 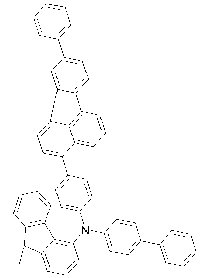 | 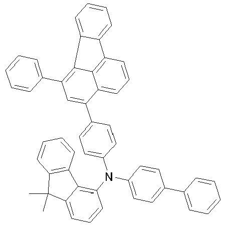 | 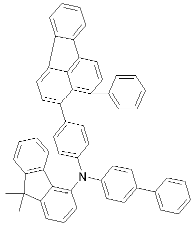 |
| 106 | 107 | 108 |
| 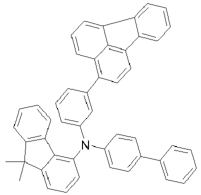 | 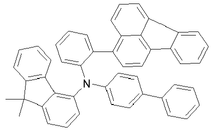 | 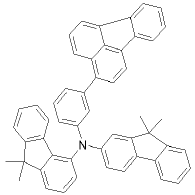 |
| 109 | 110 | 111 |
| 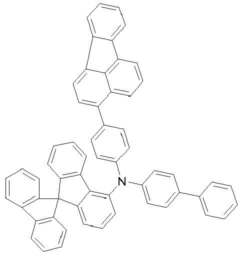 | 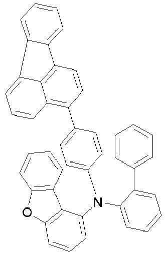 | 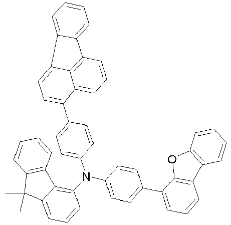 |
| 112 | 113 | 114 |
| 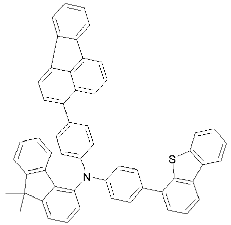 | 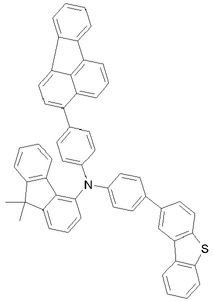 | 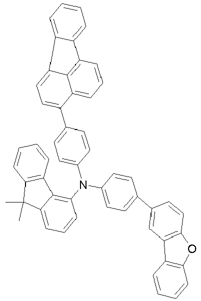 |
| 115 | 116 | 117 |
| 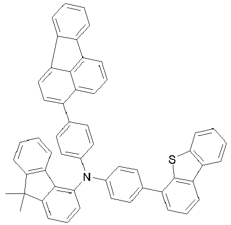 | 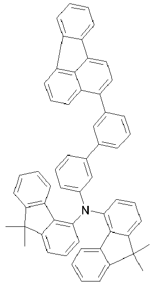 | 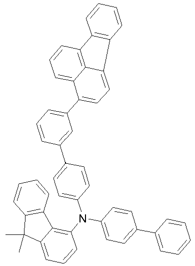 |
| 118 | 119 | 120 |
| 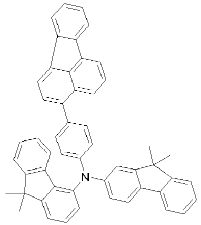 | 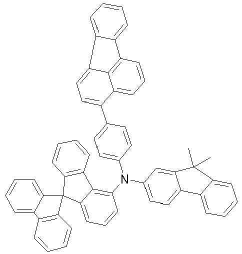 | 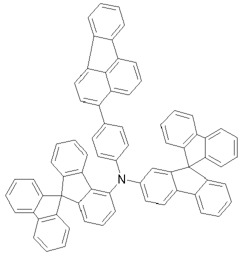 |
| 121 | 122 | 123 |
| 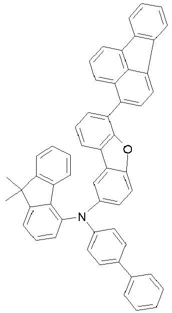 | 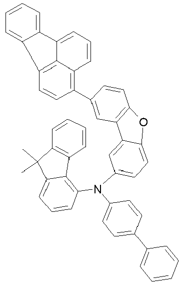 | 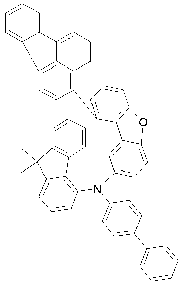 |
| 124 | 125 | 126 |
| 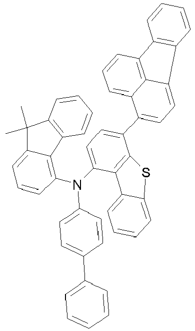 | 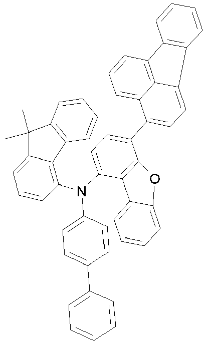 | 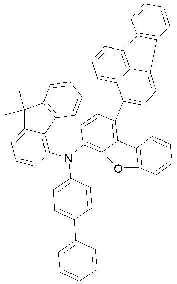 |
| 127 | 128 | 129 |
| 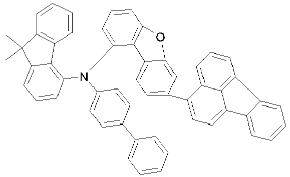 | 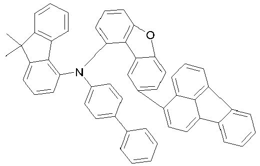 | 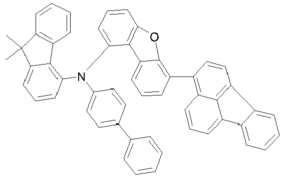 |
| 130 | 131 | 132 |
| 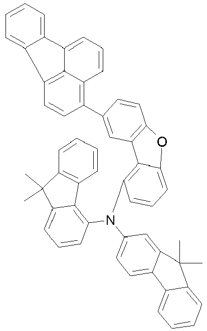 | 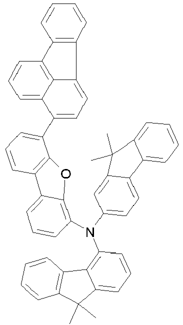 | 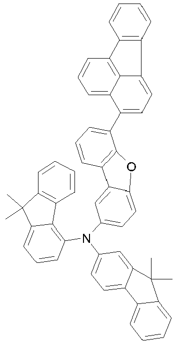 |
| 133 | 134 | 135 |
| 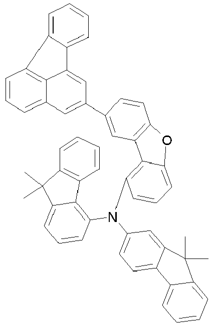 | 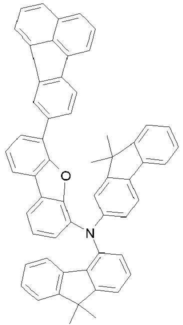 | 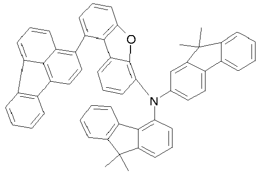 |
| | | |
| 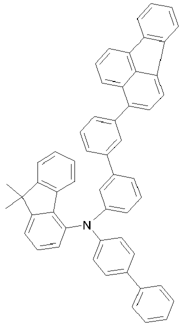 | 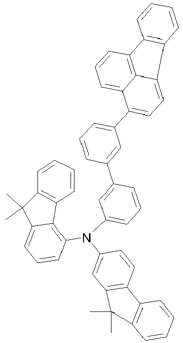 | 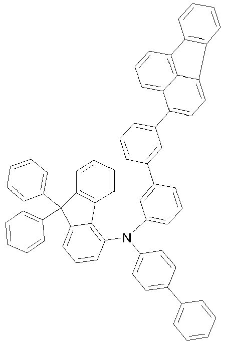 |
| 139 | 140 | 141 |
| 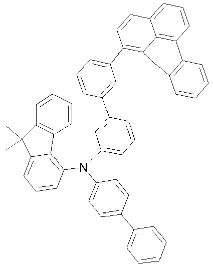 | 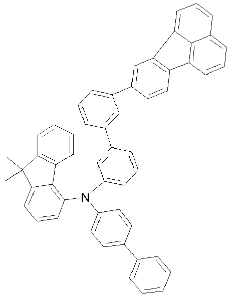 | 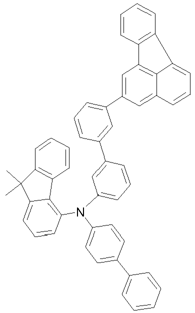 |
| 142# | 143# | 144# |
| 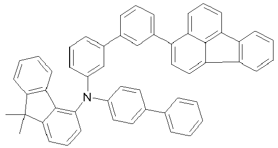 | 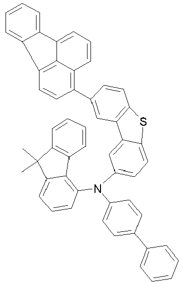 | 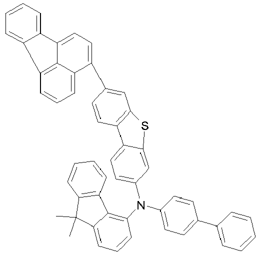 |
| 145 | 146 | 147 |
| 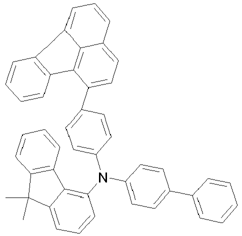 | 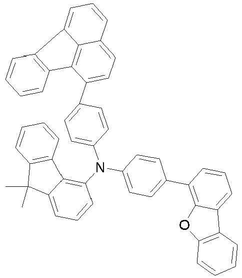 | 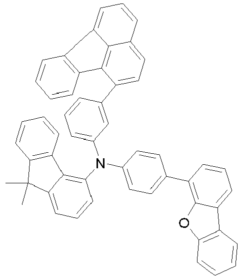 |
| 148# | 149# | 150# |
| 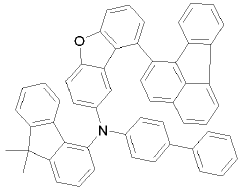 | 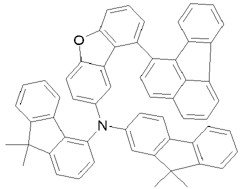 | 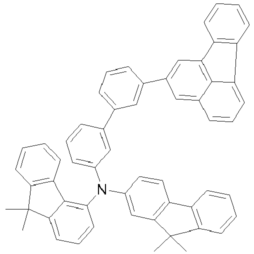 |
| 151# | 152# | 153# |
| 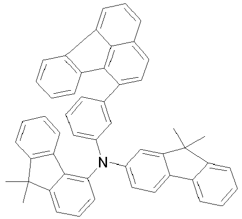 | 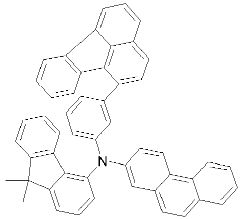 | 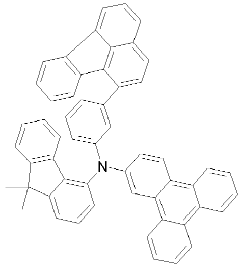 |
| 154# | 155# | 156# |
| 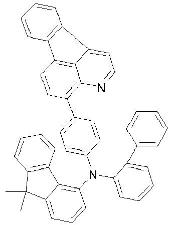 | 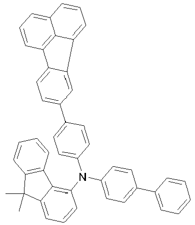 | 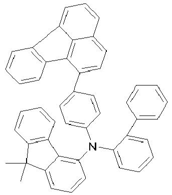 |
| 157 | 158# | 159# |
| 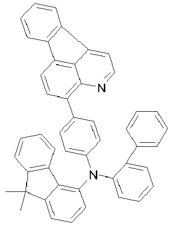 | 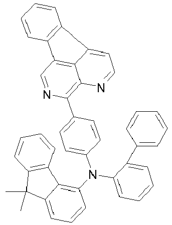 | 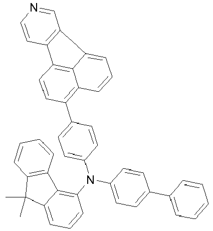 |
| 160 | 161 | 162 |
| 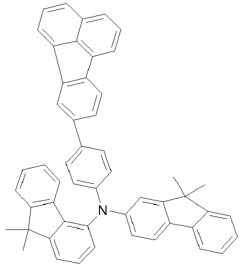 | 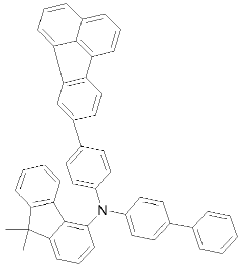 | 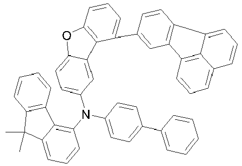 |
| 163# | 164# | 165# |
| 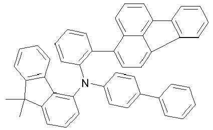 | 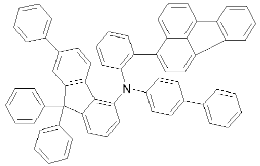 | 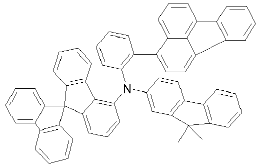 |
| 166 | 167 | 168 |
| 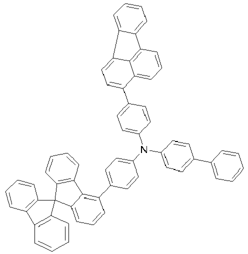 | 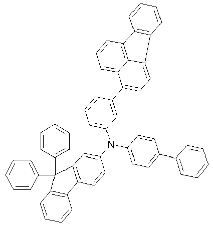 | 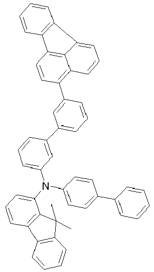 |
| 169 | 170 | 171 |
| 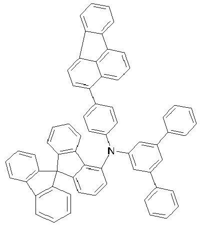 | 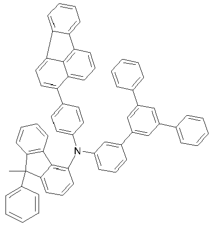 | 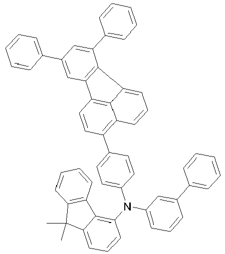 |
| 172 | 173 | 174 |
| 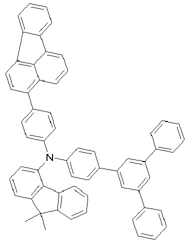 | 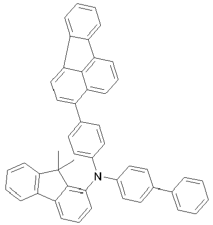 | 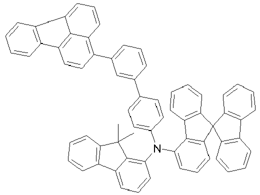 |
| 175 | 176 | 177 |
| 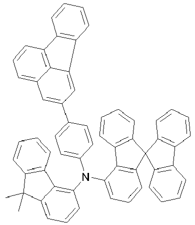 | 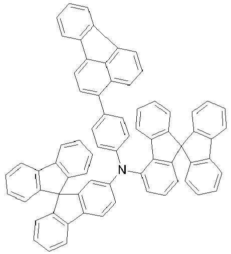 | 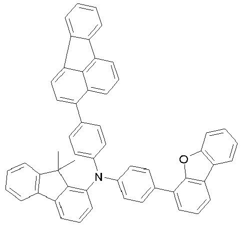 |
| 178# | 179 | 180 |
| 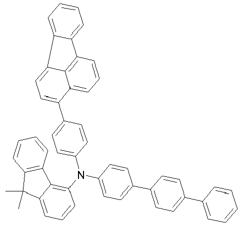 | 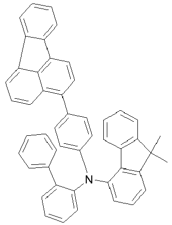 | 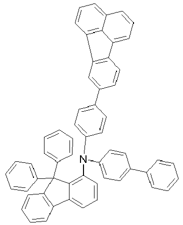 |
| 181 | 182 | 183# |
| 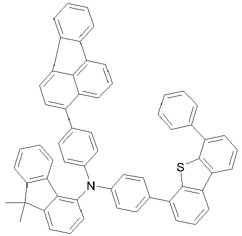 | 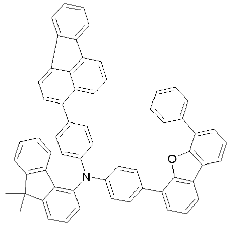 | 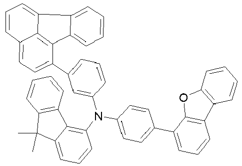 |
| 184 | 185 | 186# |
| 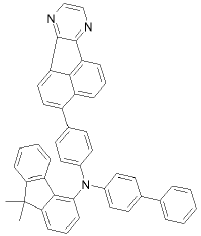 | 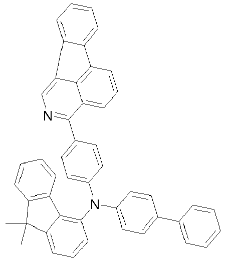 | 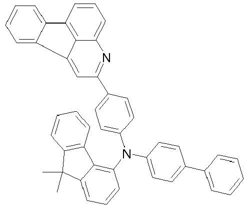 |
| 187 | 188 | 189# |
| 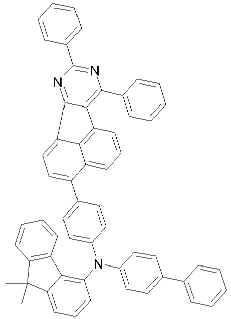 | 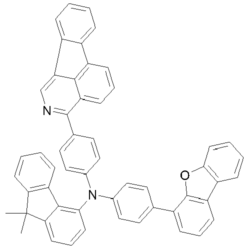 | 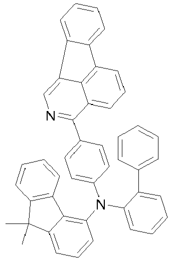 |
| 190 | 191 | 192 |
| 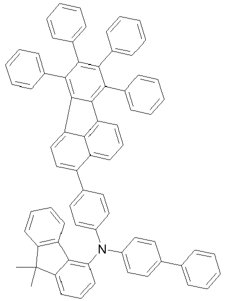 | 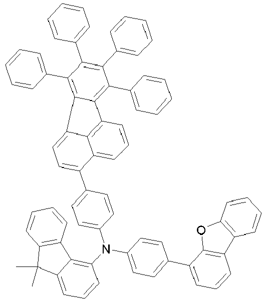 | 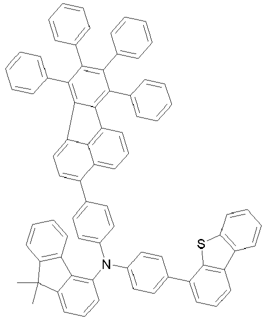 |
| 193 | 194 | 195 |
| 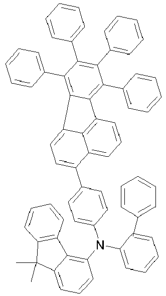 | 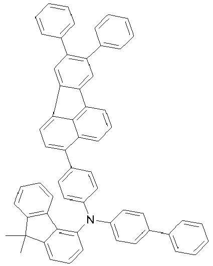 | 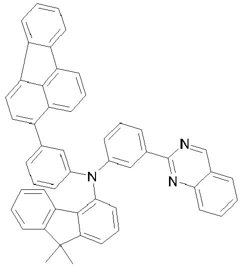 |
| 196 | 197 | 198 |
| 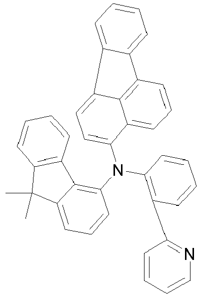 | 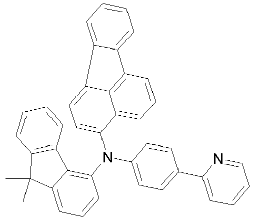 | 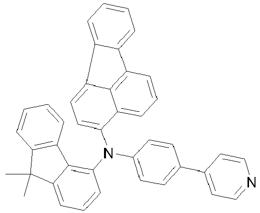 |
| 199# | 200# | 201# |
| 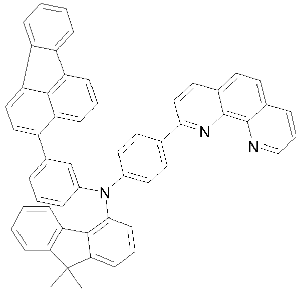 | 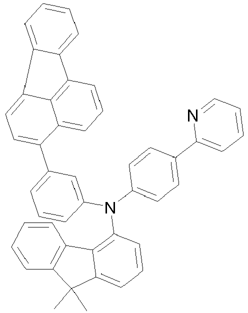 | 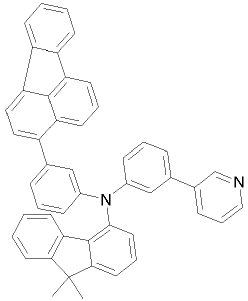 |
| 202 | 203 | 204 |
| 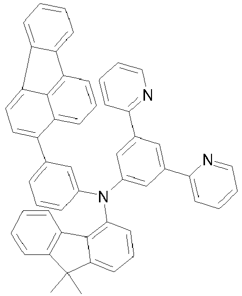 | 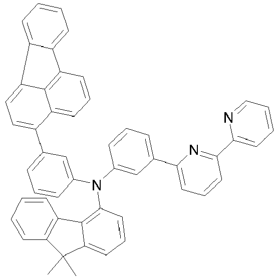 | 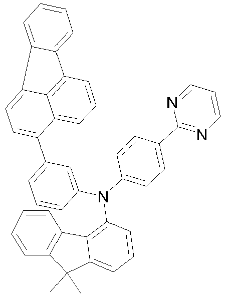 |
| 205 | 206 | 207 |
| 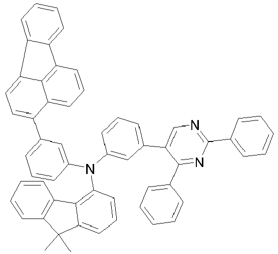 | 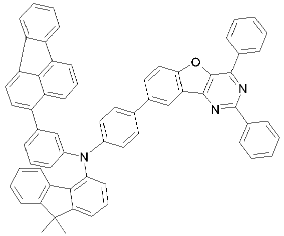 | 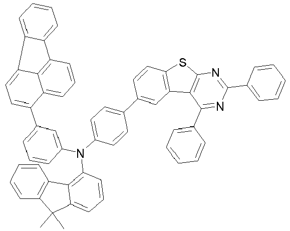 |
| 208 | 209 | 210 |
| 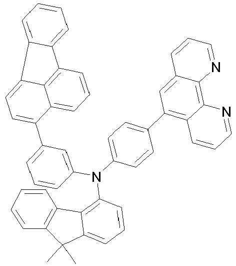 | 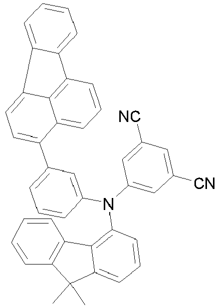 | 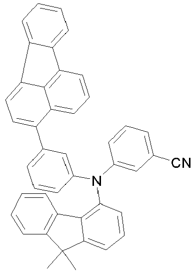 |
| 211 | 212 | 213 |
| 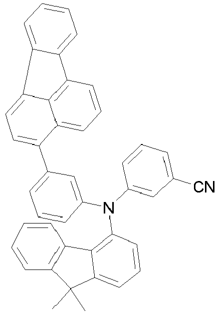 | 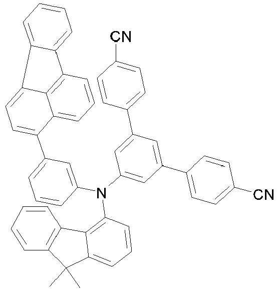 | |
| 214 | 215 | |

Die Verbindungen der Formel (I) können gemäß üblichen Verfahren der organischen Synthesechemie, die dem Fachmann bekannt sind, hergestellt werden. Bei der Herstellung der Verbindungen werden insbesondere übergangsmetallkatalysierte Kupplungsreaktionen, wie Buchwald-Kupplungsreaktionen und Suzuki-Kupplungsreaktionen eingesetzt.

Zur Herstellung von Verbindungen gemäß Formel (I), bei denen die Fluoranthenylgruppe direkt an das Amino-Stickstoffatom gebunden ist, wird bevorzugt nach dem folgenden Verfahren vorgegangen (Schema 1, Verfahren nicht anspruchsgemäß). Dabei kann anstelle einer Fluorenylgruppe gleichermaßen eine Spirobifluorenylgruppe eingesetzt werden. 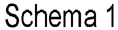 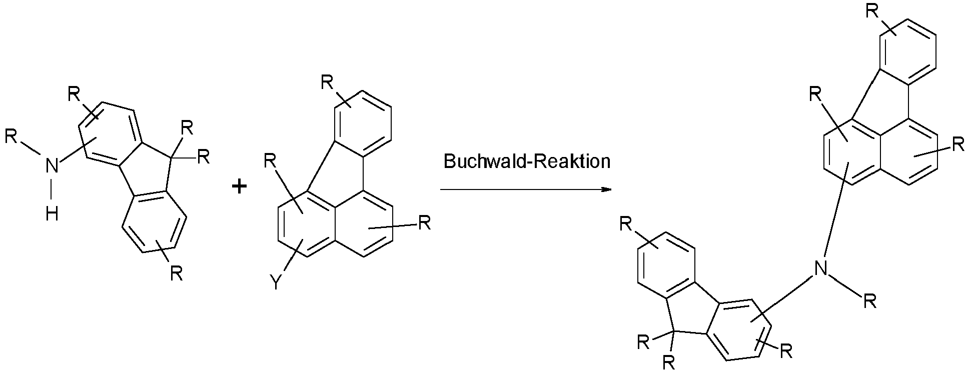
R = optionaler organischer Rest
Y = reaktive Gruppe, bevorzugt Cl, Br oder I

Dabei wird ein Fluorenyl-Amin in einer Buchwald-Reaktion mit einem Fluoranthenyl-Derivat umgesetzt, das eine reaktive Gruppe trägt. Dabei wird eine Verbindung gemäß Formel (I) erhalten.

Zur Herstellung von Verbindungen gemäß Formel (I), bei denen die Fluoranthenylgruppe über einen aromatischen Linker mit dem Amino-Stickstoffatom verbunden ist, wird bevorzugt nach einem der beiden folgenden Verfahren vorgegangen (Schema 2 und 3). Dabei kann anstelle einer Fluorenylgruppe gleichermaßen eine Spirobifluorenylgruppe eingesetzt werden. 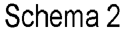 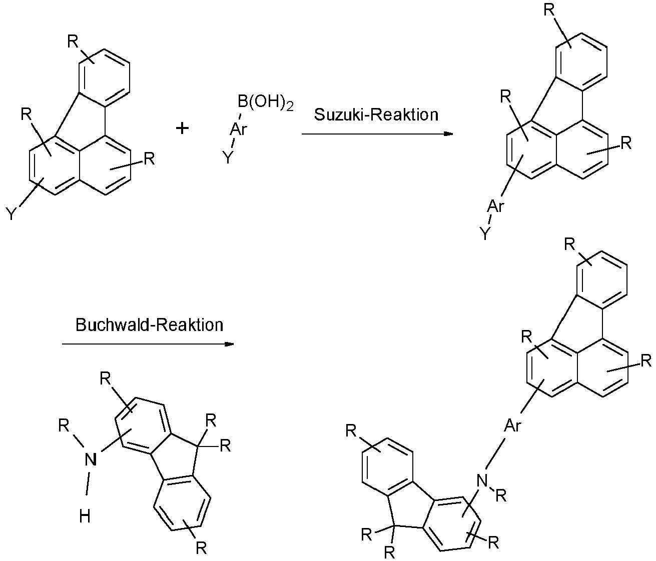
R = optionaler organischer Rest
Y = reaktive Gruppe, bevorzugt Cl, Br oder I
Ar = Arylen- oder Heteroarylengruppe

Dabei wird ein Fluoranthenyl-Derivat, das eine reaktive Gruppe trägt, in einer Suzuki-Reaktion mit einer Arylgruppe umgesetzt, die eine weitere reaktive Gruppe trägt. Dadurch wird eine Aryl-substituierte Fluoranthenylgruppe hergestellt. Diese wird anschließend in einer Buchwald-Reaktion mit einem Fluorenyl-Amin zu einer Verbindung gemäß Formel (I) umgesetzt. 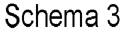 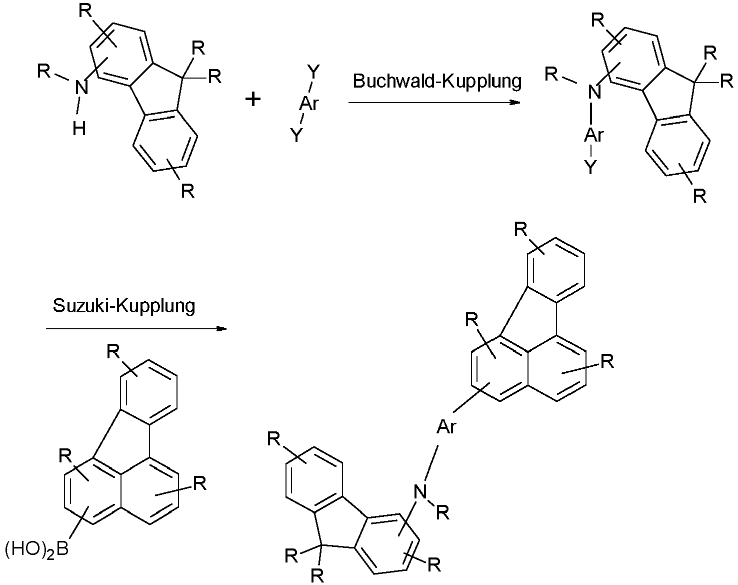
R = optionaler organischer Rest
Y = reaktive Gruppe, bevorzugt Cl, Br oder I
Ar = Arylen- oder Heteroarylengruppe

Gemäß der Variante von Schema 3 ist die Reihenfolge der Schritte Suzuki-Kupplung und Buchwald-Kupplung gegenüber der Variante von Schema 2 vertauscht. Dabei wird zunächst in einer Buchwald-Kupplung am Fluorenyl-Amin eine Arylgruppe mit reaktiver Gruppe an das Amin gebunden. Dann wird in einer folgenden Reaktion in einer Suzuki-Kupplung die Fluoranthenyl-Gruppe eingeführt.

Weiterer Gegenstand der vorliegenden Erfindung ist damit ein Verfahren zur Herstellung einer Verbindung gemäß Formel (I), welches dadurch gekennzeichnet ist, dass ein Fluorenyl-Amin in einer Buchwald-Kupplungsreaktion mit einer aromatischen oder heteroaromatischen Verbindung umgesetzt wird.

Gemäß einer bevorzugten Ausführungsform der Erfindung umfasst die genannte aromatische oder heteroaromatische Verbindung eine Fluoranthenylgruppe. Gemäß einer alternativen, ebenfalls bevorzugten Ausführungsform der Erfindung umfasst die genannte aromatische oder heteroaromatische Verbindung keine Fluoranthenylgruppe. In diesem Fall wird bevorzugt in einer folgenden Suzuki-Reaktion eine Fluoranthenylgruppe eingeführt.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß Formel (I), wobei die Bindung(en) zum Polymer, Oligomer oder Dendrimer an beliebigen, in Formel (I) mit R¹, R², R³, R⁴ oder R⁵ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung enthaltend die Verbindung der Formel (I) ist bevorzugt die folgende: Anode-Lochinjektionsschicht-Lochtransportschicht-wahlweise weitere Lochtransportschicht(en)-wahlweise Elektronenblockierschichtemittierende Schicht-wahlweise Lochblockierschicht-Elektronentransportschicht-Elektroneninjektionsschicht-Kathode. Es können zusätzlich weitere Schichten in der OLED vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen (für den prinzipiellen Aufbau siehe z. B. WO 2005/011013). Die erfindungsgemäßen Verbindungen sind dabei bevorzugt in einer Lochtransportschicht, Lochinjektionsschicht, Elektronenblockierschicht, emittierenden Schicht, lochblockierenden Schicht und/oder elektronentransportierenden Schicht vorhanden, besonders bevorzugt in einer emittierenden Schicht als Matrixmaterial, in einer Lochblockierschicht und/oder in einer Elektronentransportschicht.

Es ist erfindungsgemäß bevorzugt, wenn die Verbindung gemäß Formel (I) in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Lochtransportschicht, einer Elektronenblockierschicht, einer Lochinjektionsschicht, einer emittierenden Schicht, einer Lochblockierschicht, und/oder einer Elektronentransportschicht enthalten sein. Besonders bevorzugt ist sie in einer emittierenden Schicht in Kombination mit einer phosphoreszierenden emittierenden Verbindung enthalten.

Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierenden Verbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen

Elektrolumineszenzvorrichtungen einsetzen. Weitere Beispiele sind in der folgenden Tabelle aufgeführt:

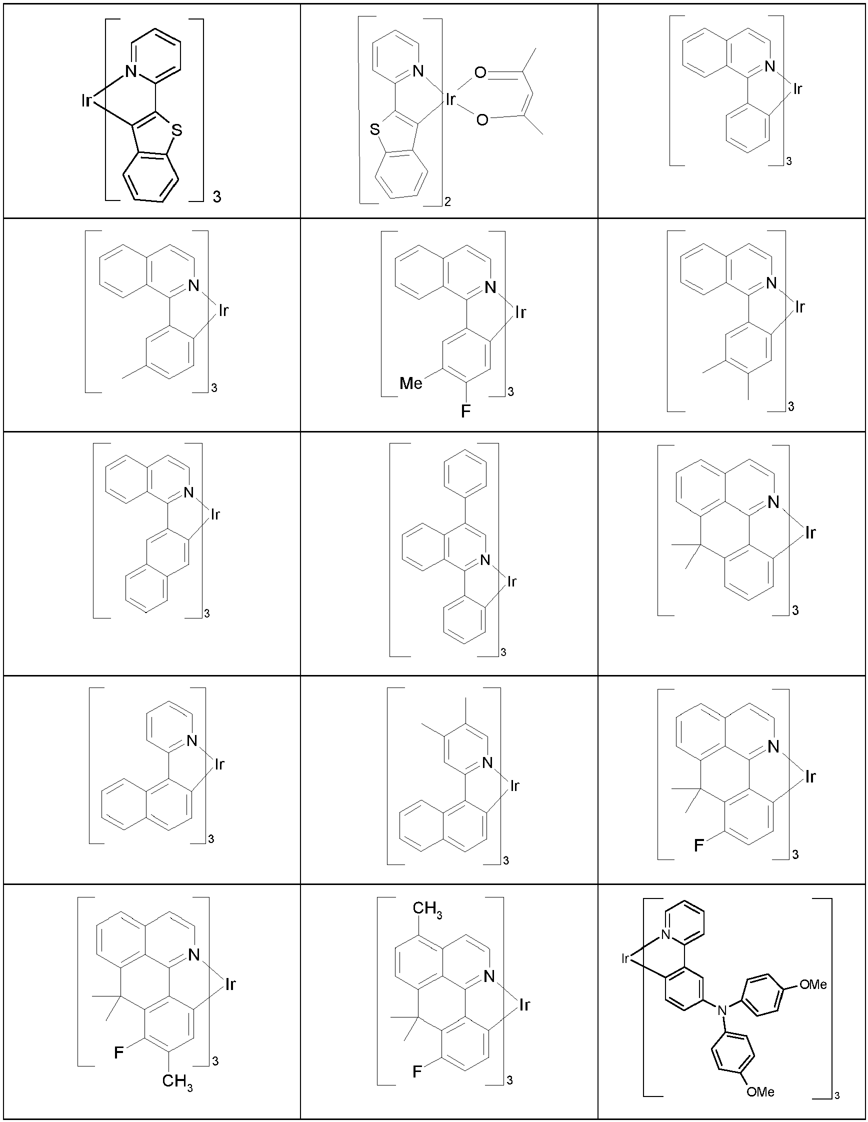

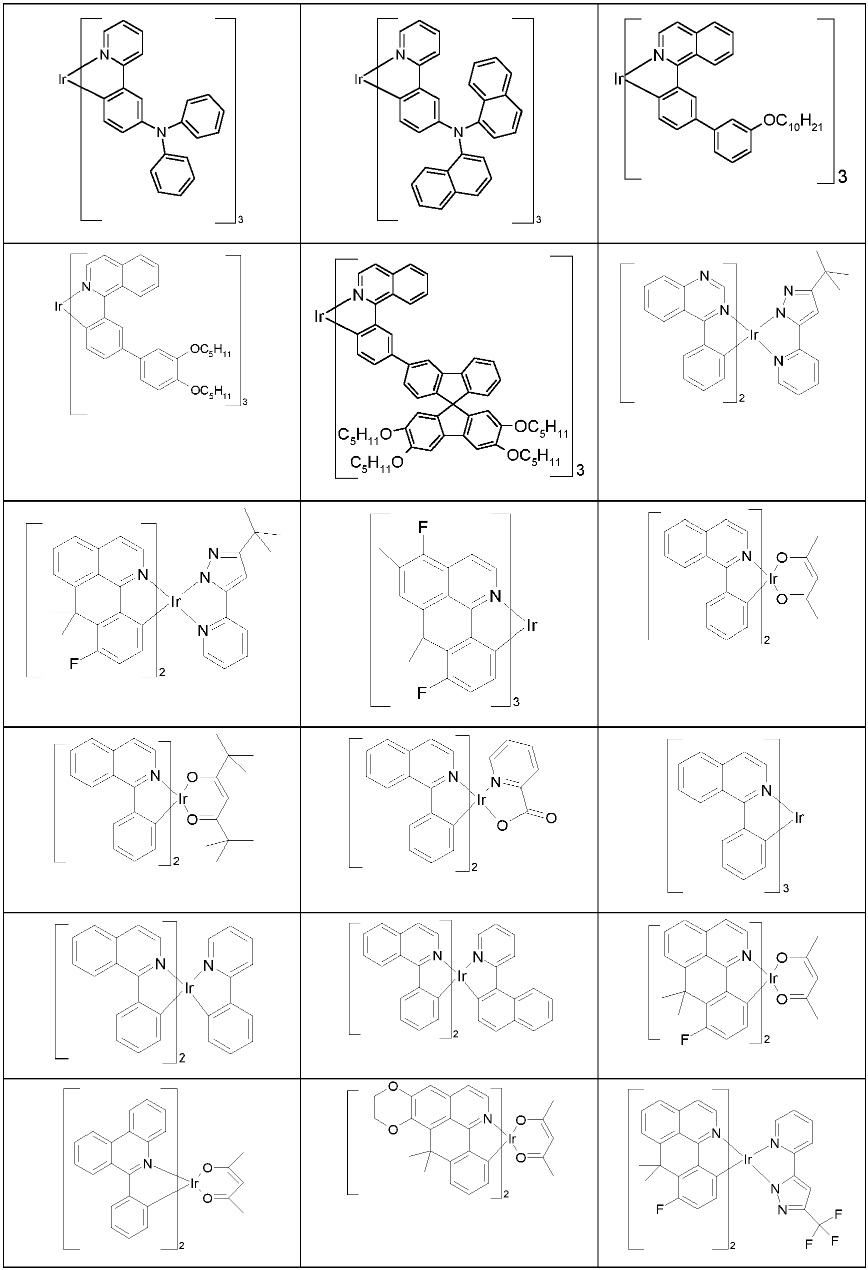

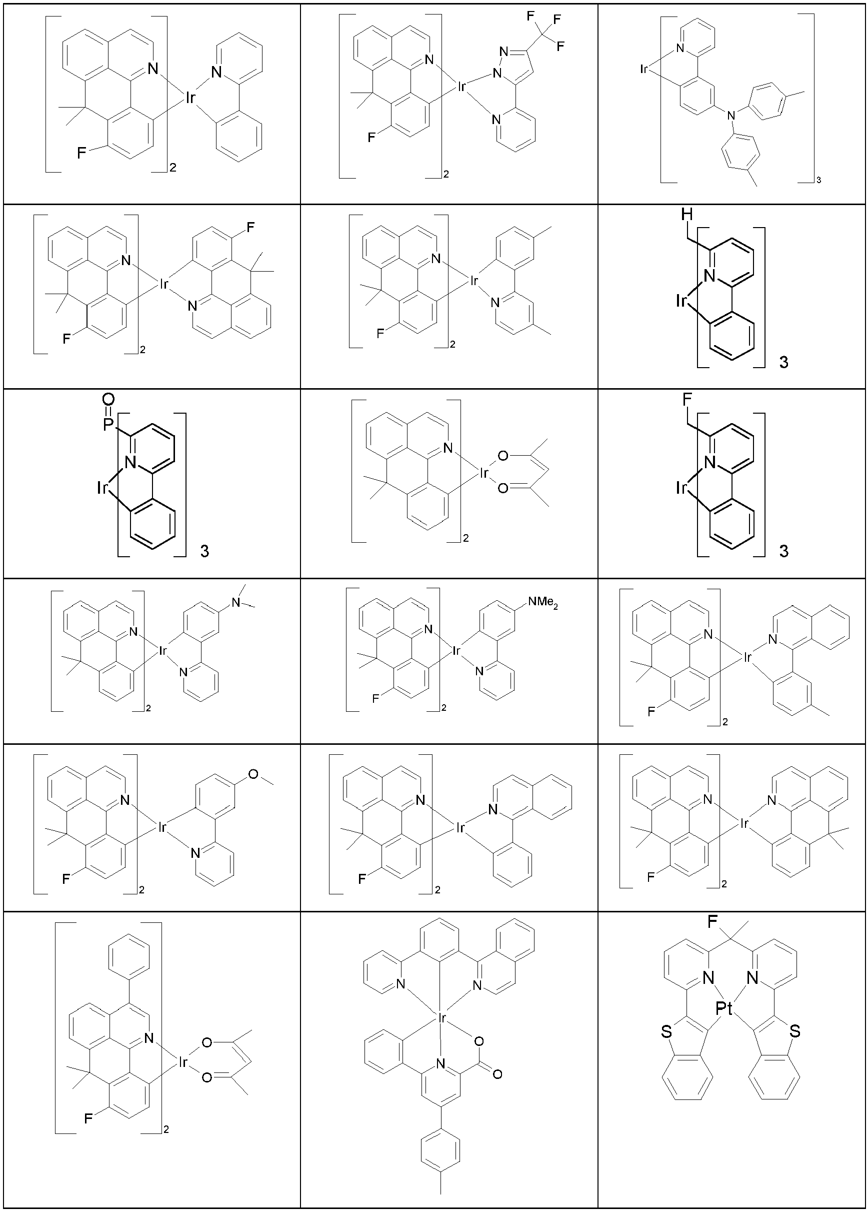

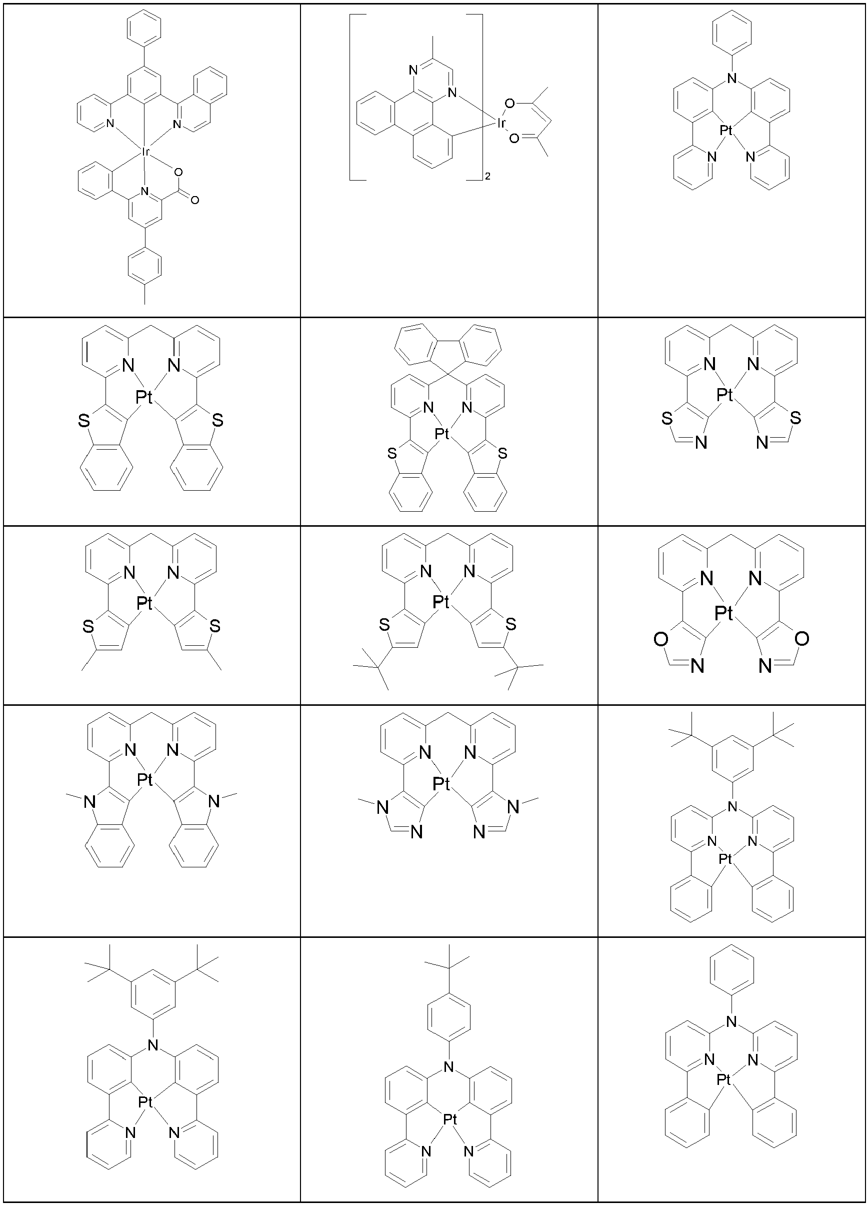

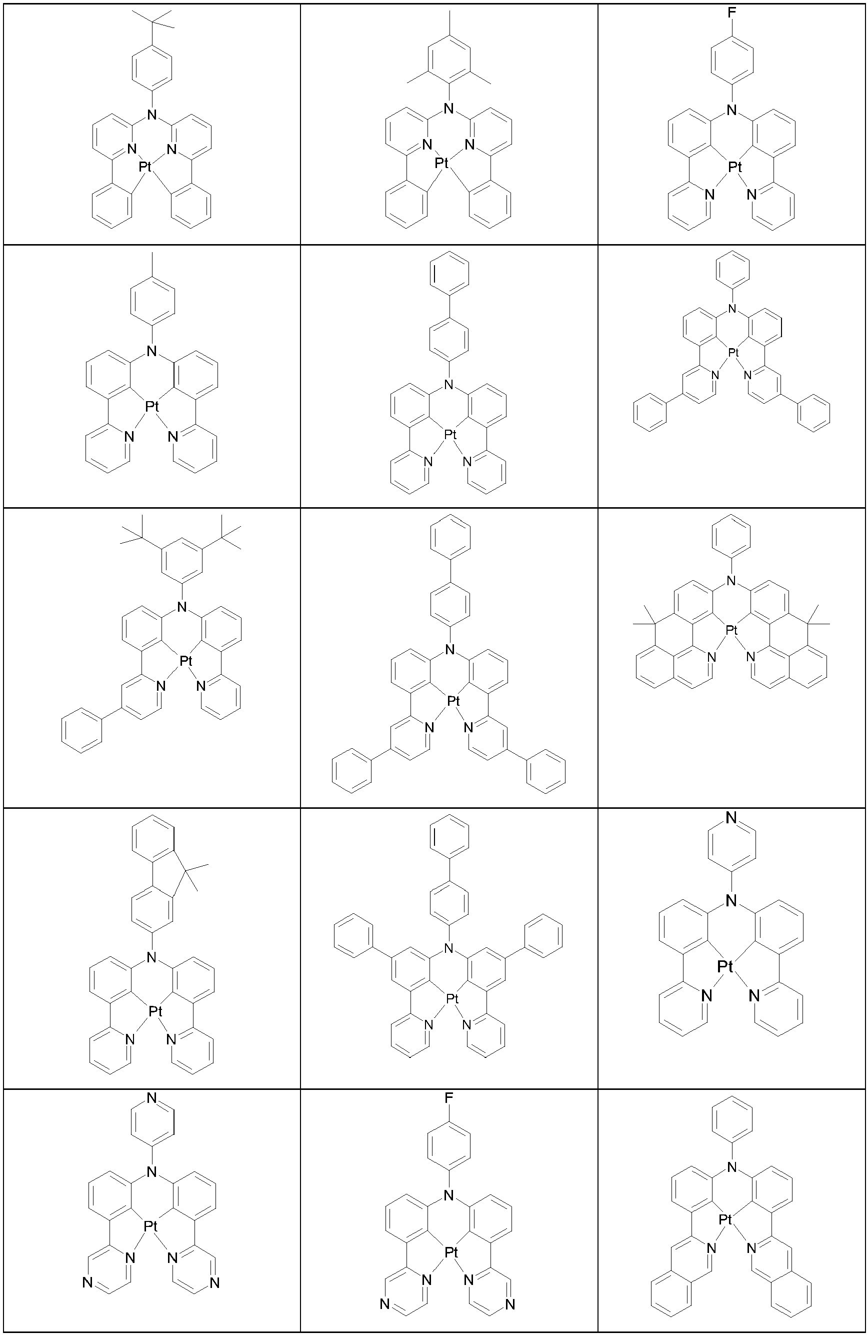

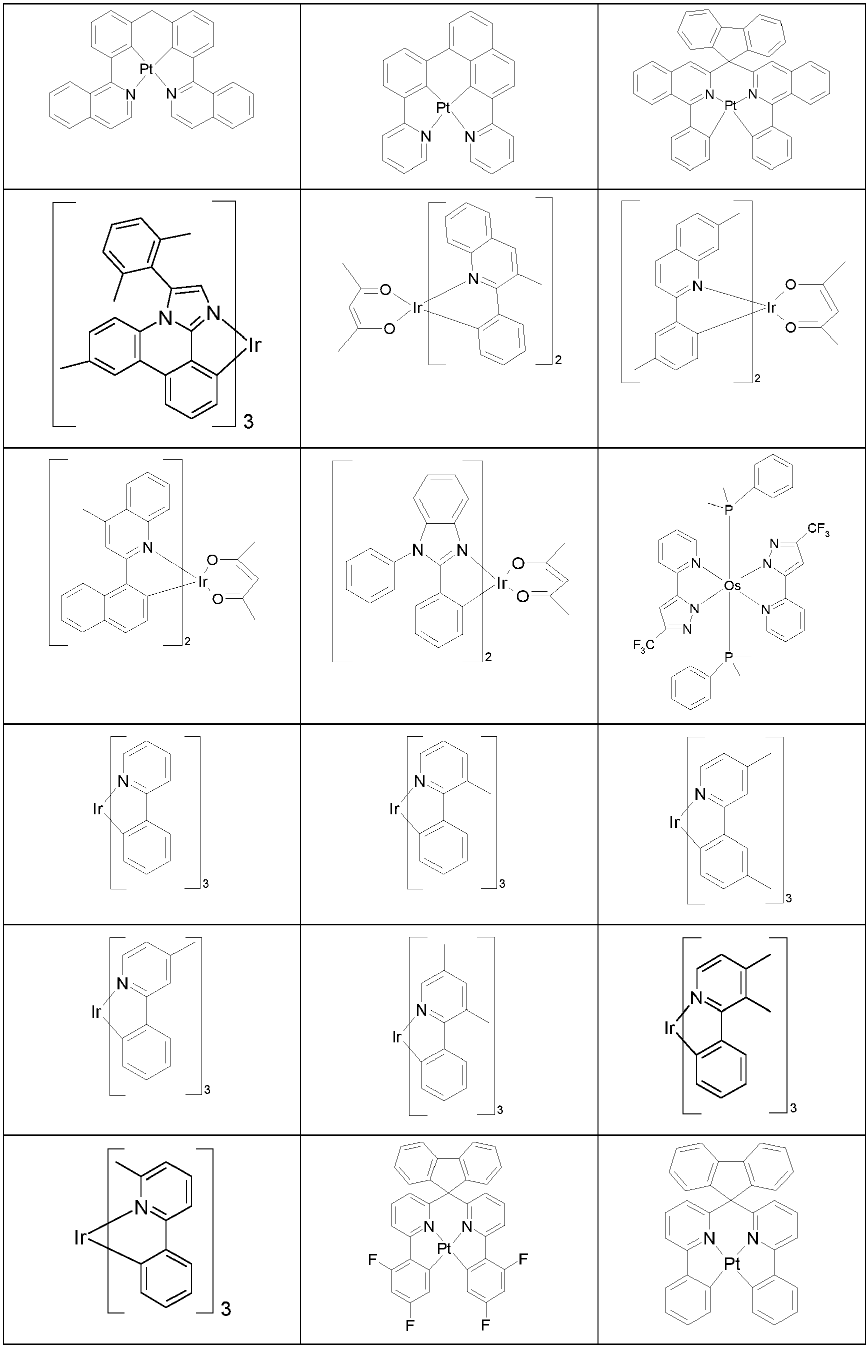

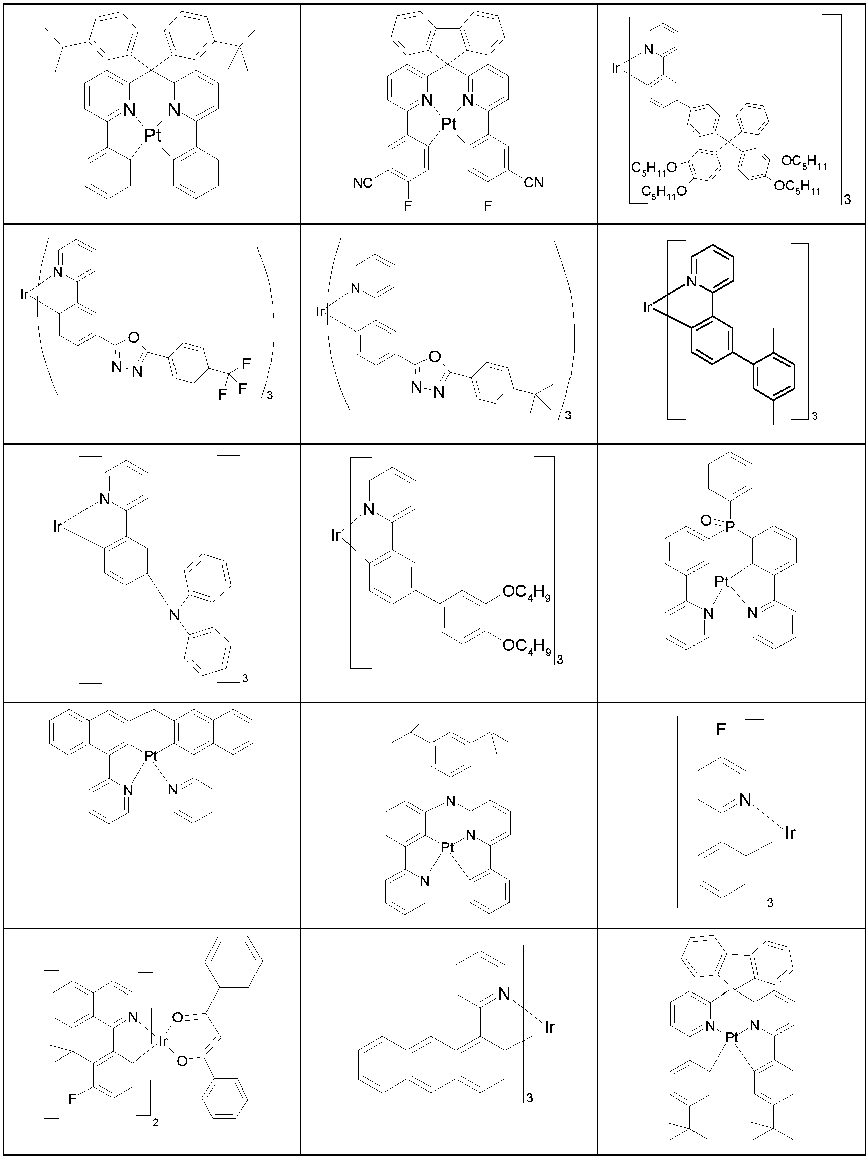

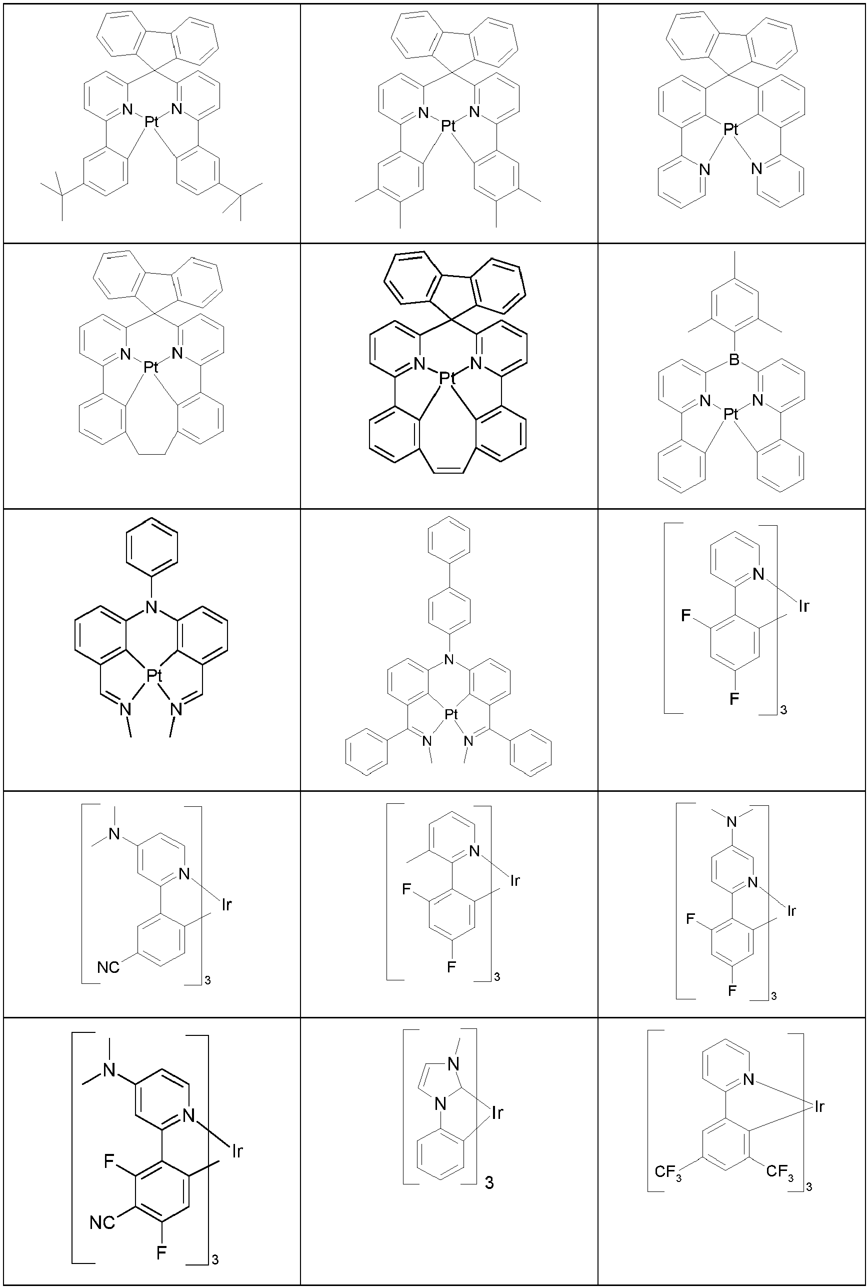

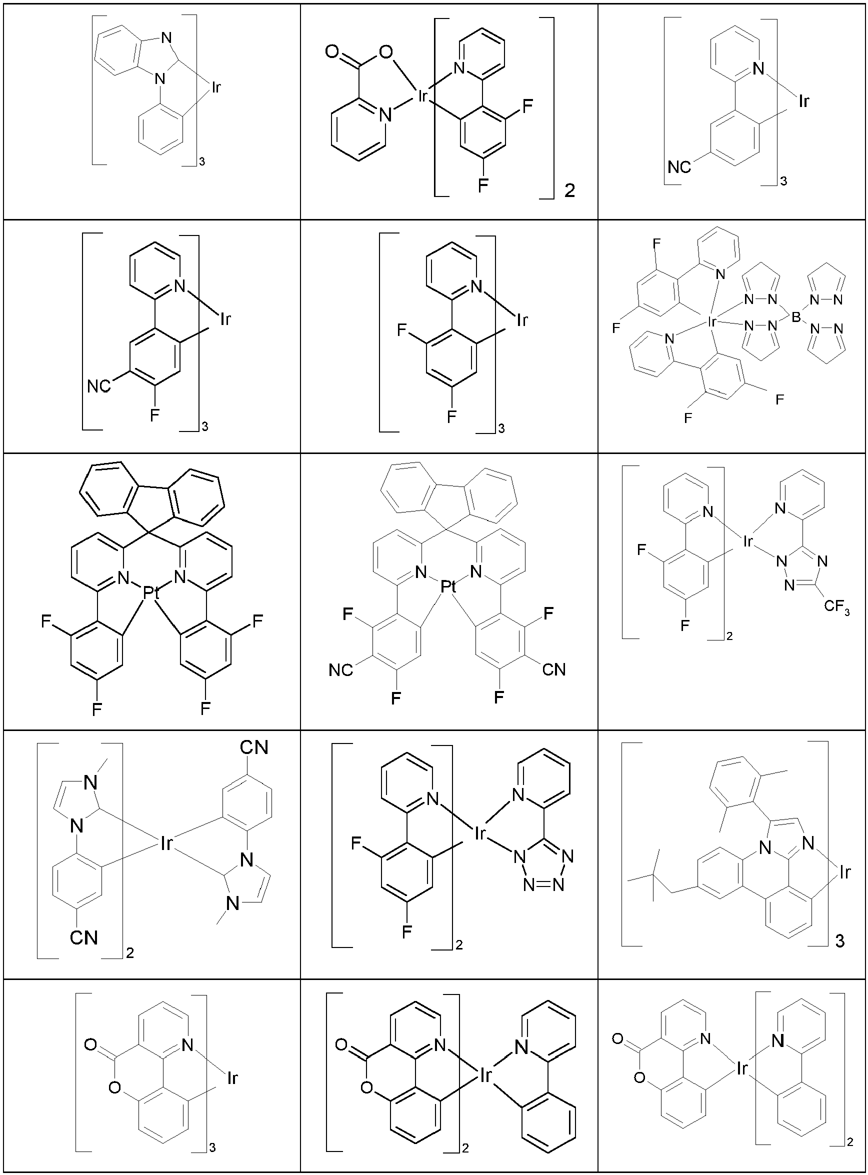

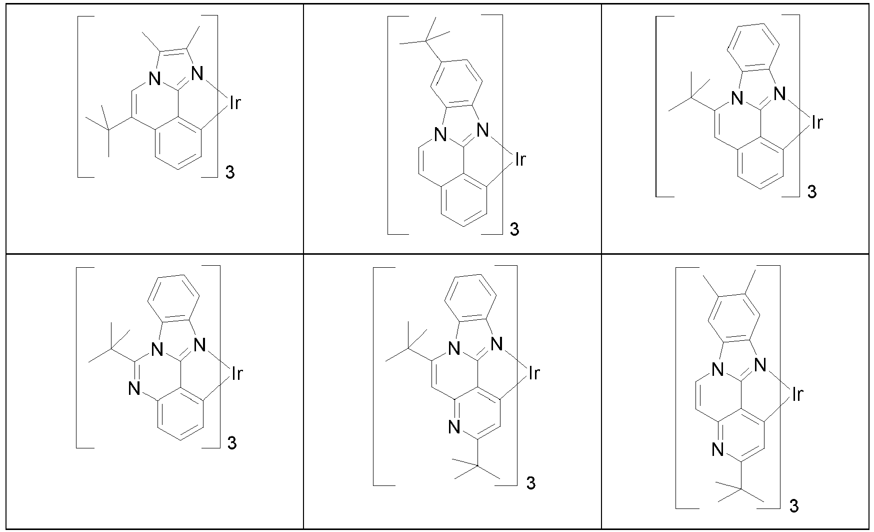

In einer bevorzugten Ausführungsform der vorliegenden Erfindung wird die Verbindung der Formel (I) in einer emittierenden Schicht als Matrixmaterial in Kombination mit einer oder mehreren emittierenden Verbindungen, vorzugsweise phosphoreszierenden emittierenden Verbindungen, eingesetzt. Die phosphoreszierende emittierende Verbindung ist dabei bevorzugt ein rot phosphoreszierender Emitter. Dies ist die am stärksten bevorzugte Verwendung der Verbindungen der Formel (I).

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) als eine Komponente von Mixed-Matrix-Systemen verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I) stellt dabei bevorzugt das Matrixmaterial mit lochtransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen oder den bevorzugten Matrixmaterialien für fluoreszierende emittierende Verbindungen, je nachdem welche Art von emittierender Verbindung im mixed-Matrix-System eingesetzt wird.

Bevorzugte phosphoreszierende emittierende Verbindungen zur Verwendung in Mixed-Matrix-Systemen sind dieselben, wie sie weiter oben als allgemein bevorzugte phosphoreszierende Emittermaterialien aufgeführt wurden.

In einer bevorzugten Ausführungsform der Erfindung werden die Verbindungen gemäß Formel (I) als lochtransportierendes Material eingesetzt. Die Verbindungen liegen dann bevorzugt in einer Lochtransportschicht, einer Elektronenblockierschicht oder einer Lochinjektionsschicht vor.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt. Bevorzugt enthält die erfindungsgemäße OLED zwei, drei oder vier lochtransportierende Schichten zwischen Anode und emittierender Schicht, von denen bevorzugt mindestens eine eine Verbindung gemäß Formel (I) enthält, besonders bevorzugt genau eine oder zwei eine Verbindung gemäß Formel (I) enthalten.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 und DE 102012209523 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden insbesondere die folgenden Verbindungen:

| | | |
|---|---|---|
| 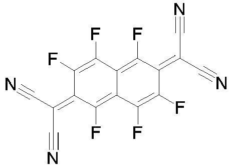 | 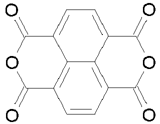 | 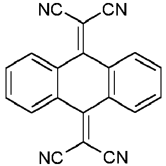 |
| (D-1) | (D-2) | (D-3) |
| 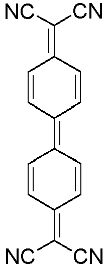 | 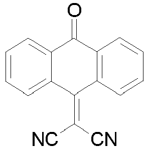 | 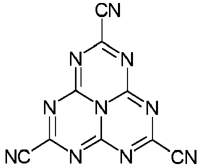 |
| (D-4) | (D-5) | (D-6) |
| 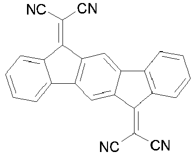 | 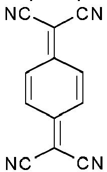 | 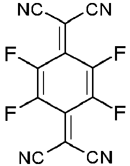 |
| (D-7) | (D-8) | (D-9) |
| 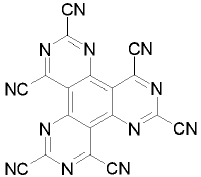 | 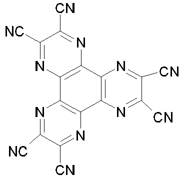 | 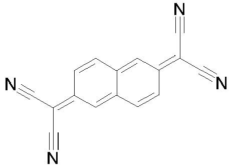 |
| (D-10) | (D-11) | (D-12) |
| 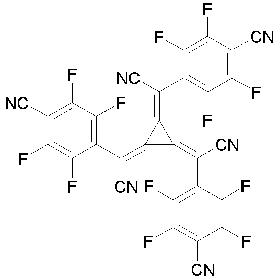 | | |
| (D-13) | | |

In einer weiteren bevorzugten Ausführungsform der Erfindung wird die Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

Gemäß einer weiteren bevorzugten Ausführungsform werden die erfindungsgemäßen Verbindungen in einer oder mehreren Schichten auf der Elektronentransportseite eingesetzt, bevorzugt in einer Loochblockierschicht und/oder einer Elektronentransportschicht. Bei der Verwendung in einer Elektronentransportschicht ist es bevorzugt, dass sie in Kombination mit einem Metallkomplex, bevorzugt einem Metall-Chinolinat, besonders bevorzugt einem Lithium-Chinolinat, eingesetzt werden.

Im Folgenden werden bevorzugte Ausführungsformen für die verschiedenen Funktionsmaterialien der elektronischen Vorrichtung aufgeführt.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, beispielsweise gemäß WO 2006/108497 oder WO 2006/122630, Benzoindenofluorenamine bzw. -diamine, beispielsweise gemäß

WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine, die in WO 2014/111269 und in WO 2017/036574 offenbarten erweiterten Benzoindenofluorene, die in WO 2017/028940 und WO 2017/028941 offenbarten Phenoxazine, und die in WO 2016/150544 offenbarten Fluoren-Derivate, die mit Furan-Einheiten oder mit Thiophen-Einheiten verbunden sind.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, die in EP 1749809, EP 1905754 und US 2012/0187826 offenbarten Pyren-Verbindungen, die in WO 2015/158409 offenbarten Benzanthracenyl-Anthracen-Verbindungen, die in WO 2017/025165 offenbarten Indeno-Benzofurane, und die in WO 2017/036573 offenbarten Phenanthryl-Anthracene.

Bevorzugte Matrixmaterialien für phosphoreszierende emittierende Verbindungen sind neben den Verbindungen der Formel (I) aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Bevorzugt umfasst die erfindungsgemäße OLED zwei oder mehr unterschiedliche lochtransportierende Schichten. Die Verbindung der Formel (I) kann dabei in einer oder in mehreren oder in allen lochtransportierenden Schichten eingesetzt werden. Gemäß einer bevorzugten Ausführungsform wird die Verbindung der Formel (I) in genau einer oder genau zwei lochtransportierenden Schichten eingesetzt, und in den weiteren vorhandenen lochtransportierenden Schichten werden andere Verbindungen eingesetzt, bevorzugt aromatische Aminverbindungen. Weitere Verbindungen, die neben den Verbindungen der Formel (I) bevorzugt in lochtransportierenden Schichten der erfindungsgemäßen OLEDs eingesetzt werden, sind insbesondere Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938, WO 2014/015935 und WO 2015/082056), Spiro-Dibenzopyran-Amine (z. B. gemäß WO 2013/083216), Dihydroacridin-Derivate (z. B. gemäß WO 2012/150001), Spirodibenzofurane und Spirodibenzothiophene, z.B. gemäß WO 2015/022051, WO 2016/102048 und WO 2016/131521, Phenanthren-Diarylamine, z.B. gemäß WO 2015/131976, Spiro-Tribenzotropolone, z.B. gemäß WO 2016/087017, Spirobifluorene mit meta-Phenyldiamingruppen, z.B. gemäß WO 2016/078738, Spiro-Bisacridine, zB. gemäß WO 2015/158411,

Xanthen-Diarylamine, z.B. gemäß WO 2014/072017, und 9,10-Dihydroanthracen-Spiroverbindungen mit Diarylaminogruppen gemäß WO 2015/086108.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele (nicht anspruchsgemäße

### Ausführungsbeispiele sind mit # gekennzeichnet)

### A) Synthesebeispiele

Die nachfolgenden Synthesen werden, sofern nicht anders angegeben, unter einer Schutzgasatmosphäre in getrockneten Lösungsmitteln durchgeführt.

### a) Biphenyl-4-yl-(9,9-dimethyl-9H-fluoren-1-yl)-amin (1a)

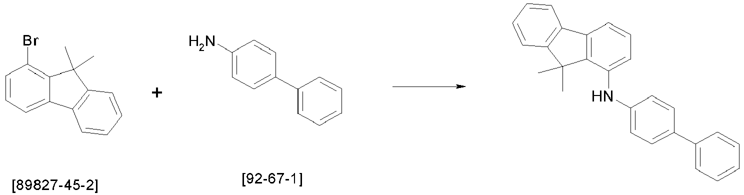

36 g (212 mmol, 1,0eq) 4-Aminobiphenyl werden zusammen mit 57,8 g (177 mmol, 1,0 eq) 1-Bromddimethylfluoren und 2,4 g (212 mmol, 1,20 eq) Natrium-*t*-pentoxid [14593-46-5] in 600ml absolutem Toluol vorgelegt und für 30 Minuten entgast. Anschließend werden 398 mg (1,77 mmol, 0,01 eq) Palladium(II)-acetat [3375-31-3] und 1,46 g (3,56 mmol, 0,02eq) 2-Dicyclohexylphosphino-2',6'-dimethoxybiphenyl SPHOS [657408-07-6] zugegeben und der Ansatz über Nacht unter Rückfluss erhitzt. Nach beendeter Reaktion wird der Ansatz auf Raumtemperatur abgekühlt und mit 500 ml Wasser extrahiert. Anschließend wird die wässrige Phase dreifach mit Toluol gewaschen, die vereinten organischen Phasen über Natriumsulfat getrocknet und das Lösungsmittel am Rotationsverdampfer entfernt. Der braune Rückstand wird in ca. 200 ml Toluol aufgenommen und über Silikagel filtriert. Zur weiteren Aufreinigung wird eine Umkristallisation aus Toluol/Heptan durchgeführt.

Ausbeute: 59 g (164 mmol), 79 % der Theorie.

Analog werden dargestellt:

| **Eintrag** | **Edukt 1** | **Edukt 2** | **Produkt 3** | **Ausbeute [%]** |
|---|---|---|---|---|
| 2a | 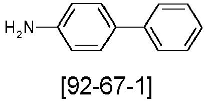 | 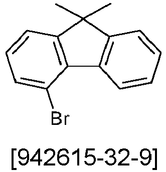 | 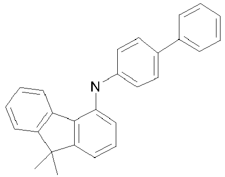 | 65 |
| 3a | 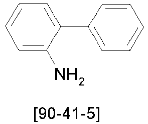 | 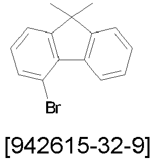 | 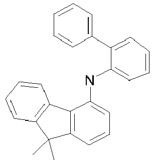 | 63 |
| 4a | 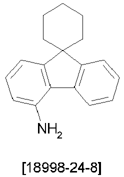 | 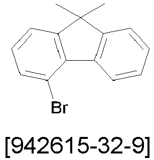 | 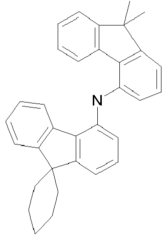 | 60 |
| 5a | 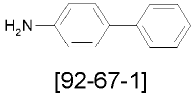 | 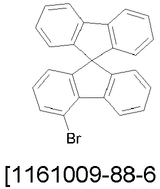 | 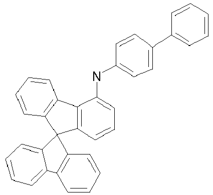 | 62 |
| 6a | 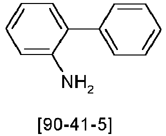 | 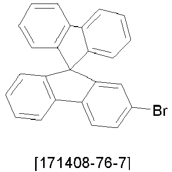 | 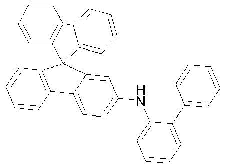 | 64 |
| 7a | 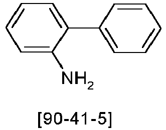 | 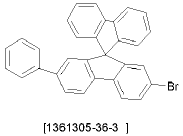 | 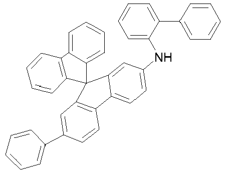 | 68 |
| 8a | 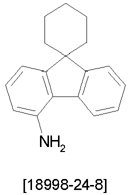 | 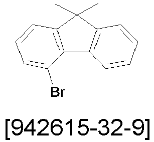 | 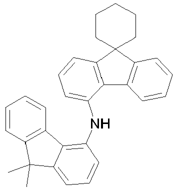 | 71 |
| 9a | 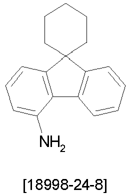 | 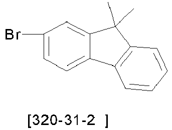 | 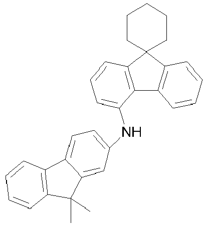 | 72 |
| 10a | 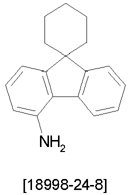 | 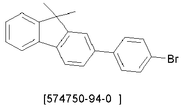 | 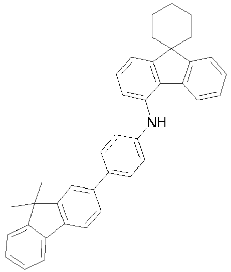 | 83 |
| 11a | 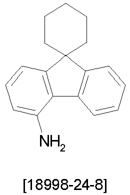 | 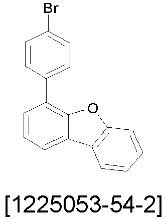 | 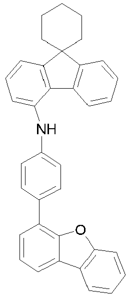 | 64 |
| 12a | 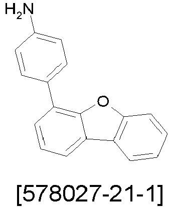 | 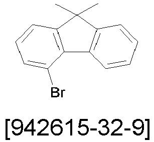 | 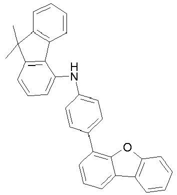 | 67 |
| 13a | 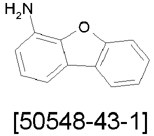 | 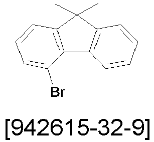 | 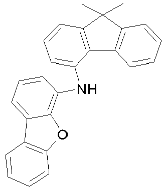 | 56 |
| 14a | 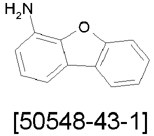 | 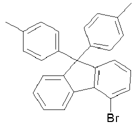 | 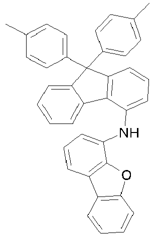 | 75 |
| 15a | 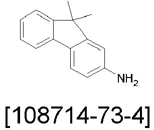 | 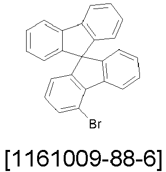 | 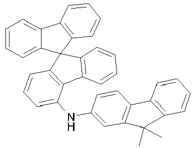 | 85 |
| 16a | 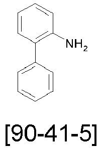 | 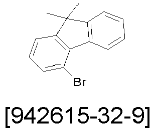 | 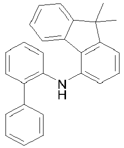 | 69 |
| 17a | 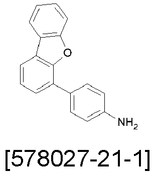 | 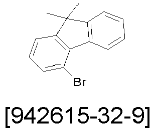 | 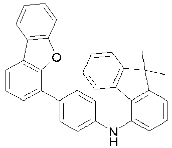 | 67 |
| 18a | 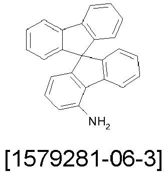 | 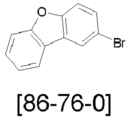 | 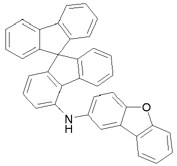 | 88 |
| 19a | 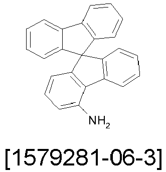 | 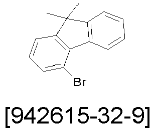 | 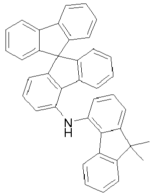 | 81 |
| 20a | 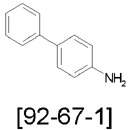 | 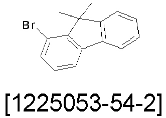 | 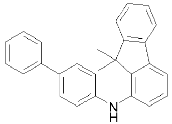 | 77 |
| 21a | 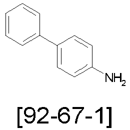 | 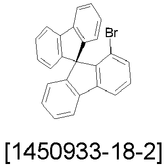 | 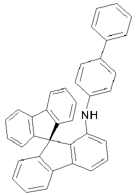 | 70 |
| 22a | 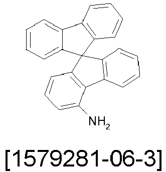 | 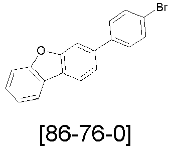 | 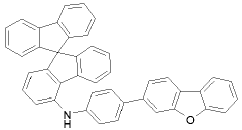 | 84 |
| 23a | 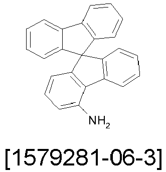 | 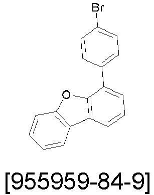 | 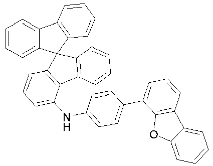 | 91 |
| 24a | 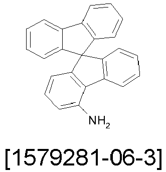 | 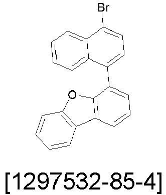 | 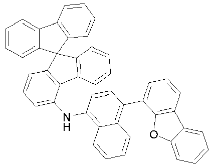 | 74 |
| 25a | 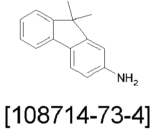 | 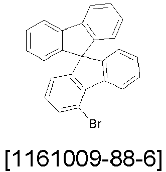 | 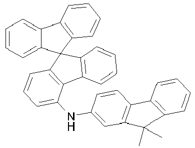 | 85 |
| 26a | 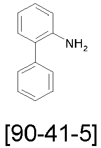 | 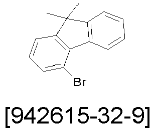 | 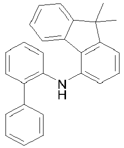 | 69 |
| 27a | 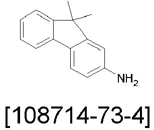 | 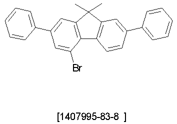 | 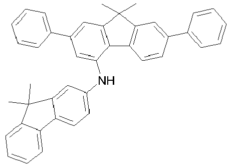 | 67 |
| 28a | 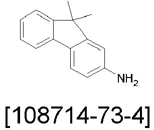 | 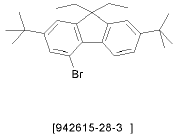 | 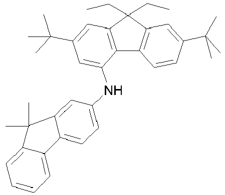 | 71 |
| 29a | 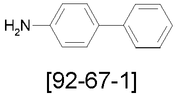 | 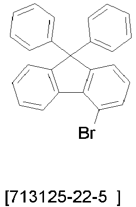 | 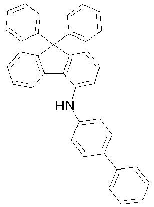 | 70 |

### b) Biphenyl-4-yl-(4-bromo-phenyl)-(9,9-dimethyl-9H-fluoren-4-yl)-amin (1b)

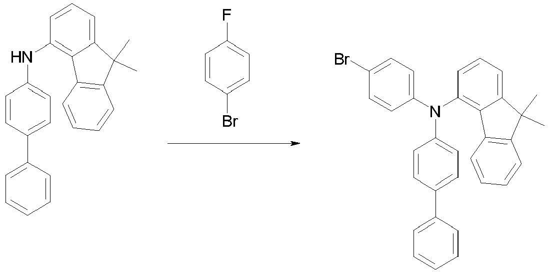

In einem 1L-Vierhalskolben werden 51.3g (142mmol, 1.00eq) Biphenyl-4-yl-(9,9-dimethyl-9H-fluoren-4-yl)-amin, sowie 75.6g (426mmol, 3.00eq) 1-Bromo-4-fluorobenzol [460-00-4] und 92.5g (284mmol, 2.00eq) Cäsiumcarbonat [534-17-8] vorgelegt und mit 500ml Dimethylacetamid versetzt. Das Reaktionsgemisch wird für drei Tage bei 150°C gerührt. Nach beendeter Reaktion wird der Ansatz auf Raumtemperatur abgekühlt und der Feststoff über Celite abfiltriert. Die Mutterlauge wird eingeengt und der ausgefallene Feststoff nach Filtration mit heißem Methanol ausgerührt. Ausbeute: 43 g (135 mmol), 95 % der Theorie.

Analog wurden dargestellt:

| **Eintrag** | **Edukt 3** | **Produkt 5** | **Ausbeute [%]** |
|---|---|---|---|
| 2b | 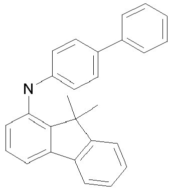 | 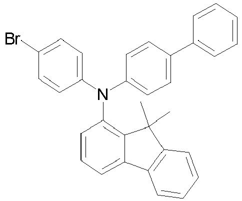 | 78 |
| 3b | 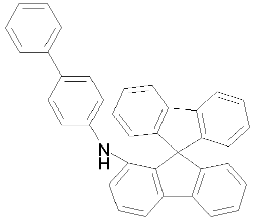 | 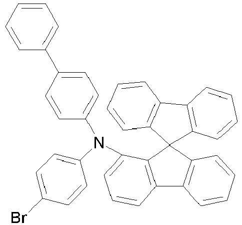 | 26 |
| 4b | 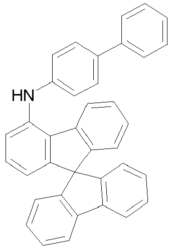 | 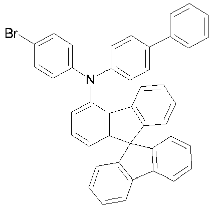 | 84 |
| 5b | 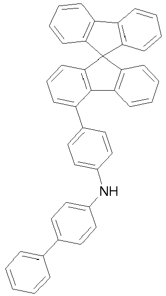 | 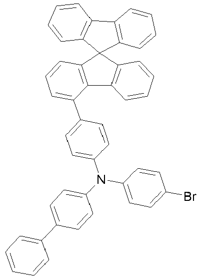 | 68 |
| 6b | 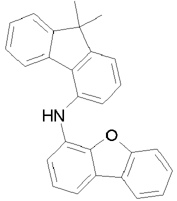 | 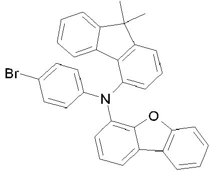 | 67 |
| 7b | 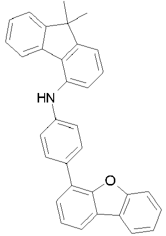 | 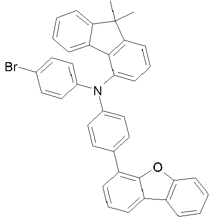 | 44 |
| 8d | 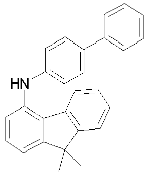 | 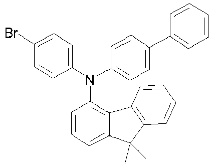 | 59 |
| 9d | 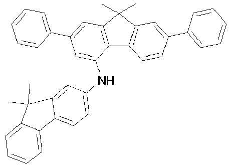 | 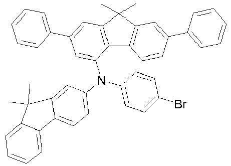 | 65 |
| 10b | 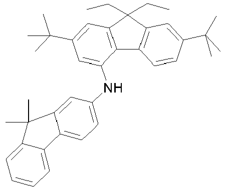 | 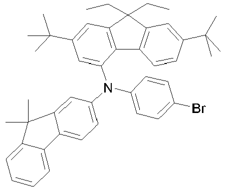 | 67 |
| 11b | 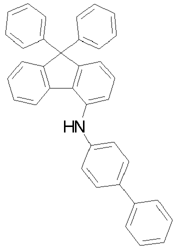 | 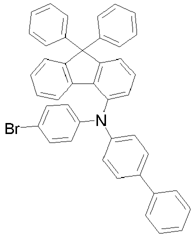 | 71 |
| 12b | 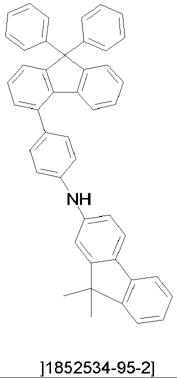 | 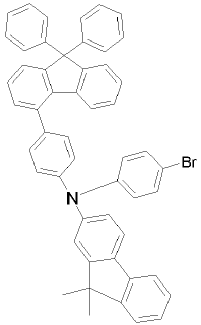 | 70 |

### c) Biphenyl-4-yl-(9,9-dimethyl-9H-fluoren-1-yl)-(4-fluoranthen-3-yl-phenyl)-amin (1)

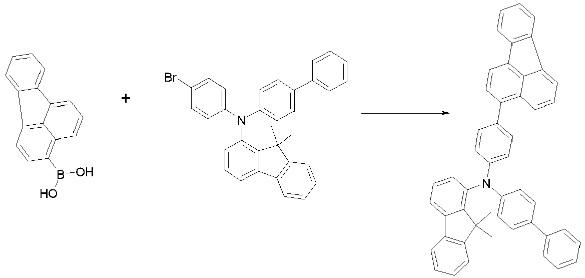

27 g (110.0 mmol) Fluranthen-3-boronsäure, 56 g (110.0 mmol) Biphenyl-4-yl-(4-bromo-phenyl)-(9,9-dimethyl-9H-fluoren-1-yl)-amin und 26 g (210.0 mmol) Natriumcarbonat werden in 500 mL Ethylenglycoldiaminether und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt.

Nach Erkalten wird die organische Phase abgetrennt, über Kieselgel filtriert, dreimal mit 200 mL Wasser gewaschen und anschließend zur Trockene eingeengt. Das Produkt wird via Säulenchromatographie an Kieselgel mit Toluol/Heptan (1:2) gereinigt und abschließend im Hochvakuum (p = 5 × 10⁻⁷ mbar) sublimiert (Reinheit 99,9%). Die Ausbeute beträgt 56 g (88 mmol), entsprechend 80 % der Theorie.

Analog dazu werden die folgenden Verbindungen hergestellt:

| | **Edukt 1** | **Edukt 2** | **Produkt** | **Ausbeute [%]** |
|---|---|---|---|---|
| 2# | 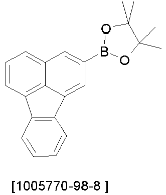 | 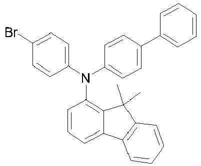 | 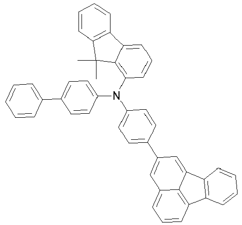 | 70 |
| 3 | 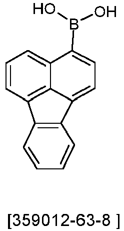 | 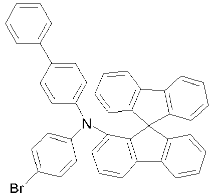 | 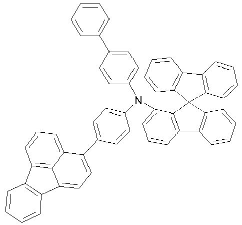 | 74 |
| 4# | 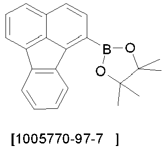 | 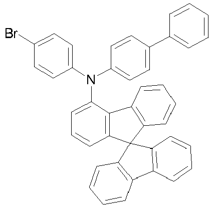 | 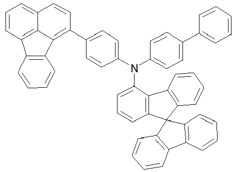 | 70 |
| 5# | 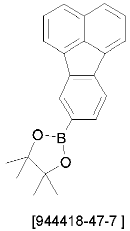 | 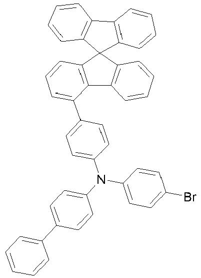 | 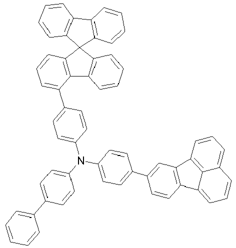 | 64 |
| 6 | 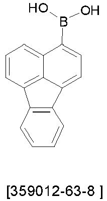 | 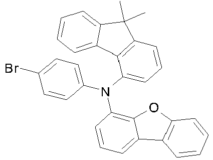 | 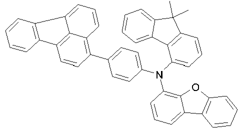 | 71 |
| 7 | 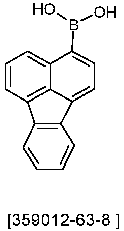 | 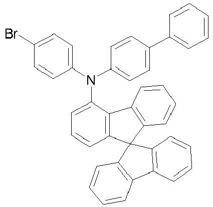 | 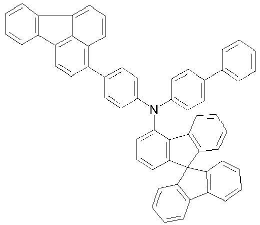 | 64 |
| 8 | 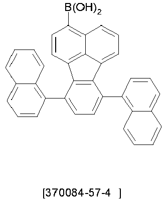 | 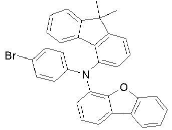 | 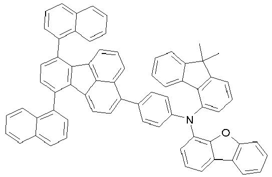 | 67 |
| 9 | 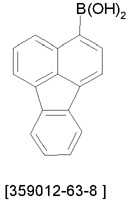 | 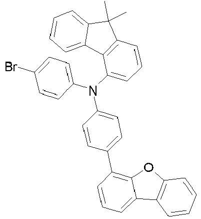 | 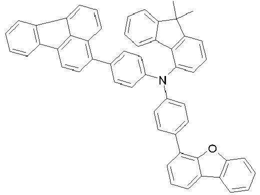 | 68 |
| 10# | 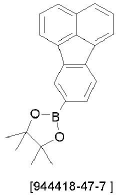 | 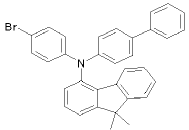 | 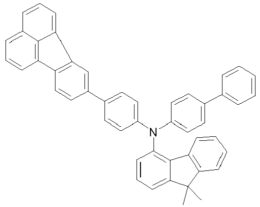 | 73 |
| 11 | 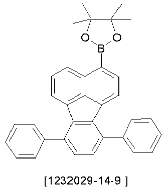 | 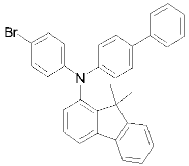 | 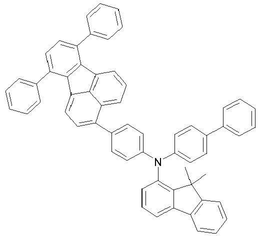 | 66 |
| 12# | 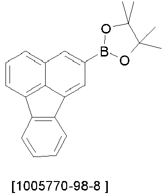 | 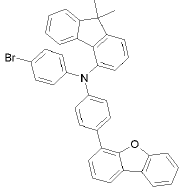 | 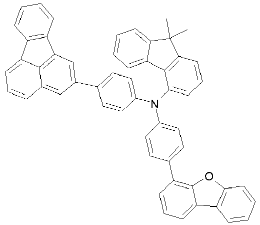 | 70 |
| 13 | 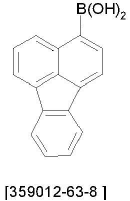 | 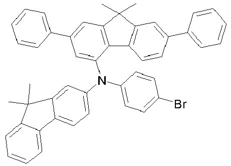 | 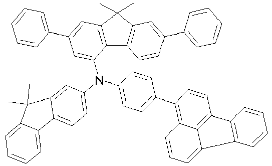 | 72 |
| 14 | 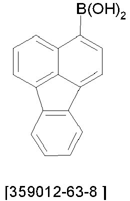 | 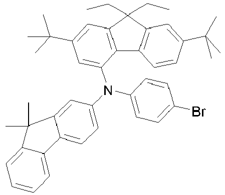 | 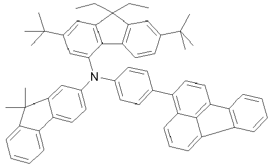 | 76 |
| 15 | 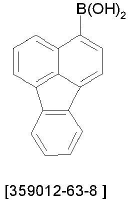 | 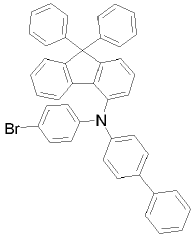 | 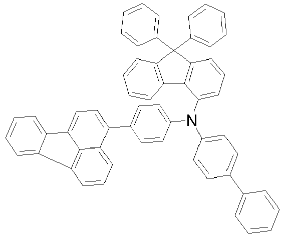 | 71 |
| 16 | 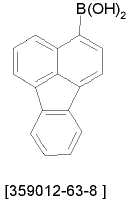 | 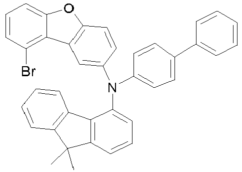 | 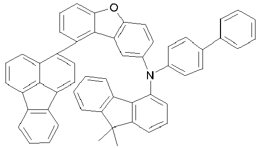 | 75 |
| 17 | 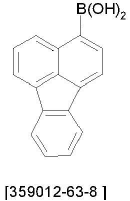 | 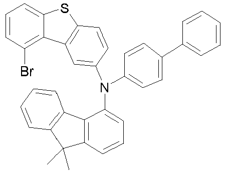 | 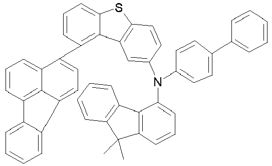 | 68 |
| 18 | 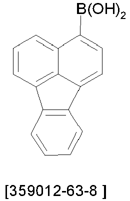 | 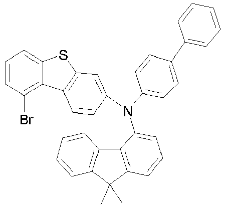 | 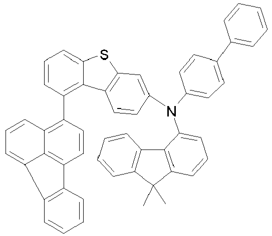 | 71 |
| 19# | 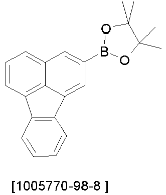 | 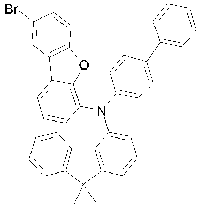 | 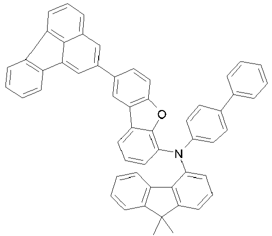 | 61 |
| 20 | 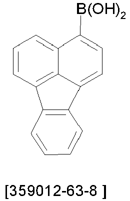 | 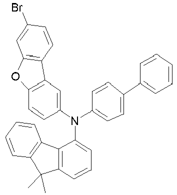 | 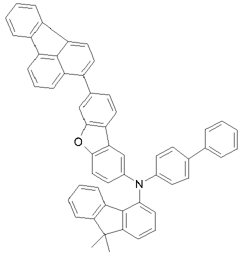 | 69 |
| 21# | 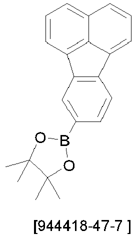 | 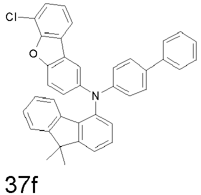 | 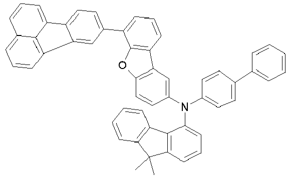 | 68 |
| 22 | 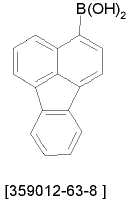 | 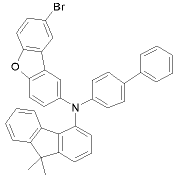 | 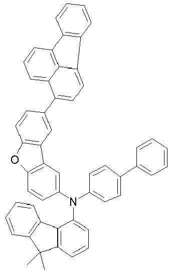 | 60 |
| 23 | 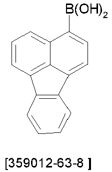 | 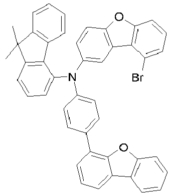 | 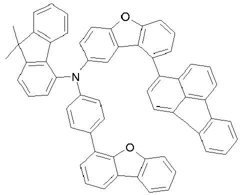 | 65 |
| **24** | 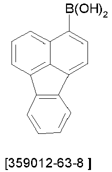 | 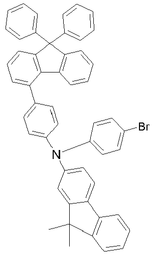 | 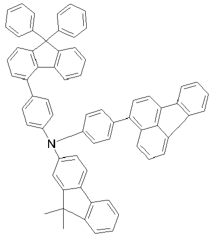 | 67 |
| 25 | 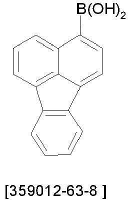 | 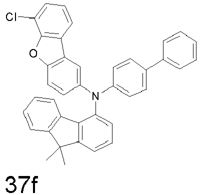 | 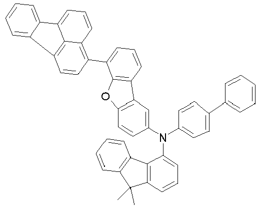 | 65 |

### d) 3-(4-Chloro-phenyl)-fluoranthen (1d)

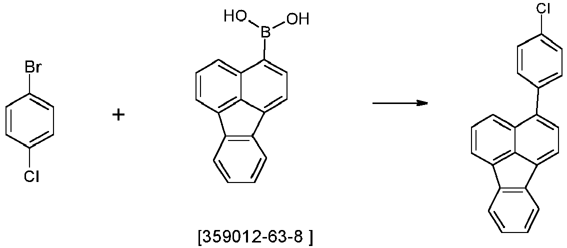

30 g (156 mmol) 1-Brom-4-Chlor-benzol, 37g (150mmol) Fluranthenyl-3-boronsäure und 36 g (340 mmol) Natriumcarbonat werden in 1000 mL Ethylenglycoldiaminether und 280 mL Wasser suspendiert. Zu dieser Suspension werden 1,8 g (1,5 mmol) Tetrakis(triphenylphosphin)-palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Die Ausbeute beträgt 30g (97 mmol), entsprechend 65 % der Theorie.

Analog dazu werden die folgenden Verbindungen hergestellt:

| | **Edukt 1** | **Edukt 2** | **Produkt** | **Ausbeute in %** |
|---|---|---|---|---|
| 2d | 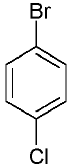 | 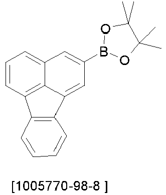 | 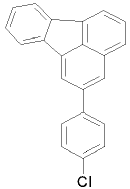 | 70 |
| 3d | 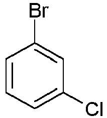 | 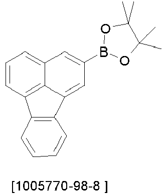 | 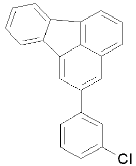 | 71 |
| 4d | 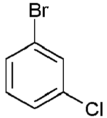 | 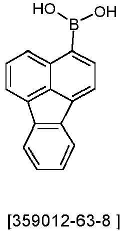 | 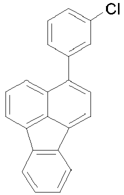 | 74 |
| 5d | 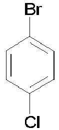 | 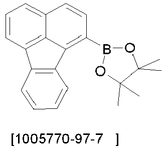 | 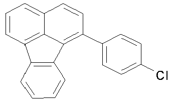 | 77 |
| 6d | 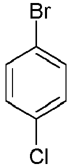 | 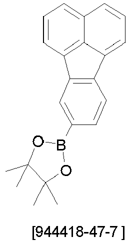 | 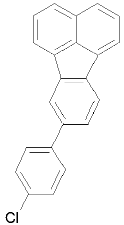 | 73 |
| 7d | 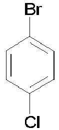 | 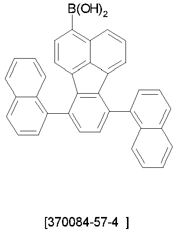 | 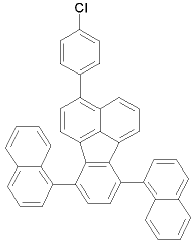 | 61 |
| 8d | 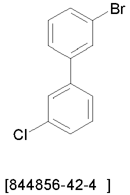 | 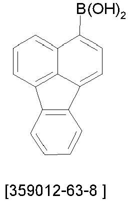 | 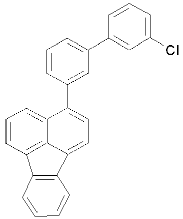 | 62 |
| 9d | 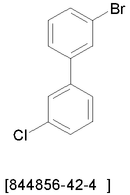 | 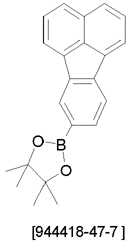 | 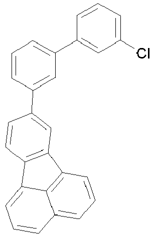 | 72 |

### e) 3-(4-Chloro-phenyl)-7,10-diphenyl-fluoranthen (1e)

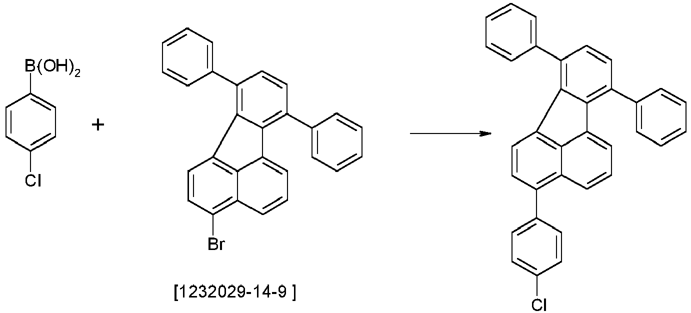

In einem 500ml Vierhalskolben werden 25,4 g (58.7mmol, 1.0eq) 3-Bromo-7,10-diphenyl-fluoranthen, 9.17g (58.7mmol, 1.0eq) 4-Chlorphenylboronsäure (CAS 1679-18-1) und 6.22g (58.7mmol, 1.0eq) Natriumcarbonat in 150ml Toluol, 36ml Ethanol und 77ml Wasser gelöst. Nach 30-minütigem Entgasen mittels eines Stickstoffstroms werden 678 mg (0.587mmol, 0.01eq) Tetrakis(triphenylphosphin)palladium zugegeben und der Ansatz wird über Nacht am Rückfluss erhitzt. Nach beendeter Reaktion werden die Phasen getrennt, die wässrige Phase dreifach mit Toluol extrahiert, die vereinten organischen Phasen anschließend mit Wasser gewaschen. Die organischen Phasen werden über Natriumsulfat getrocknet und die Lösung am Rotationsverdampfer eingeengt. Der Rückstand wird in 250ml Ethanol eingetragen und der entstandene Feststoff abgesaugt.

Die Ausbeute beträgt 25,6g (55 mmol), entsprechend 94 % der Theorie.

Analog werden hergestellt:

| **Verbindung** | **Edukt 1** | **Edukt 2** | **Produkt** | **Ausbeute [%]** |
|---|---|---|---|---|
| 1e | 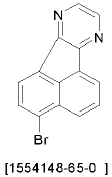 | 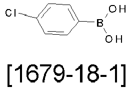 | 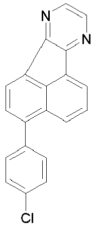 | 91 |
| 2e | 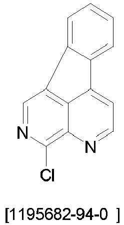 | 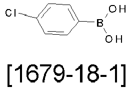 | 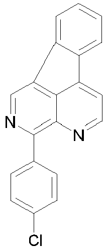 | 32 |
| 3e | 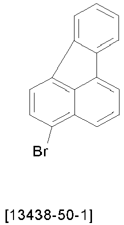 | 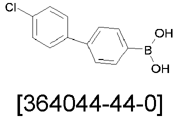 | 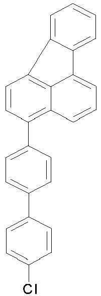 | 88 |
| 4e | 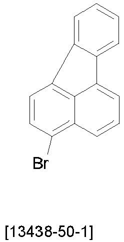 | 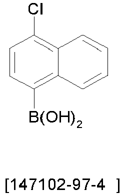 | 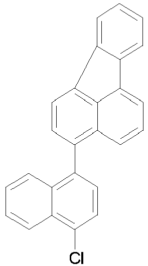 | 91 |
| 5e | 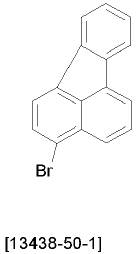 | 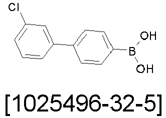 | 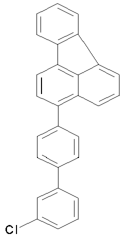 | 97 |
| 6e | 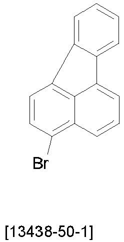 | 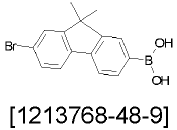 | 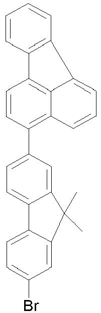 | 23 |
| 7e | 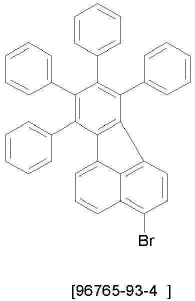 | 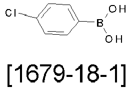 | 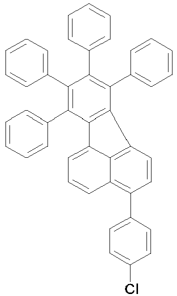 | 86 |

### f) Biphenyl-4-yl-(9,9-dimethyl-9H-fluoren-4-yl)-(4-fluoranthen-3-yl-phenyl)-amin (26)

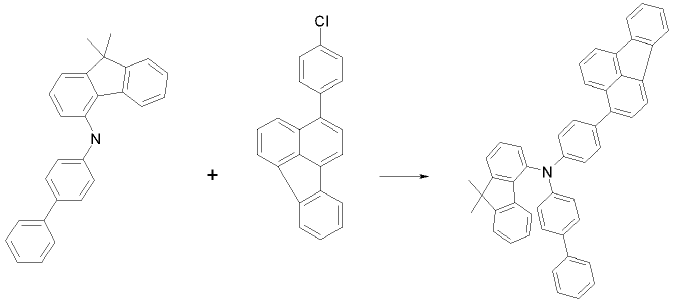

Ein Gemisch aus 21,6 g (60 mmol) 8-(4-Chloro-phenyl)-fluoranthen, 18,7 g (60 mmol) Biphenyl-4-yl-(9,9-dimethyl-9H-fluoren-4-yl)-amin, 7,7 g (80 mmol) Natrium-tert-butylat, 1,4 g (5 mmol) Tricyclohexylamin, 561 mg (2,5 mmol) Palladium(II)acetat und 300 ml Mesitylen wird 24 h unter Rückfluss erhitzt. Nach Erkalten versetzt man mit 200 ml Wasser, rührt 30 min. nach, trennt die organische Phase ab, filtriert diese über ein kurzes Celite-Bett und entfernt dann das Lösungsmittel im Vakuum. Der Rückstand wird fünfmal aus DMF umkristallisiert und abschließend zweimal fraktioniert sublimiert (p ca. 10⁻⁶ mbar, T = 360 - 390 °C). Ausbeute: 27 g (42 mmol), 71 % der Theorie: 99,9 % n. HPLC.

Analog werden folgende Verbindungen erhalten:

| **Bsp.** | **Edukt 1** | **Edukt 2** | **Produkt** | **Ausbeute [%]** |
|---|---|---|---|---|
| 27 | 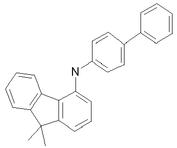 | 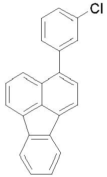 | 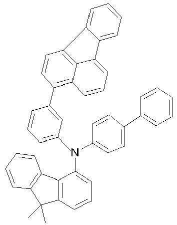 | 68 |
| 28# | 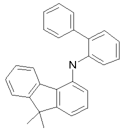 | 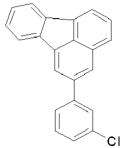 | 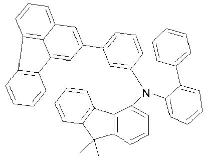 | 72 |
| 29 | 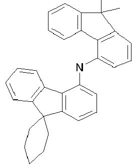 | 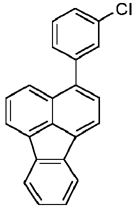 | 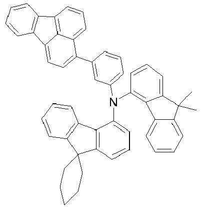 | 70 |
| 30# | 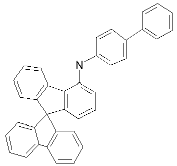 | 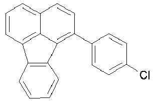 | 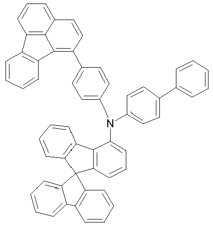 | 69 |
| 31# | 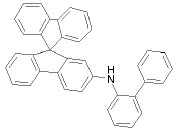 | 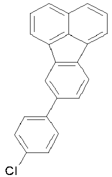 | 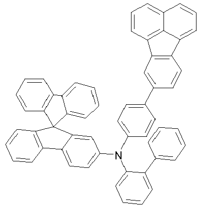 | 74 |
| 32 | 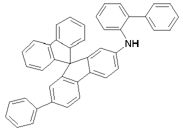 | 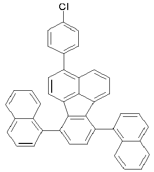 | 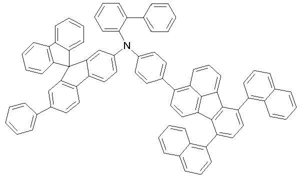 | 75 |
| 33 | 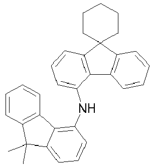 | 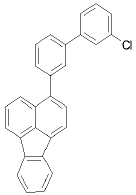 | 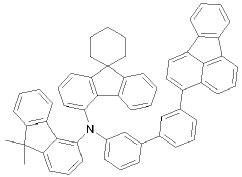 | 72 |
| 34 | 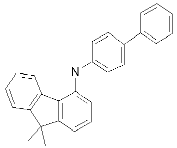 | 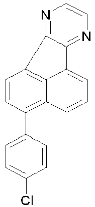 | 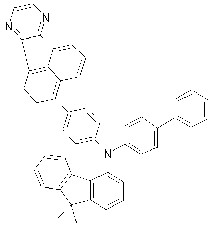 | 70 |
| 35 | 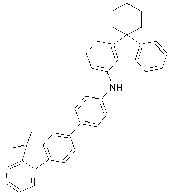 | 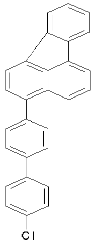 | 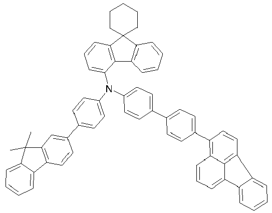 | 75 |
| 36 | 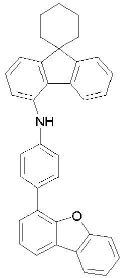 | 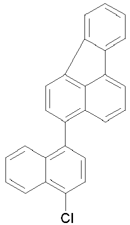 | 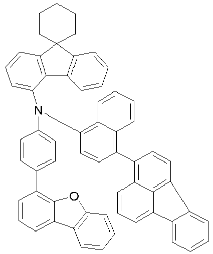 | 63 |
| 37 | 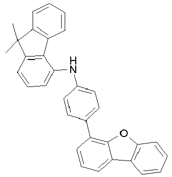 | 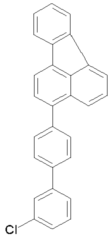 | 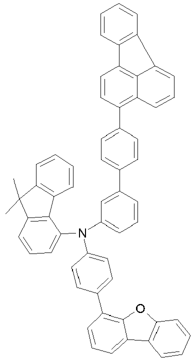 | 73 |
| 38 | 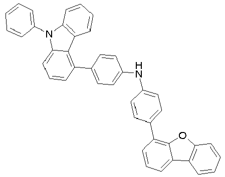 | 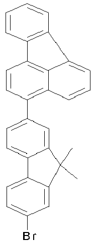 | 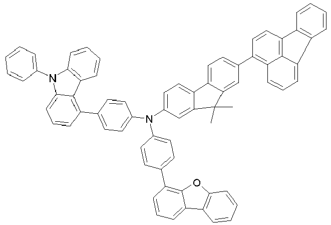 | 72 |
| 39# | 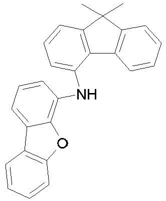 | 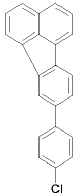 | 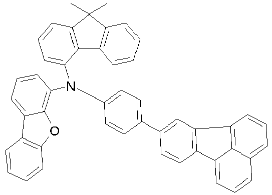 | 77 |
| 40# | 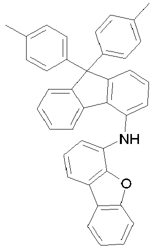 | 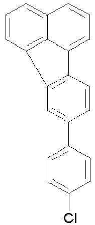 | 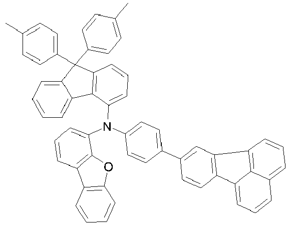 | 72 |
| 41# | 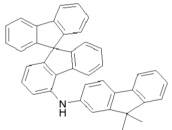 | 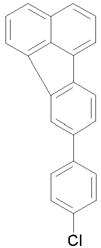 | 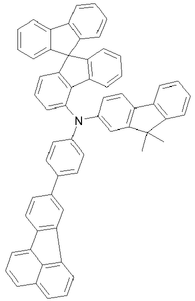 | 63 |
| 42# | 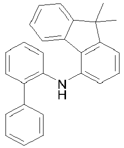 | 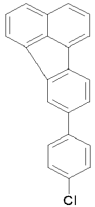 | 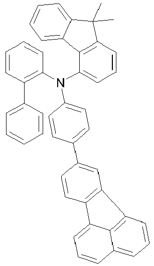 | 67 |
| 43# | 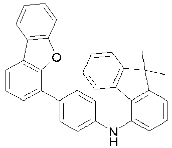 | 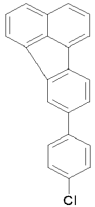 | 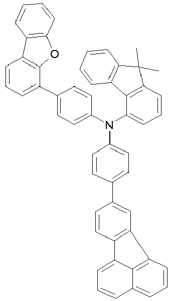 | 60 |
| 44# | 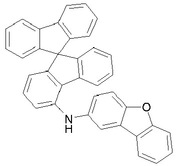 | 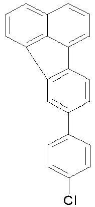 | 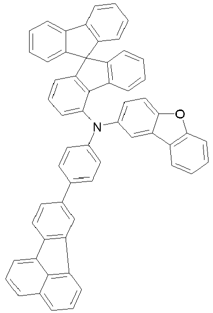 | 65 |
| 45# | 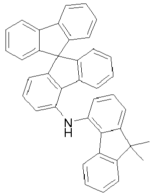 | 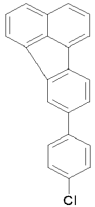 | 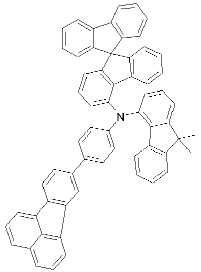 | 73 |
| 46# | 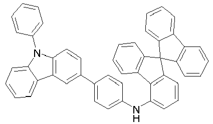 | 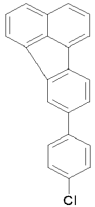 | 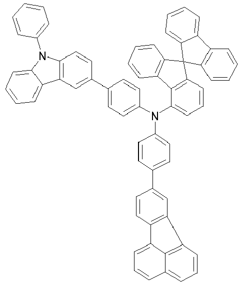 | 70 |
| 47# | 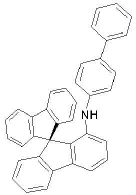 | 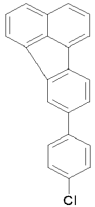 | 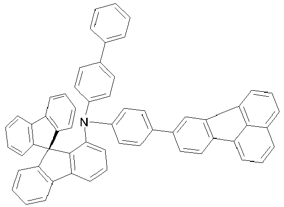 | 69 |
| 48# | 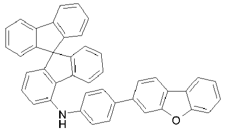 | 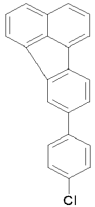 | 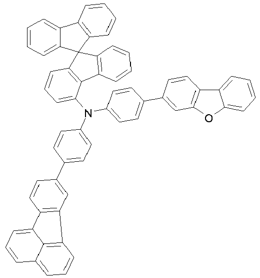 | 77 |
| 49 | 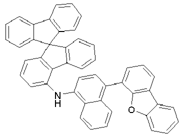 | 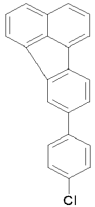 | 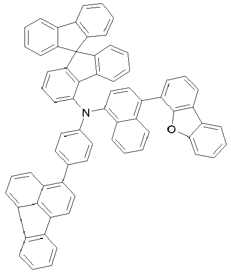 | 72 |
| 50 | 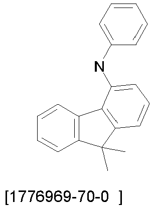 | 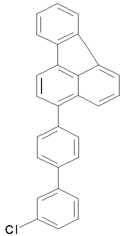 | 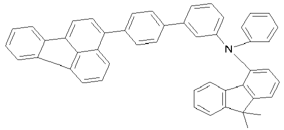 | 70 |
| 51 | 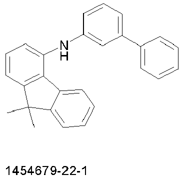 | 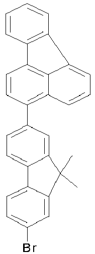 | 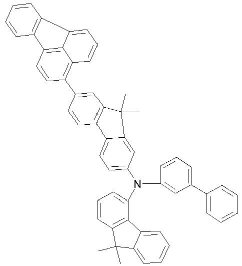 | 64 |
| 52# | 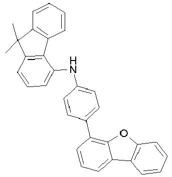 | 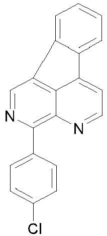 | 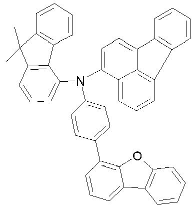 | 71 |
| 53 | 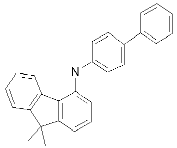 | 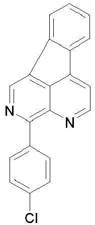 | 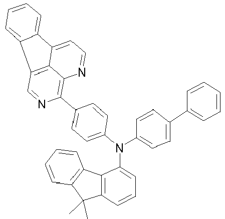 | 60 |
| 54 | 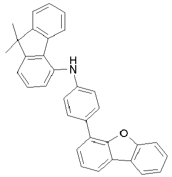 | 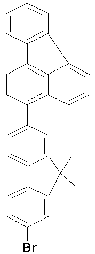 | 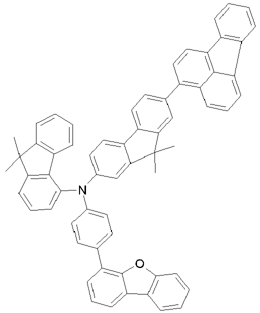 | 64 |
| 1f | 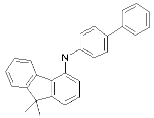 | 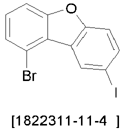 | 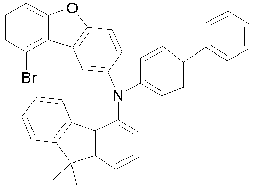 | 61 |
| 2f | 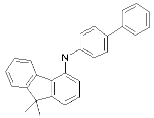 | 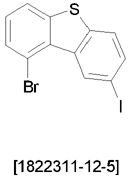 | 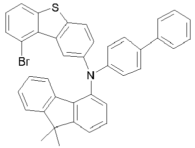 | 65 |
| 3f | 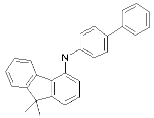 | 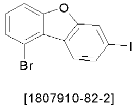 | 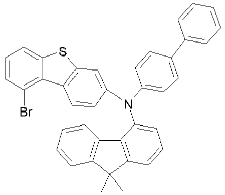 | 67 |
| 4f | 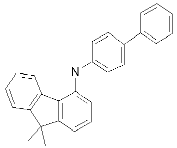 | 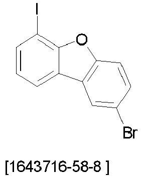 | 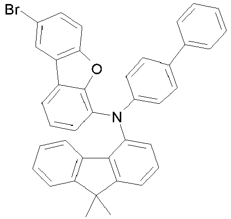 | 60 |
| 5f | 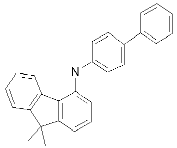 | 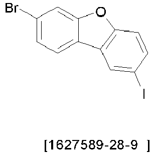 | 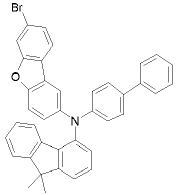 | 59 |
| 6f | 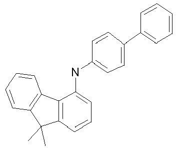 | 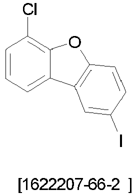 | 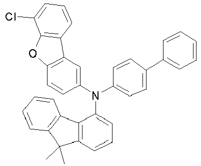 | 68 |
| 7f | 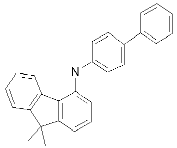 | 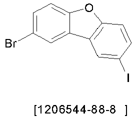 | 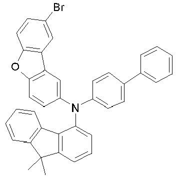 | 61 |
| 8f | 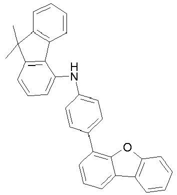 | 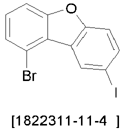 | 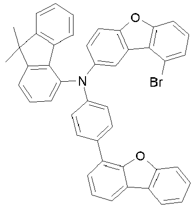 | 64 |
| 55# | 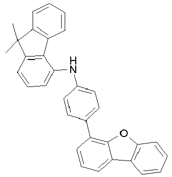 | 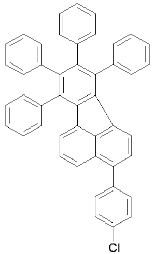 | 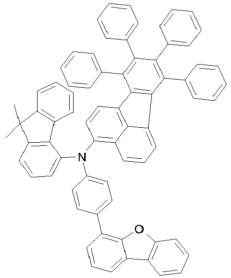 | 66 |
| 56# | 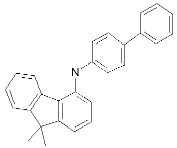 | 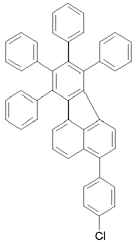 | 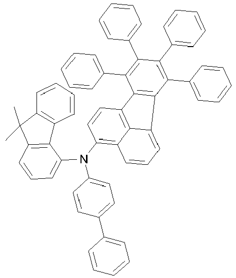 | 61 |
| 57# | 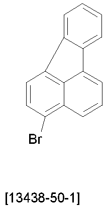 | 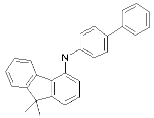 | 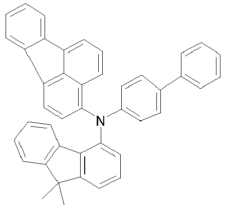 | 70 |
| 58# | 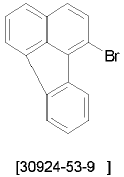 | 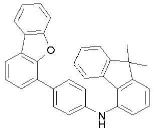 | 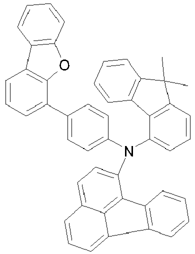 | 74 |
| 59# | 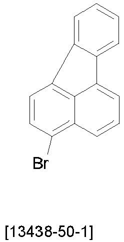 | 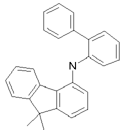 | 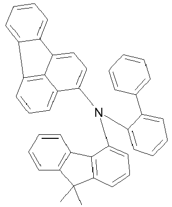 | 77 |
| 60# | 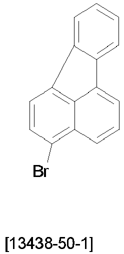 | 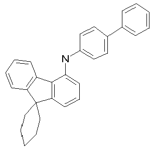 | 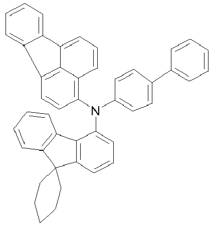 | 72 |
| 61# | 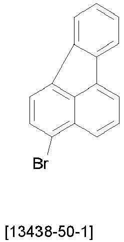 | 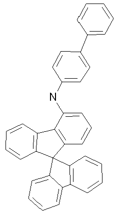 | 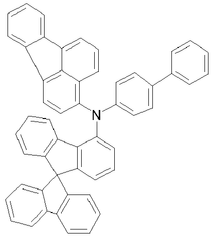 | 71 |
| 62# | 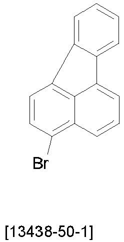 | 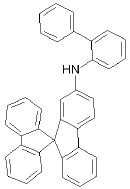 | 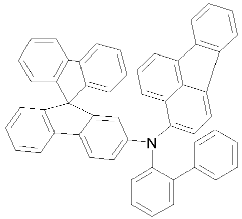 | 78 |
| 63# | 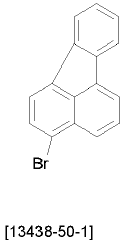 | 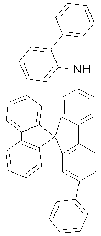 | 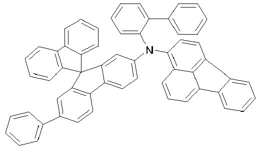 | 74 |
| 64# | 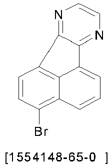 | 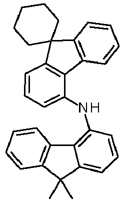 | 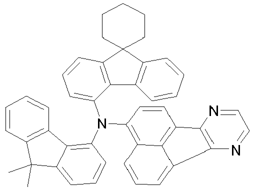 | 79 |
| 65# | 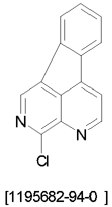 | 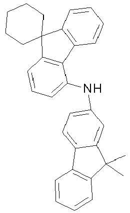 | 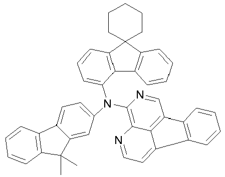 | 76 |
| 66# | 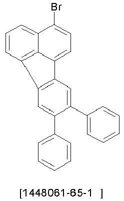 | 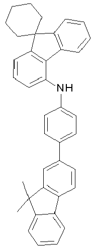 | 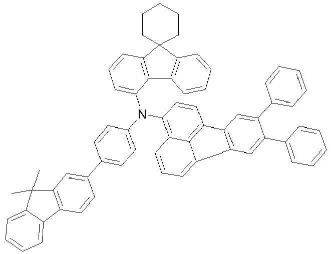 | 69 |
| 67# | 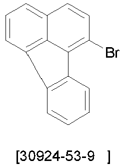 | 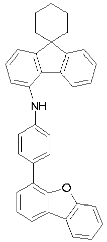 | 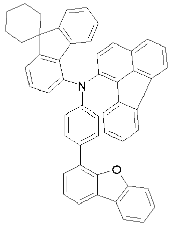 | 73 |
| 68# | 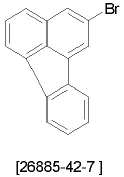 | 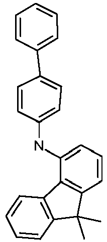 | 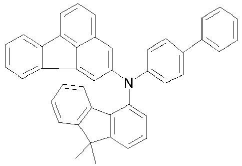 | 75 |
| 69# | 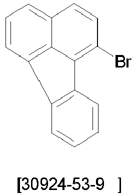 | 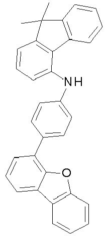 | 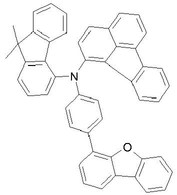 | 78 |
| 70# | 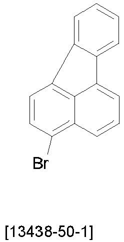 | 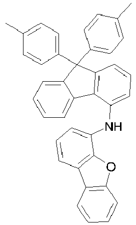 | 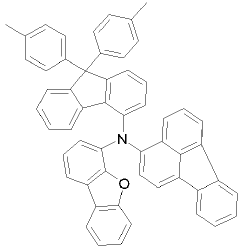 | 77 |
| 71# | 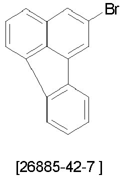 | 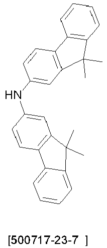 | 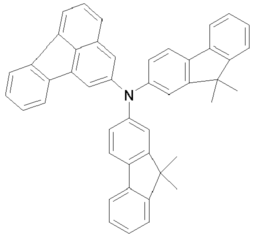 | 72 |
| 72# | 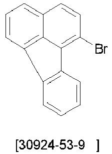 | 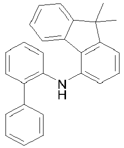 | 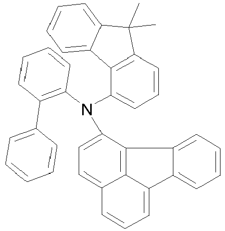 | 72 |
| 73 | 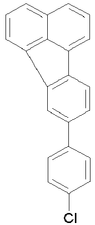 | 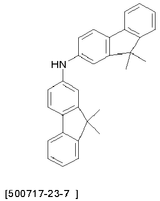 | 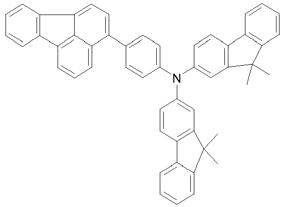 | 75 |
| 74# | 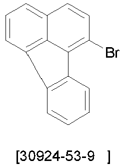 | 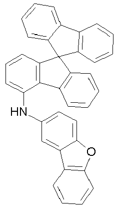 | 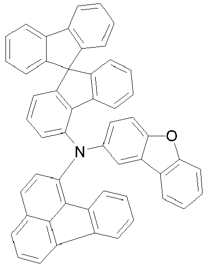 | 76 |
| 75# | 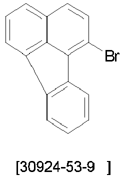 | 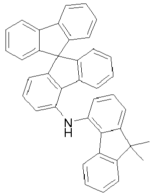 | 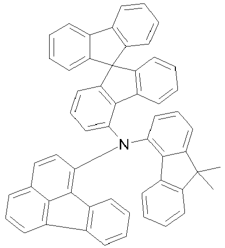 | 78 |
| 76# | 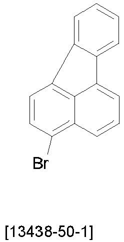 | 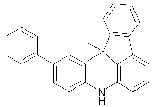 | 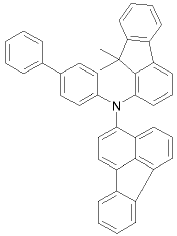 | 79 |
| 77# | 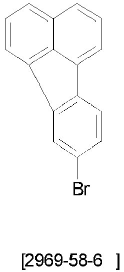 | 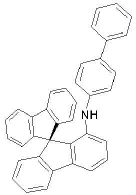 | 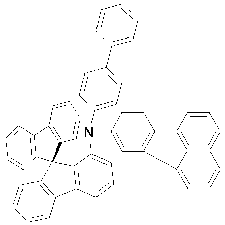 | 72 |
| 78# | 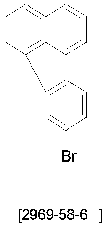 | 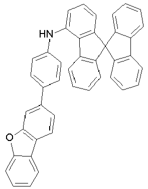 | 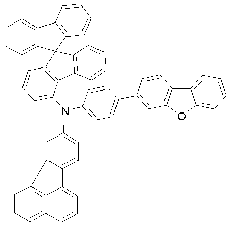 | 76 |
| 79# | 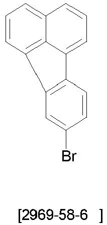 | 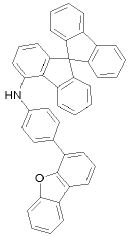 | 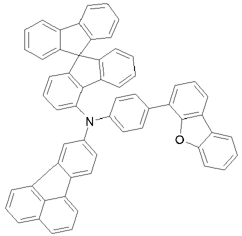 | 71 |
| 80# | 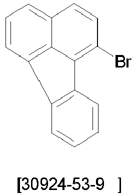 | 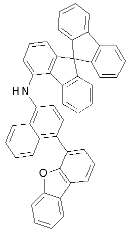 | 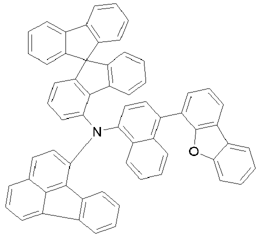 | 79 |
| 81# | 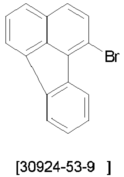 | 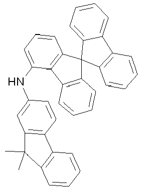 | 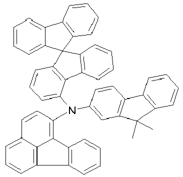 | 74 |
| 82# | 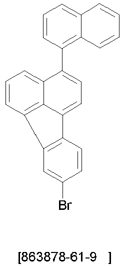 | 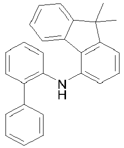 | 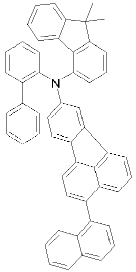 | 77 |
| 83# | 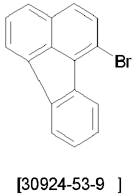 | 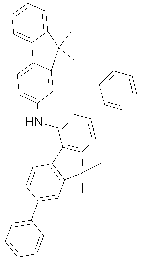 | 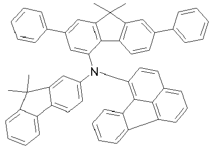 | 72 |
| 84# | 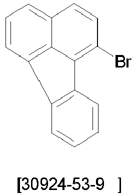 | 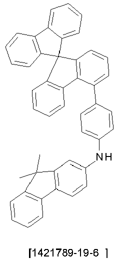 | 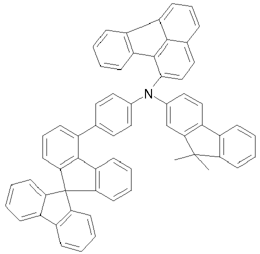 | 73 |
| 85# | 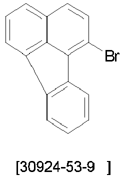 | 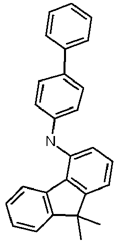 | 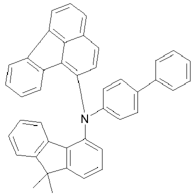 | 68 |
| 86# | 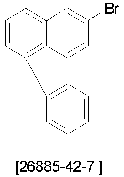 | 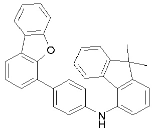 | 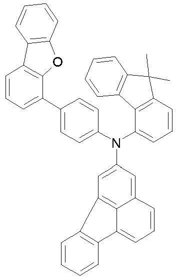 | 60 |
| 87 | 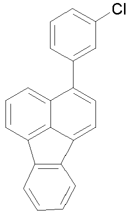 | 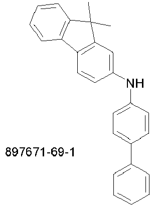 | 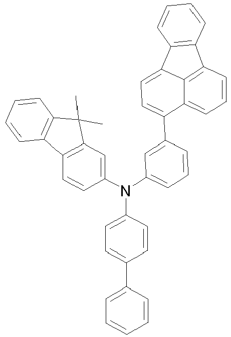 | 71 |
| 88 | 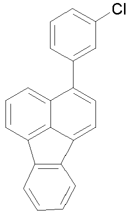 | 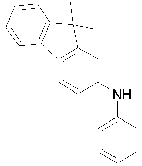 | 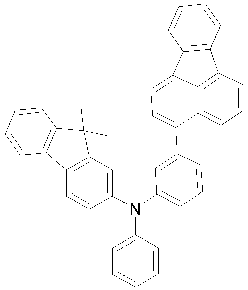 | 65 |

### B) Devicebeispiele

Es werden die erfindungsgemäßen OLEDs E1 bis E10 und die Vergleichs-OLEDs V1 bis V3 hergestellt, und ihre Eigenschaften werden vermessen (Tabellen 1 und 2).

Die OLEDs werden wie folgt hergestellt: Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC5:IC3:TEG2 (55%:35%:10%) bedeutet hierbei, dass das Material IC5 in einem Volumenanteil von 55%, IC3 in einem Anteil von 35% und TEG2in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = a mA/cm², L1 = b % bedeutet, dass die Leuchtdichte bei Betrieb mit a mA/cm² nach der Zeit LD auf b % ihres Anfangswertes absinkt.

Die erhaltenen Daten für die OLEDs sind in Tabelle 2 zusammengefasst.

| Tabelle 1: Aufbau der OLEDs | | | | | | |
|---|---|---|---|---|---|---|
| Bsp. | HIL Dicke | HTL Dicke | EBL Dicke | EML Dicke | HBL Dicke | ETL Dicke |
| V1 | HATCN | SpMA1 | SpMA3 | SdT1:TER5 | ST2 | ST2:LiQ (50%:50%) |
| | 5nm | 125nm | 10nm | (97%:3%) 35nm | 10nm | 30nm |
| V2 | HATCN | SpMA1 | SpMA3 | SdT2:TER5 | ST2 | ST2:LiQ (50%:50%) |
| | 5nm | 125nm | 10nm | (97%:3%) 35nm | 10nm | 30nm |
| V3 | HATCN | SpMA1 | SpMA3 | SdT3:TER5 | ST2 | ST2:LiQ (50%:50%) |
| | 5nm | 125nm | 10nm | (97%:3%) 35nm | 10nm | 30nm |
| E1 | HATCN | SpMA1 | SpMA3 | EG26:TER5 | ST2 | ST2:LiQ (50%:50%) |
| | 5nm | 125nm | 10nm | (97%:3%) 35nm | 10nm | 30nm |
| E2 | HATCN | SpMA1 | SpMA3 | EG87:TER5 | ST2 | ST2:LiQ (50%:50%) |
| | 5nm | 125nm | 10nm | (97%:3%) 35nm | 10nm | 30nm |
| E3 | HATCN | SpMA1 | SpMA3 | EG88:TER5 | ST2 | ST2:LiQ (50%:50%) |
| | 5nm | 125nm | 10nm | (97%:3%) 35nm | 10nm | 30nm |
| E4 | HATCN | SpMA1 | SpMA3 | EG23:TER5 | ST2 | ST2:LiQ (50%:50%) |
| | 5nm | 125nm | 10nm | (97%:3%) 35nm | 10nm | 30nm |
| E5# | HATCN | SpMA1 | SpMA3 | EG30:TER5 | ST2 | ST2:LiQ (50%:50%) |
| | 5nm | 125nm | 10nm | (97%:3%) 35nm | 10nm | 30nm |
| E6 | HATCN | SpMA1 | SpMA3 | IC5:IC3:TEG2 | EG18 | ST2:LiQ (50%:50%) |
| | 5nm | 230nm | 20nm | (60%:30%:10%) 30nm | 10nm | 30nm |
| E7# | HATCN | SpMA1 | SpMA3 | IC5:IC3:TEG2 | EG71 | ST2:LiQ (50%:50%) |
| | 5nm | 230nm | 20nm | (60%:30%:10%) 30nm | 10nm | 30nm |
| E8# | HATCN | SpMA1 | SpMA3 | IC5:IC3:TEG2 | EG85 | ST2:LiQ (50%:50%) |
| | 5nm | 230nm | 20nm | (60%:30%:10%) 30nm | 10nm | 30nm |
| E9 | HATCN | SpMA1 | SpMA3 | IC5:IC3:TEG2 | ST2 | EG3:LiQ (50%:50%) |
| | 5nm | 230nm | 20nm | (60%:30%:10%) 30nm | 10nm | 30nm |
| E10 | HATCN | SpMA1 | SpMA3 | IC5:IC3:TEG2 | ST2 | EG37:LiQ (50%:50%) |
| | 5nm | 230nm | 20nm | (60%:30%:10%) 30nm | 10nm | 30nm |

| Tabelle 2: Leistungsdaten der OLEDs | | | | | | |
|---|---|---|---|---|---|---|
| Bsp. | U1000 (V) | EQE 1000 | CIE x/y bei 1000 cd/m² | L₀; j₀ | L1 % | LD (h) |
| V1 | 3.4 | 16.8% | 0.67/0.33 | 60mA/cm² | 95 | 25 |
| V2 | 3.4 | 16.6% | 0.67/0.33 | 60mA/cm² | 95 | 20 |
| V3 | 3.4 | 16.7% | 0.67/0.33 | 60mA/cm² | 95 | 15 |
| E1 | 3.5 | 16.0% | 0.67/0.33 | 60mA/cm² | 95 | 105 |
| E2 | 3.4 | 16.4% | 0.67/0.33 | 60 mA/cm² | 95 | 80 |
| E3 | 3.4 | 16.4% | 0.67/0.33 | 60 mA/cm² | 95 | 45 |
| E4 | 3.5 | 16.2% | 0.67/0.33 | 60 mA/cm² | 95 | 115 |
| E5# | 3.6 | 16.3% | 0.67/0.33 | 60 mA/cm² | 95 | 95 |
| E6 | 3.3 | 18.1% | 0.33/0.63 | 40 mA/cm² | 80 | 220 |
| E7# | 3.4 | 17.5% | 0.33/0.63 | 40 mA/cm² | 80 | 200 |
| E8# | 3.4 | 17.7% | 0.33/0.63 | 40 mA/cm² | 80 | 210 |
| E9 | 3.5 | 17.6% | 0.33/0.63 | 40 mA/cm² | 80 | 215 |
| E10 | 3.6 | 17.3% | 0.33/0.63 | 40 mA/cm² | 80 | 195 |

| Tabelle 3: Strukturformeln der verwendeten Materialien | |
|---|---|
| 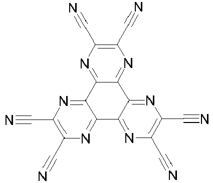 | 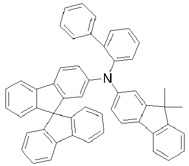 |
| HATCN | SpMA1 |
| 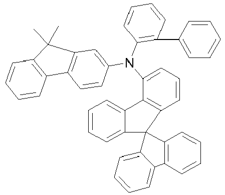 | 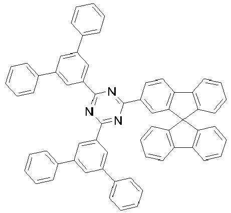 |
| SpMA3 | ST2 |
| 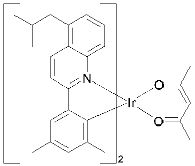 | 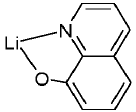 |
| TER5 | LiQ |
| 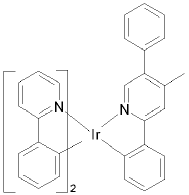 | 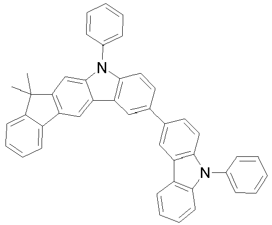 |
| TEG2 | IC3 |
| 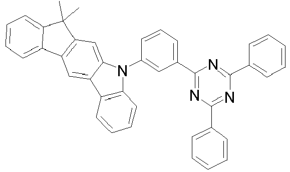 | 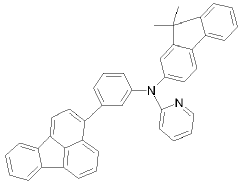 |
| IC5 | SdT1 |
| 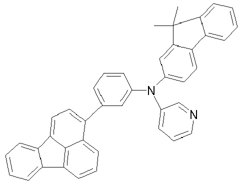 | 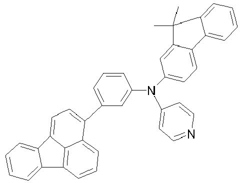 |
| SdT2 | SdT3 |
| 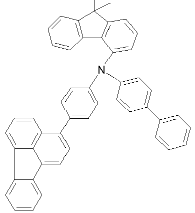 | 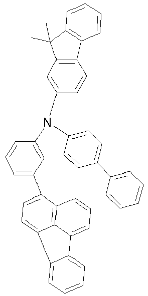 |
| EG26 | EG87 |
| 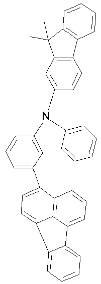 | 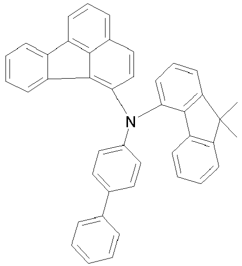 |
| EG88 | EG85 # |
| 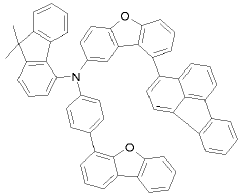 | 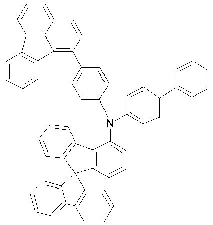 |
| EG23 | EG30# |
| 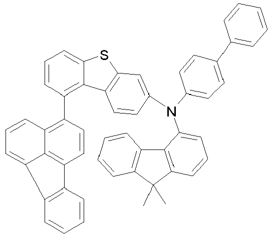 | 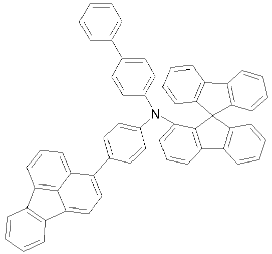 |
| EG18 | EG3 |
| 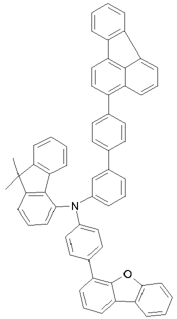 | 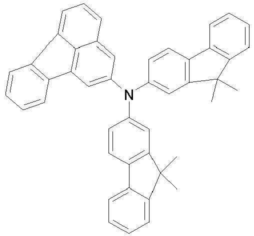 |
| EG37 | EG71 # |

In den Versuchen E1 bis E4 werden die Materialien gemäß der vorliegenden Anmeldung EG26, EG87, EG88 und EG23 als Matrixmaterialien für rot phosphoreszierende Emitter in der emittierenden Schicht eingesetzt. Die OLEDs V1 bis V3 unterscheiden sich von den OLEDs E1 bis E4 lediglich durch das in der emittierenden Schicht als Matrix eingesetzte Material (SdT1, SdT2, und SdT3). Ansonsten sind sie identisch aufgebaut. Es wird gefunden, dass die erfindungsgemäßen OLEDs E1 bis E4 durchweg eine deutlich höhere Lebensdauer aufweisen als die Vergleichs-OLEDs V1 bis V3.

Der Versuch E6 zeigt, dass sich die erfindungsgemäße Verbindung EG18 hervorragend als Lochblockiermaterial eignet.

Die Versuche E9 und E10 zeigen, dass sich die erfindungsgemäßen Verbindungen (EG3, EG37) hervorragend als Elektronentransportmaterialien eignen.

## Patentansprüche

1. Verbindung gemäß Formel (I) 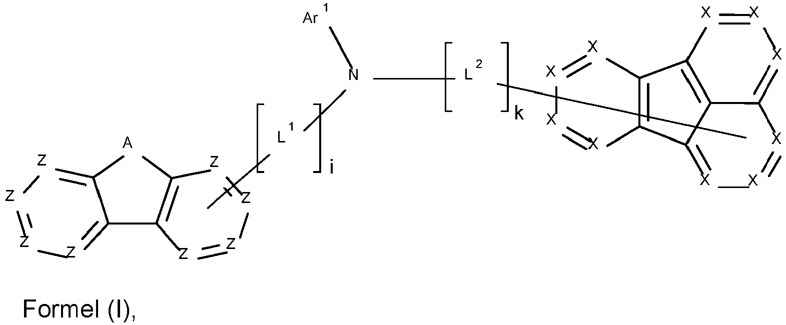 wobei für die auftretenden Variablen gilt:
A ist gleich C(R¹)₂ oder gleich 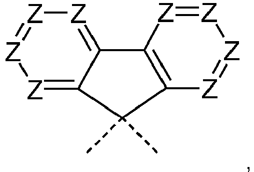
wobei die gestrichelten Linien die Bindungen hin zu den aromatischen Sechsringen darstellen;
Z ist bei jedem Auftreten gleich oder verschieden CR² oder N oder C, wobei eine Gruppe Z genau dann gleich C ist, wenn an sie die Gruppe [L¹]ᵢ gebunden ist;
X ist bei jedem Auftreten gleich oder verschieden CR³ oder N oder C, wobei eine Gruppe X genau dann gleich C ist, wenn an sie die Gruppe [L²]ₖ gebunden ist;
L¹,L² sind bei jedem Auftreten gleich oder verschieden gewählt aus einer divalenten Gruppe abgeleitet von wahlweise mit Resten R⁴ substituiertem Benzol, Biphenyl, Terphenyl, Fluoren, Spirobifluoren, Indenofluoren, Carbazol, Dibenzofuran, Dibenzothiophen, oder einer Kombination von zwei oder mehr dieser Gruppen;
Ar¹ ist ein aromatisches Ringsystem mit 6 bis 24 aromatischen Ringatomen, das mit einem oder mehreren Resten R⁵ substituiert sein kann, oder ein heteroaromatisches Ringsystem mit 5 bis 24 aromatischen Ringatomen, das mit einem oder mehreren Resten R⁵ substituiert sein kann, wobei Ar¹ keine stickstoffhaltige Heteroarylgruppe umfasst, die direkt an das Amin-Stickstoffatom der Formel (I) gebunden ist, und wobei Ar¹ und daran gebundene Substituenten keine Carbazolgruppe enthalten;
R¹, R³, R⁴ sind bei jedem Auftreten gleich oder verschieden gewählt aus H, D, F, C(=O)R⁶, CN, Si(R⁶)₃, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyloder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R³ bzw. R⁴ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R² ist bei jedem Auftreten gleich oder verschieden gewählt aus H, D, F, C(=O)R⁶, CN, Si(R⁶)₃, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy- und Alkinylgruppen durch -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R⁵ ist bei jedem Auftreten gleich oder verschieden gewählt aus H, D, Si(R⁶)₃, OR⁶, geradkettigen Alkyl- oder Alkoxygruppen mit 1 bis 20 C-Atomen, verzweigten oder cyclischen Alkyloder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, NR⁶, O- oder -S- ersetzt sein können;
R⁶ ist bei jedem Auftreten gleich oder verschieden gewählt aus H, D, F, C(=O)R⁷, CN, Si(R⁷)₃, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁶ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁷ substituiert sein können; und wobei eine oder mehrere CH₂-Gruppen in den genannten Alkyl-, Alkoxy-, Alkenyl- und Alkinylgruppen durch -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
R⁷ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁷ miteinander verknüpft sein können und einen Ring bilden können; und wobei die genannten Alkyl-, Alkoxy-, Alkenyl-und Alkinylgruppen, aromatischen Ringsysteme und heteroaromatischen Ringsysteme mit F oder CN substituiert sein können;
i ist gleich 0, 1, 2 oder 3; und
k ist gleich 1, 2 oder 3,
wobei die Fluoranthengruppe in Formel (I) in der Position 3 oder 4 gebunden ist, wobei die Positionen wie folgt nummeriert sind:

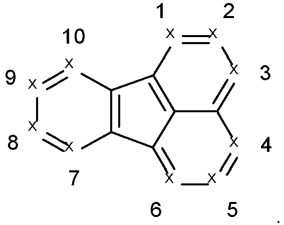

2. Verbindung nach Anspruch 1, **dadurch gekennzeichnet, dass** die Gruppe der Formel 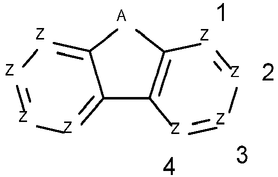 in Formel (I) in einer der Positionen 1, 3, und 4 gebunden ist.

3. Verbindung nach Anspruch 1 oder 2, **dadurch gekennzeichnet, dass** Ar¹ gewählt ist aus wahlweise mit Resten R⁵ substituiertem Phenyl, Biphenyl, verzweigtem Terphenyl, unverzweigtem Terphenyl, verzweigtem Quaterphenyl, unverzweigtem Quaterphenyl, Fluorenyl, Dibenzofuranyl, Dibenzothiophenyl, Fluorenyl-Phenylen, Dibenzofuranyl-Phenylen, Dibenzothiophen-Phenylen, Phenanthrenyl und Triphenylyl.

4. Verbindung nach einem oder mehreren der Ansprüche 1 bis 3, **dadurch gekennzeichnet, dass** R¹ bei jedem Auftreten gleich oder verschieden gewählt ist aus F, Si(R⁶)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R⁶ substituiert sein können; und wobei zwei Gruppen R¹, die an dasselbe Kohlenstoffatom binden, miteinander zu einem Ring verbunden sein können, so dass ein Spiro-Kohlenstoffatom entsteht.

5. Verbindung nach einem oder mehreren der Ansprüche 1 bis 4, **dadurch gekennzeichnet, dass** R², R³, und R⁴ bei jedem Auftreten gleich oder verschieden gewählt sind aus H, D, F, CN, Si(R⁶)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R⁶ substituiert sein können; und wobei in den genannten Alkylgruppen eine oder mehrere CH₂-Gruppen durch -C=C-, Si(R⁶)₂, C=O, C=NR⁶, -NR⁶-, -O-, -S-, -C(=O)O- oder -C(=O)NR⁶- ersetzt sein können.

6. Verbindung nach einem oder mehreren der Ansprüche 1 bis 5, **dadurch gekennzeichnet, dass** R⁵ bei jedem Auftreten gleich oder verschieden gewählt ist aus H, D, Si(R⁶)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R⁶ substituiert sein können, wobei R⁵ und Substituenten, die an R⁵ binden, keine Carbazolgruppe enthalten.

7. Verbindung nach einem oder mehreren der Ansprüche 1 bis 6, **dadurch gekennzeichnet, dass** R⁶ bei jedem Auftreten gleich oder verschieden gewählt ist aus H, D, F, CN, Si(R⁷)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R⁷ substituiert sein können; und wobei in den genannten Alkylgruppen eine oder mehrere CH₂-Gruppen durch -C=C-, -R⁷C=CR⁷- Si(R⁷)₂, C=O, C=NR⁷, -NR⁷-, -O-, -S-, - C(=O)O- oder -C(=O)NR⁷- ersetzt sein können.

8. Verbindung nach einem oder mehreren der Ansprüche 1 bis 7, **dadurch gekennzeichnet, dass** i gleich 0 ist.

9. Verbindung nach einem oder mehreren der Ansprüche 1 bis 8, **dadurch gekennzeichnet, dass** k gleich 1 ist.

10. Verbindung nach einem oder mehreren der Ansprüche 1 bis 9, **dadurch gekennzeichnet, dass** die Verbindung einer der folgenden Formeln entspricht 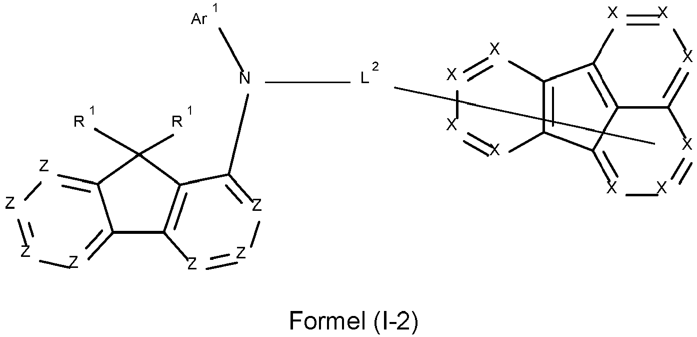 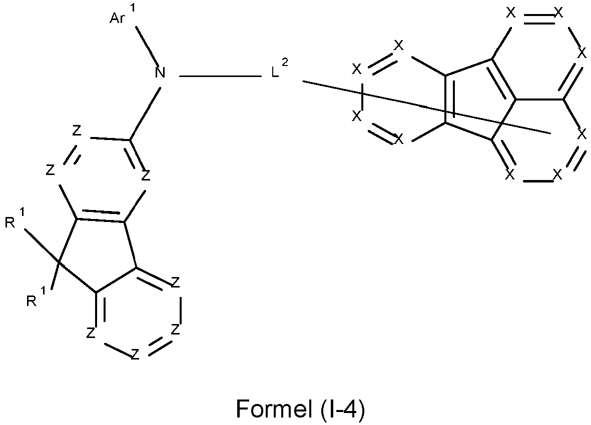 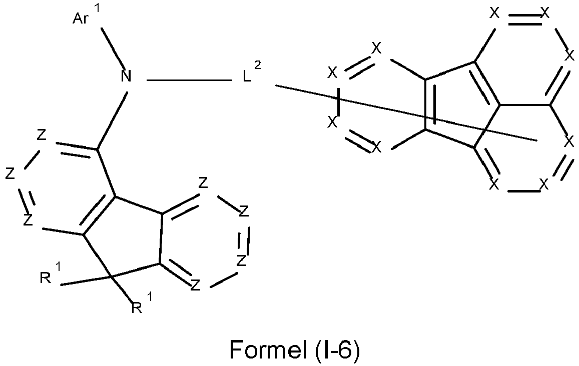 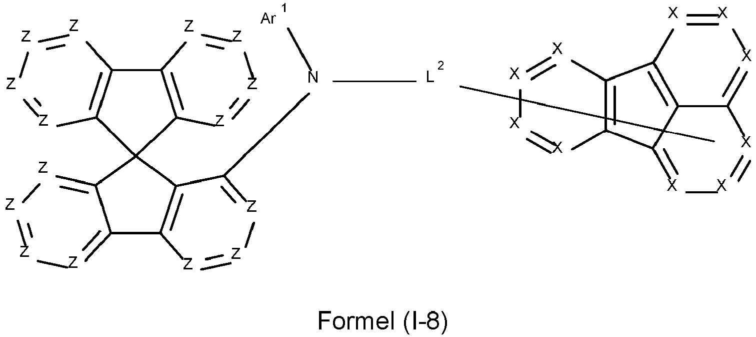 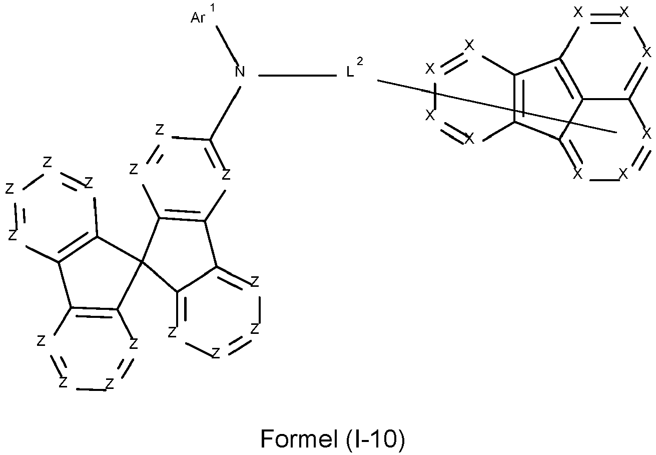 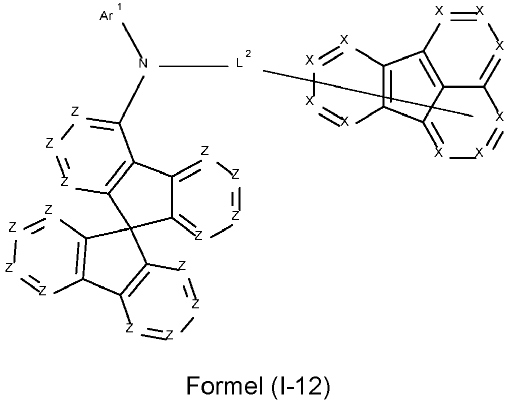 wobei die auftretenden Variablen definiert sind wie in einem oder mehreren der Ansprüche 1 bis 9.

11. Verfahren zur Herstellung einer Verbindung gemäß Formel (I) nach einem oder mehreren der Ansprüche 1 bis 10, welches **dadurch gekennzeichnet ist, dass** ein Fluorenyl-Amin in einer Buchwald-Kupplungsreaktion mit einer aromatischen oder heteroaromatischen Verbindung umgesetzt wird.

12. Oligomer, Polymer oder Dendrimer, enthaltend eine oder mehrere Verbindungen gemäß Formel (I) nach einem oder mehreren der Ansprüche 1 bis 10, wobei die Bindung(en) zum Polymer, Oligomer oder Dendrimer an beliebigen, in Formel (I) mit R¹, R², R³, R⁴ oder R⁵ substituierten Positionen lokalisiert sein können.

13. Formulierung, enthaltend mindestens eine Verbindung nach einem oder mehreren der Ansprüche 1 bis 10 oder ein Polymer, Oligomer oder Dendrimer nach Anspruch 12, sowie mindestens ein Lösungsmittel.

14. Elektronische Vorrichtung, enthaltend mindestens eine Verbindung nach einem oder mehreren der Ansprüche 1 bis 10, oder ein Polymer, Oligomer oder Dendrimer nach Anspruch 12.

15. Elektronische Vorrichtung nach Anspruch 14, **dadurch gekennzeichnet, dass** sie eine organische Elektrolumineszenzvorrichtung ist, enthaltend Anode, Kathode und mindestens eine emittierende Schicht, wobei mindestens eine organische Schicht der Vorrichtung, die eine emittierende Schicht oder eine lochtransportierende Schicht sein kann, die mindestens eine Verbindung enthält.

16. Elektronische Vorrichtung nach Anspruch 15, **dadurch gekennzeichnet, dass** sie eine organische Elektrolumineszenzvorrichtung ist, enthaltend Anode, Kathode, und mindestens eine emittierende Schicht enthaltend einen rot emittierenden phosphoreszierenden Emitter, wobei die mindestens eine Verbindung in der emittierenden Schicht als Matrixmaterial enthalten ist.

17. Verwendung einer Verbindung nach einem oder mehreren der Ansprüche 1 bis 10 in einer elektronischen Vorrichtung.

## Claims

1. Compound of formula (I) 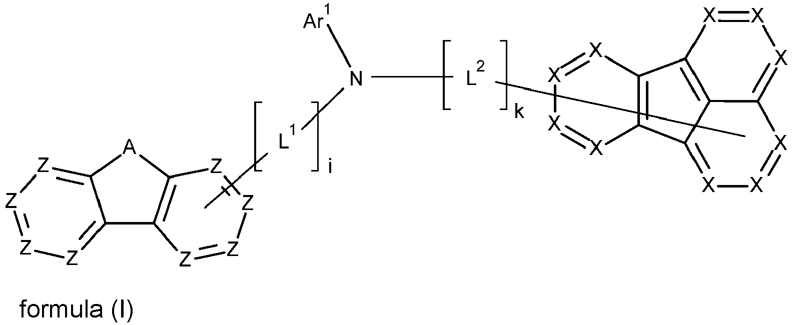 where the following applies to the variables occurring:
A is equal to C(R¹)₂ or is equal to 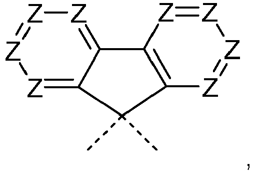
where the dashed lines represent the bonds to the aromatic sixmembered rings;
Z is on each occurrence, identically or differently, CR² or N or C, where a group Z is equal to C precisely if the group [L¹]ᵢ is bonded to it;
X is on each occurrence, identically or differently, CR³ or N or C, where a group X is equal to C precisely if the group [L²]ₖ is bonded to it;
L¹, L² are selected on each occurrence, identically or differently, from a divalent group derived from benzene, biphenyl, terphenyl, fluorene, spirobifluorene, indenofluorene, carbazole, dibenzofuran, dibenzothiophene, each of which is optionally substituted by radicals R⁴, or a combination of two or more of these groups;
Ar¹ is an aromatic ring system having 6 to 24 aromatic ring atoms, which may be substituted by one or more radicals R⁵, or a heteroaromatic ring system having 5 to 24 aromatic ring atoms, which may be substituted by one or more radicals R⁵, where Ar¹ does not encompass a nitrogen-containing heteroaryl group which is bonded directly to the amine nitrogen atom of the formula (I), and where Ar¹ and substituents bonded thereto do not contain a carbazole group;
R¹, R³, R⁴ are selected on each occurrence, identically or differently, from H, D, F, C(=O)R⁶, CN, Si(R⁶)₃, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ or R³ or R⁴ may be linked to one another and may form a ring; where the said alkyl, alkoxy, alkenyl and alkynyl groups and the said aromatic ring systems and heteroaromatic ring systems may in each case be substituted by one or more radicals R⁶; and where one or more CH₂ groups in the said alkyl, alkoxy, alkenyl and alkynyl groups may be replaced by -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO or SO₂;
R² is selected on each occurrence, identically or differently, from H, D, F, C(=O)R⁶, CN, Si(R⁶)₃, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkynyl groups having 2 to 20 C atoms, aromatic ring systems having 6 to 40 aromatic ring atoms, and heteroaromatic ring systems having 5 to 40 aromatic ring atoms; where two or more radicals R² may be linked to one another and may form a ring; where the said alkyl, alkoxy and alkynyl groups and the said aromatic ring systems and heteroaromatic ring systems may in each case be substituted by one or more radicals R⁶; and where one or more CH₂ groups in the said alkyl, alkoxy and alkynyl groups may be replaced by -C≡C-, Si(R⁶)₂, C=O, C=NR⁶, -C(=O)O-, -C(=O)NR⁶-, NR⁶, P(=O)(R⁶), -O-, -S-, SO or SO₂;
R⁵ is selected on each occurrence, identically or differently, from H, D, Si(R⁶)₃, OR⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁵ may be linked to one another and may form a ring; where the said alkyl, alkoxy, alkenyl and alkynyl groups and the said aromatic ring systems and heteroaromatic ring systems may in each case be substituted by one or more radicals R⁶; and where one or more CH₂ groups in the said alkyl, alkoxy, alkenyl and alkynyl groups may be replaced by -R⁶C=CR⁶-, -C=C-, Si(R⁶)₂, NR⁶, O- or -S-;
R⁶ is selected on each occurrence, identically or differently, from H, D, F, C(=O)R⁷, CN, Si(R⁷)₃, N(R⁷)₂, P(=O)(R⁷)₂ OR⁷, S(=O)R⁷, S(=O)₂R⁷, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁶ may be linked to one another and may form a ring; where the said alkyl, alkoxy, alkenyl and alkynyl groups and the said aromatic ring systems and heteroaromatic ring systems may in each case be substituted by one or more radicals R⁷; and where one or more CH₂ groups in the said alkyl, alkoxy, alkenyl and alkynyl groups may be replaced by -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO or SO₂;
R⁷ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁷ may be linked to one another and may form a ring; and where the said alkyl, alkoxy, alkenyl and alkynyl groups, aromatic ring systems and heteroaromatic ring systems may be substituted by F or CN;
i is equal to 0, 1, 2 or 3; and
k is equal to 1, 2 or 3,
where
the fluoranthene group in formula (I) is bonded in position 3 or 4, where the positions are numbered as follows:

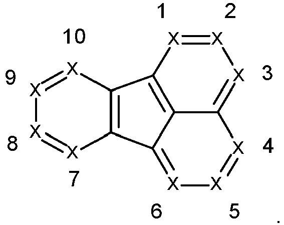

2. Compound according to Claim 1, **characterised in that** the group of the formula 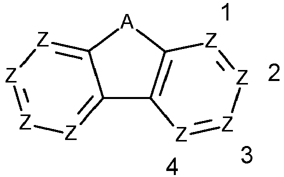 in formula (I) is bonded in one of positions 1, 3 and 4.

3. Compound according to Claim 1 or 2, **characterised in that** Ar¹ is selected from phenyl, biphenyl, branched terphenyl, unbranched terphenyl, branched quaterphenyl, unbranched quaterphenyl, fluorenyl, dibenzofuranyl, dibenzothiophenyl, fluorenylphenylene, dibenzofuranylphenylene, dibenzothiophenephenylene, phenanthrenyl and triphenylyl, each of which is optionally substituted by radicals R⁵.

4. Compound according to one or more of Claims 1 to 3, **characterised in that** R¹ is selected on each occurrence, identically or differently, from F, Si(R⁶)₃, straight-chain alkyl groups having 1 to 20 C atoms, branched or cyclic alkyl groups having 3 to 20 C atoms, aromatic ring systems having 6 to 24 aromatic ring atoms, and heteroaromatic ring systems having 5 to 24 aromatic ring atoms; where the said alkyl groups, the said aromatic ring systems and the said heteroaromatic ring systems may in each case be substituted by one or more radicals R⁶; and where two groups R¹ that are bonded to the same carbon atom may be connected to one another to form a ring, giving rise to a spiro carbon atom.

5. Compound according to one or more of Claims 1 to 4, **characterised in that** R², R³ and R⁴ are selected on each occurrence, identically or differently, from H, D, F, CN, Si(R⁶)₃, straight-chain alkyl groups having 1 to 20 C atoms, branched or cyclic alkyl groups having 3 to 20 C atoms, aromatic ring systems having 6 to 24 aromatic ring atoms and heteroaromatic ring systems having 5 to 24 aromatic ring atoms; where the said alkyl groups, the said aromatic ring systems and the said heteroaromatic ring systems may in each case be substituted by one or more radicals R⁶; and where one or more CH₂ groups in the said alkyl groups may be replaced by -C=C-, Si(R⁶)₂, C=O, C=NR⁶, -NR⁶-, -O-, -S-, -C(=O)O- or -C(=O)NR⁶-.

6. Compound according to one or more of Claims 1 to 5, **characterised in that** R⁵ is selected on each occurrence, identically or differently, from H, D, Si(R⁶)₃, straight-chain alkyl groups having 1 to 20 C atoms, branched or cyclic alkyl groups having 3 to 20 C atoms, aromatic ring systems having 6 to 24 aromatic ring atoms, and heteroaromatic ring systems having 5 to 24 aromatic ring atoms; where the said alkyl groups, the said aromatic ring systems and the said heteroaromatic ring systems may in each case be substituted by one or more radicals R⁶, where R⁵ and substituents bonded to R⁵ do not contain a carbazole group.

7. Compound according to one or more of Claims 1 to 6, **characterised in that** R⁶ is selected on each occurrence, identically or differently, from H, D, F, CN, Si(R⁷)₃, straight-chain alkyl groups having 1 to 20 C atoms, branched or cyclic alkyl groups having 3 to 20 C atoms, aromatic ring systems having 6 to 24 aromatic ring atoms and heteroaromatic ring systems having 5 to 24 aromatic ring atoms; where the said alkyl groups, the said aromatic ring systems and the said heteroaromatic ring systems may in each case be substituted by one or more radicals R⁷; and where one or more CH₂ groups in the said alkyl groups may be replaced by -C=C-, -R⁷C=CR⁷-, Si(R⁷)₂, C=O, C=NR⁷, -NR⁷-, -O-, -S-, -C(=O)O- or -C(=O)NR⁷-.

8. Compound according to one or more of Claims 1 to 7, **characterised in that** i is equal to 0.

9. Compound according to one or more of Claims 1 to 8, **characterised in that** k is equal to 1.

10. Compound according to one or more of Claims 1 to 9, **characterised in that** the compound corresponds to one of the following formulae: 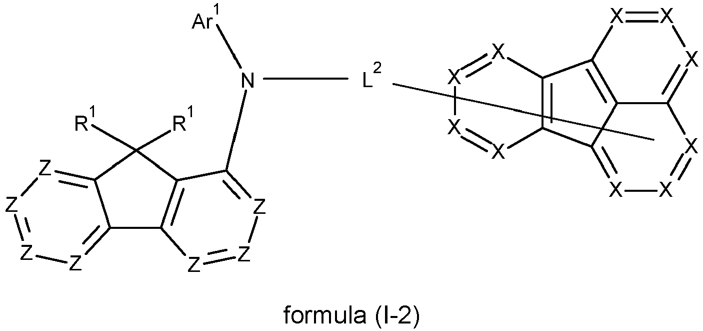 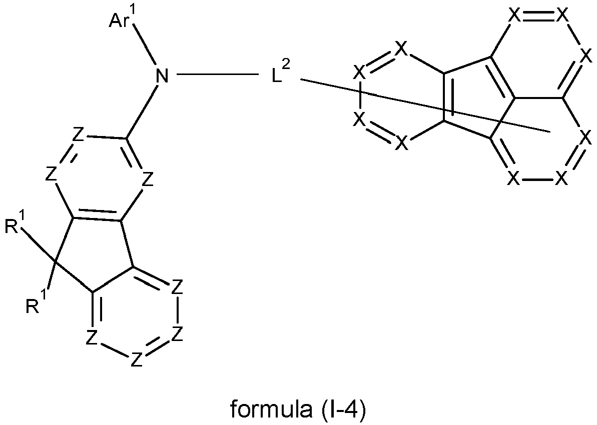 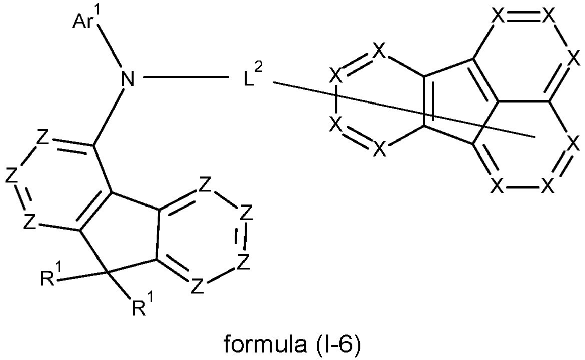 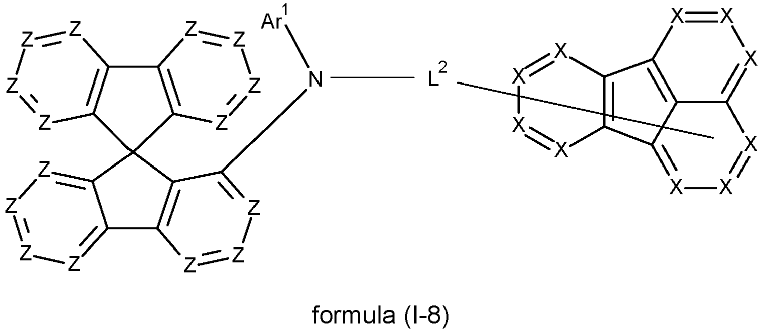 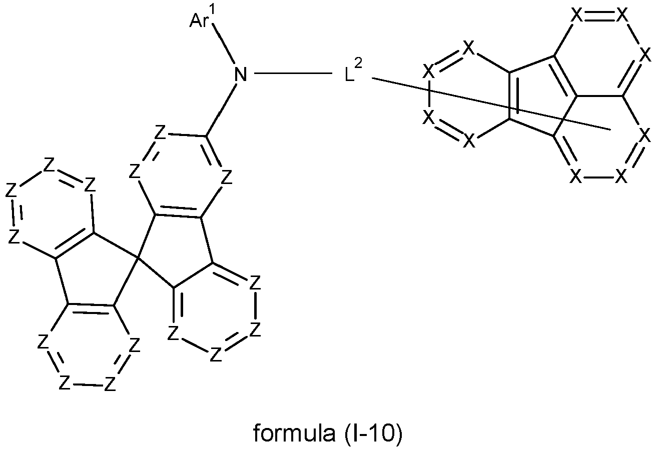 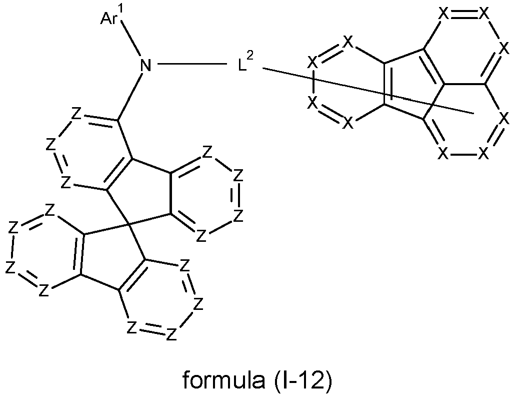 where the variables occurring are defined as in one or more of Claims 1 to 9.

11. Process for the preparation of a compound of formula (I) according to one or more of Claims 1 to 10, which is **characterised in that** a fluorenylamine is reacted with an aromatic or heteroaromatic compound in a Buchwald coupling reaction.

12. Oligomer, polymer or dendrimer containing one or more compounds of formula (I) according to one or more of Claims 1 to 10, where the bond(s) to the polymer, oligomer or dendrimer can be localised at any desired positions in formula (I) that are substituted by R¹, R², R³, R⁴ or R⁵.

13. Formulation comprising at least one compound according to one or more of Claims 1 to 10 or a polymer, oligomer or dendrimer according to Claim 12, and at least one solvent.

14. Electronic device containing at least one compound according to one or more of Claims 1 to 10 or a polymer, oligomer or dendrimer according to Claim 12.

15. Electronic device according to Claim 14, **characterised in that** it is an organic electroluminescent device comprising anode, cathode and at least one emitting layer, where at least one organic layer of the device, which can be an emitting layer or a hole-transporting layer, comprises the at least one compound.

16. Electronic device according to Claim 15, **characterised in that** it is an organic electroluminescent device comprising anode, cathode and at least one emitting layer comprising a red-emitting phosphorescent emitter, where the at least one compound is present in the emitting layer as matrix material.

17. Use of a compound according to one or more of Claims 1 to 10 in an electronic device.

## Revendications

1. Composé de formule (I) 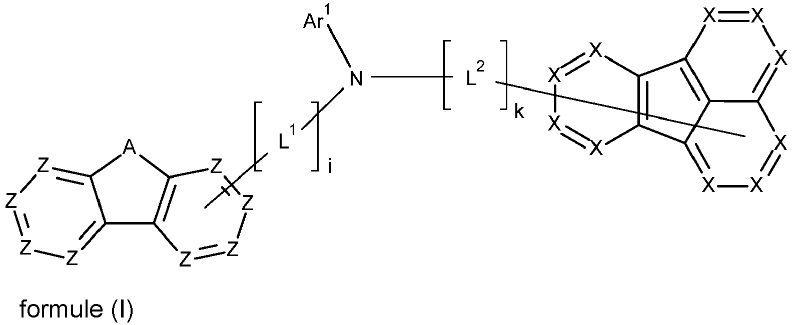 dans laquelle ce qui suit s'applique aux variables présentes :
A est égal à C(R¹)₂ ou est égal à 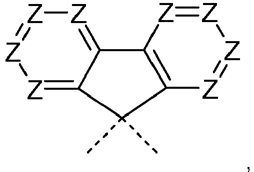
où les lignes en pointillés représentent les liaisons aux cycles à six chaînons aromatiques ;
Z est à chaque occurrence, de manière identique ou différente, CR² ou N ou C, où un groupement Z est égal à C précisément si le groupement [L¹]ᵢ est lié à celui-ci ;
X est à chaque occurrence, de manière identique ou différente, CR³ ou N ou C, où un groupement X est égal à C précisément si le groupement [L²]ₖ est lié à celui-ci ;
L¹, L² sont choisis à chaque occurrence, de manière identique ou différente, parmi un groupement divalent dérivé de benzène, biphényle, terphényle, fluorène, spirobifluorène, indénofluorène, carbazole, dibenzofurane, dibenzothiophène, chacun d'entre eux étant éventuellement substitué par des radicaux R⁴, ou une combinaison de deux, ou plus, parmi ces groupements ;
Ar¹ est un noyau aromatique ayant de 6 à 24 atomes de cycle aromatique, qui peut être substitué par un ou plusieurs radicaux R⁵, ou un noyau hétéroaromatique ayant de 5 à 24 atomes de cycle aromatique, qui peut être substitué par un ou plusieurs radicaux R⁵, où Ar¹ n'englobe pas de groupement hétéroaryle azoté qui est lié directement à l'atome d'azote d'amine de la formule (I), et où Ar¹ et les substituants y étant liés ne contiennent pas de groupement carbazole ;
R¹, R³, R⁴ sont choisis à chaque occurrence, de manière identique ou différente, parmi H, D, F, C(=O)R⁶, CN, Si(R⁶)₃, P(=O)(R⁶)₂, OR⁶, S(=O)R⁶, S(=O)₂R⁶, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹ ou R³ ou R⁴ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁶ ; et où un ou plusieurs groupements CH₂ dans lesdits groupements alkyle, alcoxy, alcényle et alcynyle peuvent être remplacés par -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO ou SO₂ ;
R² est choisi à chaque occurrence, de manière identique ou différente, parmi H, D, F, C(=O)R⁶, CN, Si(R⁶)₃, P(=O)(R⁶)₂, OR⁶, S(=O)R⁶, S(=O)₂R⁶, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R² peuvent être liés les uns aux autres et peuvent former un cycle ; où lesdits groupements alkyle, alcoxy et alcynyle et lesdits noyaux aromatiques et noyaux hétéroaromatiques peuvent dans chaque cas être substitués par un ou plusieurs radicaux R⁶ ; et où un ou plusieurs groupements CH₂ dans lesdits groupements alkyle, alcoxy et alcynyle peuvent être remplacés par -C=C-, Si(R⁶)₂, C=O, C=NR⁶, -C(=O)O-, -C(=O)NR⁶-, NR⁶, P(=O)(R⁶), -O-, -S-, SO ou SO₂ ;
R⁵ est choisi à chaque occurrence, de manière identique ou différente, parmi H, D, Si(R⁶)₃, OR⁶, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁵ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁶ ; et où un ou plusieurs groupements CH₂ dans lesdits groupements alkyle, alcoxy, alcényle et alcynyle peuvent être remplacés par -R⁶C=CR⁶-, -C=C-, Si(R⁶)₂, NR⁶, O- ou -S- ;
R⁶ est choisi à chaque occurrence, de manière identique ou différente, parmi H, D, F, C(=O)R⁷, CN, Si(R⁷)₃, N(R⁷)₂, P(=O)(R⁷)₂, OR⁷, S(=O)R⁷, S(=O)₂R⁷, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁶ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁷ ; et où un ou plusieurs groupements CH₂ dans lesdits groupements alkyle, alcoxy, alcényle et alcynyle peuvent être remplacés par -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO ou SO₂ ;
R⁷ est choisi à chaque occurrence, de manière identique ou différente, parmi H, D, F, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R⁷ peuvent être liés les uns aux autres et peuvent former un cycle ; et où lesdits groupements alkyle, alcoxy, alcényle et alcynyle, noyaux aromatiques et noyaux hétéroaromatiques peuvent être substitués par F ou CN ;
i est égal à 0, 1, 2 ou 3 ; et
k est égal à 1, 2 ou 3,
où le groupement fluoranthène dans la formule (I) est lié en position 3 ou 4, où les positions sont numérotées comme suit :

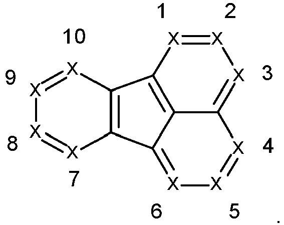

2. Composé selon la revendication 1, **caractérisé en ce que** le groupement de formule 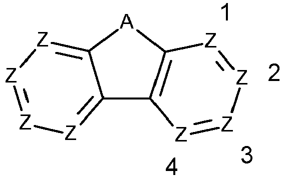 dans la formule (l) est lié à l'une des positions 1, 3 et 4.

3. Composé selon la revendication 1 ou 2, **caractérisé en ce que** Ar¹ est choisi parmi phényle, biphényle, terphényle ramifié, terphényle non ramifié, quaterphényle ramifié, quaterphényle non ramifié, fluorényle, dibenzofuranyle, dibenzothiophényle, fluorénylphénylène, dibenzofuranylphénylène, dibenzothiophènephénylène, phénanthrényle et triphénylyle, chacun d'entre eux étant éventuellement substitué par des radicaux R⁵.

4. Composé selon l'une ou plusieurs parmi les revendications 1 à 3, **caractérisé en ce que** R¹ est choisi à chaque occurrence, de manière identique ou différente, parmi F, Si(R⁶)₃, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 24 atomes de cycle aromatique, et des noyaux hétéroaromatiques ayant de 5 à 24 atomes de cycle aromatique ; où lesdits groupements alkyle, lesdits noyaux aromatiques et lesdits noyaux hétéroaromatiques peuvent dans chaque cas être substitués par un ou plusieurs radicaux R⁶ ; et où deux groupements R¹ qui sont liés au même atome de carbone peuvent être reliés l'un à l'autre afin de former un cycle, conduisant à un atome de carbone spiro.

5. Composé selon l'une ou plusieurs parmi les revendications 1 à 4, **caractérisé en ce que** R², R³ et R⁴ sont choisis à chaque occurrence, de manière identique ou différente, parmi H, D, F, CN, Si(R⁶)₃, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 24 atomes de cycle aromatique et des noyaux hétéroaromatiques ayant de 5 à 24 atomes de cycle aromatique ; où lesdits groupements alkyle, lesdits noyaux aromatiques et lesdits noyaux hétéroaromatiques peuvent dans chaque cas être substitués par un ou plusieurs radicaux R⁶ ; et où un ou plusieurs groupements CH₂ dans lesdits groupements alkyle peuvent être remplacés par -C=C-, Si(R⁶)₂, C=O, C=NR⁶, -NR⁶-, -O-, -S-, - C(=O)O- ou -C(=O)NR⁶-.

6. Composé selon l'une ou plusieurs parmi les revendications 1 à 5, **caractérisé en ce que** R⁵ est choisi à chaque occurrence, de manière identique ou différente, parmi H, D, Si(R⁶)₃, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 24 atomes de cycle aromatique, et des noyaux hétéroaromatiques ayant de 5 à 24 atomes de cycle aromatique ; où lesdits groupements alkyle, lesdits noyaux aromatiques et lesdits noyaux hétéroaromatiques peuvent dans chaque cas être substitués par un ou plusieurs radicaux R⁶, où R⁵ et les substituants liés à R⁵ ne contiennent pas de groupement carbazole.

7. Composé selon l'une ou plusieurs parmi les revendications 1 à 6, **caractérisé en ce que** R⁶ est choisi à chaque occurrence, de manière identique ou différente, parmi H, D, F, CN, Si(R⁷)₃, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 24 atomes de cycle aromatique et des noyaux hétéroaromatiques ayant de 5 à 24 atomes de cycle aromatique ; où lesdits groupements alkyle, lesdits noyaux aromatiques et lesdits noyaux hétéroaromatiques peuvent dans chaque cas être substitués par un ou plusieurs radicaux R⁷ ; et où un ou plusieurs groupements CH₂ dans lesdits groupements alkyle peuvent être remplacés par -C=C-, -R⁷C=CR⁷- Si(R⁷)₂, C=O, C=NR⁷, -NR⁷-, -O-, -S-, -C(=O)O- ou -C(=O)NR⁷-.

8. Composé selon l'une ou plusieurs parmi les revendications 1 à 7, **caractérisé en ce que** i est égal à 0.

9. Composé selon l'une ou plusieurs parmi les revendications 1 à 8, **caractérisé en ce que** k est égal à 1.

10. Composé selon l'une ou plusieurs parmi les revendications 1 à 9, **caractérisé en ce que** le composé correspond à l'une des formules suivantes : 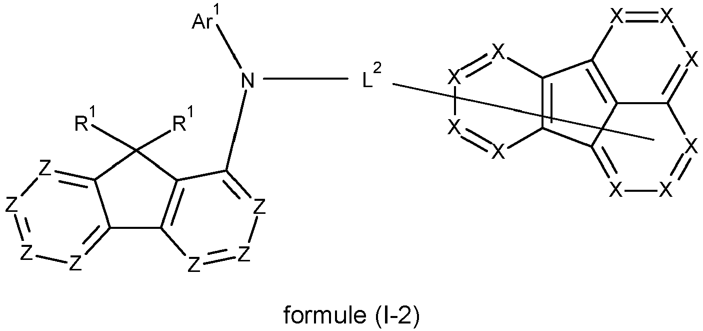 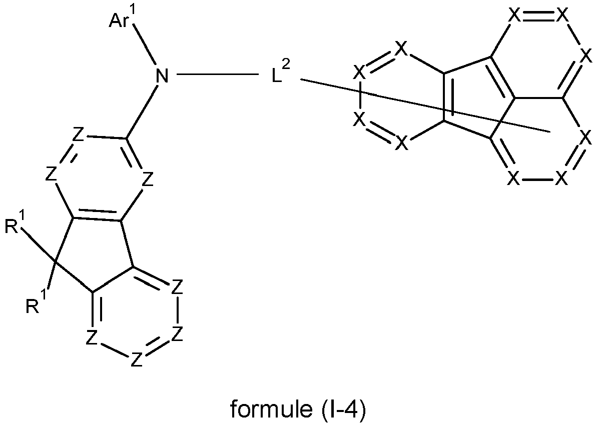 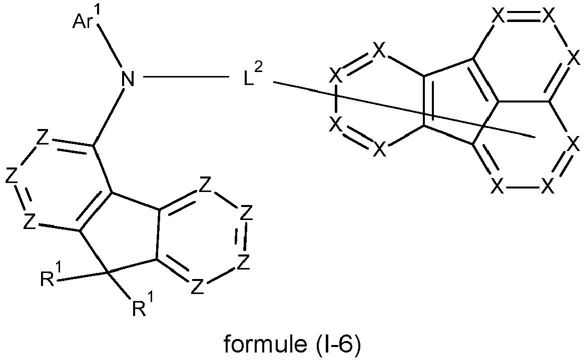 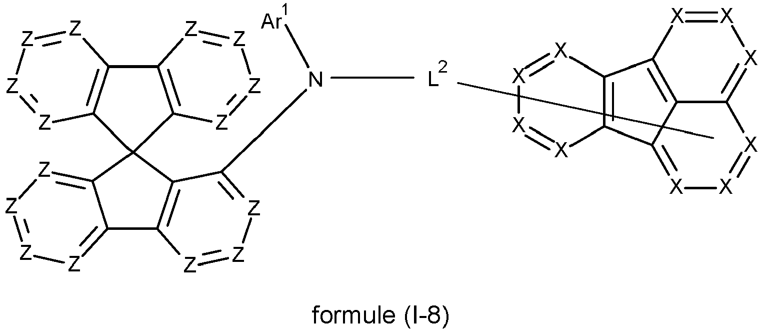 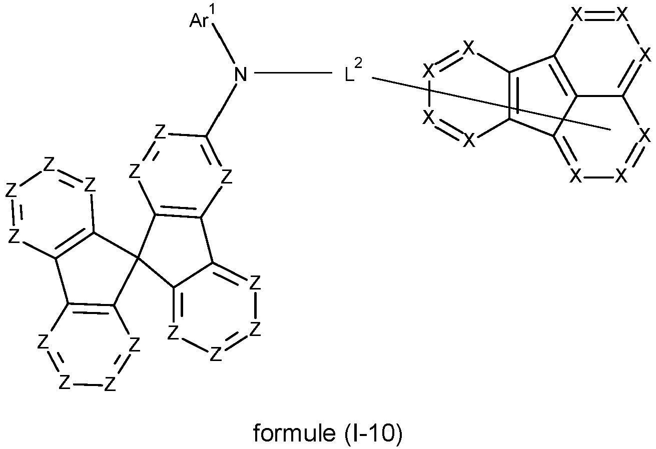 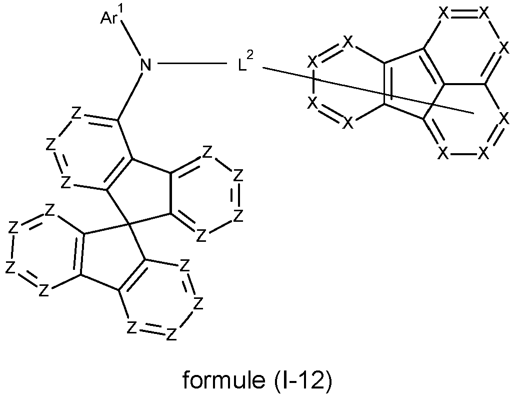 où les variables présentes sont telles que définies selon l'une ou plusieurs parmi les revendications 1 à 9.

11. Procédé de préparation d'un composé de formule (I) selon l'une ou plusieurs parmi les revendications 1 à 10, qui est **caractérisé en ce qu'**une fluorénylamine est réagie avec un composé aromatique ou hétéroaromatique dans une réaction de couplage de Buchwald.

12. Oligomère, polymère ou dendrimère contenant un ou plusieurs composés de formule (I) selon l'une ou plusieurs parmi les revendications 1 à 10, où la ou les liaisons au polymère, à l'oligomère ou au dendrimère peuvent être situées au niveau de positions désirées quelconques dans la formule (I) qui sont substituées par R¹, R², R³, R⁴ ou R⁵.

13. Formulation comprenant au moins un composé selon l'une ou plusieurs parmi les revendications 1 à 10 ou un polymère, oligomère ou dendrimère selon la revendication 12, et au moins un solvant.

14. Dispositif électronique contenant au moins un composé selon l'une ou plusieurs parmi les revendications 1 à 10 ou un polymère, oligomère ou dendrimère selon la revendication 12.

15. Dispositif électronique selon la revendication 14, **caractérisé en ce qu'**il s'agit d'un dispositif électroluminescent organique, comprenant une anode, une cathode et au moins une couche émettrice, où au moins une couche organique du dispositif, qui peut être une couche émettrice ou une couche de transport de trous, comprend le au moins un composé.

16. Dispositif électronique selon la revendication 15, **caractérisé en ce qu'**il s'agit d'un dispositif électroluminescent organique, comprenant une anode, une cathode et au moins une couche émettrice comprenant un émetteur phosphorescent émettant dans le rouge, où le au moins un composé est présent dans la couche émettrice sous forme de matériau de matrice.

17. Utilisation d'un composé selon l'une ou plusieurs parmi les revendications 1 à 10 dans un dispositif électronique.
